# EUROPEAN PATENT SPECIFICATION

(11) **EP 2 166 112 B1**
(45) Date of publication and mention of the grant of the patent: **05.06.2013**
(21) Application number: 08016477.5
(22) Date of filing: 18.09.2008
(51) Int. Cl.: C12Q 1/68

(54) **Means and methods for diagnosing predisposition for treatment emergent suicidal ideation**
Mittel und Verfahren zur Diagnose der Prädisposition zur Behandlung von auftauchenden Suizidgedanken
Supports et procédés pour le diagnostic d'une prédisposition pour le traitement d'idéation suicide émergeant

(43) Date of publication of application: 24.03.2010
(73) Proprietor: Max-Planck-Gesellschaft zur Förderung der Wissenschaften e.V., 80539 München (DE)
(72) Inventor: Menke, Andreas, 80796 München (DE); Binder, Elisabeth B., 80336 München (DE); Holsboer, Florian, 80805 München (DE)
(74) Representative: Vossius & Partner

(56) References cited:
- WO-A-2008/052167
- US-A1- 2008 233 657
- PERLIS R H ETAL: "Association between treatment-emergent suicidal ideation with citalopram and polymorphisms near cyclic adenosine monophosphate response element binding protein in the STAR*D study" ARCHIVES OF GENERAL PSYCHIATRY, AMERICAN MEDICAL ASSOCIATION, vol. 64, no. 6, 1 June 2007 (2007-06-01), pages 689-697, XP008093988 ISSN: 0003-990X
- LAJE GONZALO ET AL: "Genetic markers of suicidal ideation emerging during citalopram treatment of major depression" AMERICAN JOURNAL OF PSYCHIATRY, vol. 164, no. 10, October 2007 (2007-10), pages 1530-1538, XP002512529 ISSN: 0002-953X
- DATABASE DBSNP Submitted SNP of reference SNP rs1630535 18 July 2005 (2005-07-18), XP002512531 retrieved from NCBI Database accession no. ss43689680
- MENKE ANDREAS ET AL: "Genetic markers within glutamate receptors associated with antidepressant treatment-emergent suicidal ideation" AMERICAN JOURNAL OF PSYCHIATRY, vol. 165, no. 7, July 2008 (2008-07), pages 917-918, XP002512530 ISSN: 0002-953X
- ILLUMINA: 'Sentrix® HumanHap300Genotyping BeadChip', [Online] 2006, Retrieved from the Internet: <URL:http://www.illuminakk.co.jp/pdf/HUMANH AP300Datasheet.pdf> [retrieved on 2011-08-15]

## Description

This invention relates to a method of diagnosing a predisposition for or the occurrence of treatment emergent suicidal ideation in an individual, the method being defined in the claims.

Major depression is a common psychiatric disease carrying a substantial loss of productivity and life quality and is associated with a significant morbidity and mortality with a suicide rate of about 15 % ^{1, 2}. While antidepressants are the most effective treatment for depressive patients, there has been controversy if antidepressants, in particular serotonin reuptake inhibitors (SSRI) are implicated in the emergence or worsening of suicidal ideation ³⁻⁵. Although treatment with antidepressants is associated with a significant reduction in suicides ⁶⁻⁹ and was proven to have a suicide preventive effect ¹⁰, there is some evidence that a subgroup of patients (about 5 to 15%) develop treatment emergent suicidal ideation (TESI) in the first weeks following treatment initiation and dose adjustments ^{6, 11, 12}. In 2003, the Regulatory Agency of the British Department of Health warned physicians to avoid treatment with SSRI for depression in children and adolescents after studies showed an increase of agitation, hostility and suicidal behavior in this group ^{13, 14}. This led to a public health advisory by the U.S. Food and Drug Administration (FDA) about the risk of suicidality in pediatric patients taking SSRIs for depression ¹⁵. In 2005 the agency issued a black box warning and medication guide for a series of antidepressants (not only SSRIs) indicating that pediatric and adult patients may be at risk for this side effect. Recent studies showed a significant decrease in diagnosis and psychopharmacologic treatment of depressive episodes in children, adolescents but also adults following these warnings ^{16, 17}. This was paralleled by an increase of suicide rates in the USA and the Netherlands between 2003 and **2005** ¹⁸ for the first time in a decade. Identification of a subgroup of patients at risk for treatment emergent suicidal behavior could therefore be critical to stop depriving patients of beneficial treatment options and to provide the risk group with closer monitoring.

While large family and twin studies estimate the heritability of suicidal behavior to be in the range of 30 to 55 % ^{19, 20}, no such formal evidence exists for TESI.

Markers for predicting the risk of developing TESI have previously been described; see for example US 2008/0102467. However, the markers described in this patent application deliver a significant number of false positives and false negatives when using them for predictive purposes.

The technical problem underlying the present invention is the provision of alternative or improved means and methods for determining a predisposition for treatment emergent suicidal ideation in a patient.

Accordingly, this invention relates to a method of diagnosing a predisposition for or the occurrence of treatment emergent suicidal ideation in an individual, the method being defined in the claims.

The term "treatment emergent suicidal ideation (TESI)" is known in the art and designates the occurrence or enhanced occurrence of suicidal ideation in response to treatment, more specifically in response to treatment with anti-depressive drugs. To explain further, suicidal ideation may be a symptom of depressive disorders prior to beginning any therapy. A subgroup of patients (about 5 to 15%), however, develop and adverse response to treatment, the treatment being directed to an alleviation of the symptoms of depressive disorders, in that they develop or develop in enhanced form suicidal ideation *upon the administration* of anti-depressant drugs. While sufficient psychopharmacological treatment may eventually cure or at least alleviate this side effect, it is necessary to closely monitor those patients which have a predisposition to develop TESI. Moreover, the additional administration of anxiolytica and/or sedative medication is to be considered in such patients.

The term "predisposition" in relation to a disease has its established meaning in the art and is used accordingly here. To explain further, the term "predisposition" relates to the susceptibility to a disease that can be triggered under certain conditions. A person exhibiting a predisposition to a certain disease is not necessarily destined to develop the disease, but exhibits a risk to do so, wherein the risk exceeds the population average. As regards predisposition for TESI, the conditions which may serve as a trigger comprise, as explained above, the administration of anti-depressant drugs.

In addition to providing a method of diagnosing a predisposition for TESI, described is a method of diagnosing the occurrence of TESI. While the former embodiment relates to patients which have not or have not yet developed TESI, the latter embodiment relates to patients which already suffer from TESI. The method of diagnosing the occurrence of TESI may be applied to the latter group of patients either as the only method of diagnosing TESI, or a conjunction with established methods of diagnosing TESI. When applying the method of diagnosing TESI in conjunction with known methods of diagnosing TESI, the performance of the overall diagnostic method is improved. In particular, the number of falls positives and falls negatives is reduced.

The term "SNP" is well-known in the art and is shorthand for "single nucleotide polymorphism". A SNP is a DNA sequence variation which is confined to a single position. Usually, polymorphisms are distinguished from mutations based on their prevalence. Sometimes a threshold of 1% prevalence in a population of individuals is considered for separating polymorphisms (more frequent) from mutations (less frequent). When present in an exon, and dependent on the occurrence of alternative splicing, the SNP may also be present in the mature mRNA. In the latter case, and depending on the degeneracy of the genetic code, a SNP may also be visible at the protein level. SNPs may furthermore be divided into SNPs occurring within a known locus, in the proximity of a known locus, and SNPs that are 5' further away than 2kb from the most 5' feature of a gene and 3' further away than 500 bases from most 3' feature of a gene. Where applicable, loci where a SNP is located in and/or loci in the proximity of a SNP are indicated in the tables provided further below. Generally speaking, a SNP may or may not be associated with a certain phenotype, disease, or predisposition for a disease. The present inventors provide a plurality of SNPs, the association of which with TESI is disclosed herein for the first time.

SNPs are being annotated, collected and maintained in various databases including the SNP database of the National Centre for Biotechnology Information of the National Institutes of Health (NCBI), US. The SNPs according to the invention are referred to below by using "rs" identifiers as used in the SNP database of the NCBI, also known as "dbSNP". More specifically, the rs identifiers refer to the dbSNP build 125, NCBI build 36.1/HG18. The SNP database of the NCBI is described in, for example, Sherry et al. (2001)²¹.

The SNPs according to the present invention are defined by the sequences comprised in the sequence listing. Generally speaking, a polymorphism may occur in at least two allelic forms, at least one of which may be referred to as the risk allele in those cases where an allelic form is associated with a disease or a risk to develop a disease. The sequences enclosed herewith and defining the SNPs of SEQ ID NOs: 1 to 100 throughout present the risk allele. In other words, presence of the respective sequence in a sample obtained from a patient is indicative of a risk to develop treatment emergent suicidal ideation. The respective allele which is not associated with a risk to develop TESI is indicated in the feature part of the respective entry of the sequence listing as "variation". While the respective entire sequence as presented in the sequence listing may be used to define the SNP according to the invention, it is also envisaged to use a subsequence of the sequence of the respective SEQ ID NO., said subsequence being, for example, 15, 17, 19, 21, 23 or 25 nucleotides in length and the variable position being located, for example, in the middle of the respective subsequence. Also subsequences consisting of an even number of nucleotides may be considered such as subsequences consisting of 16, 18, 20, 22 and 24 nucleotides, wherein the variable position may be, for example, one of the two central positions of the respective subsequence. The nucleobase characterizing the risk allele is furthermore presented in Tables 2 and 3.

Table 1 below presents the 100 SNPs according to the invention in the same order as they are presented in the enclosed sequence listing. More specifically, the SNPs are ordered according to the p-value. The p-value in this case is the likelihood that the observed association with TESI occurs by chance. The first column of Table 1 provides the database identifier of the respective SNP in dbSNP build 125. The corresponding sequences as presented in the enclosed sequence listing may also be retrieved from database NCBI build 36.1/HG18. The fifth column of the table presents the nucleobase present at the variable position in the risk allele. In addition to the sequence listing, also Table 6 presents the sequences of the SNPs of SEQ ID NOs:1 to 100.

**Table 1**

| **SNP** | **CHR** | **MAP** | **Gene** | **Risk allele** | **p-value** |
|---|---|---|---|---|---|
| rs1630535 | 15 | 58297467 | | A | 1.3x-7 |
| rs4724701 | 7 | 5495408 | #FBXL18 | A | 2x10-6 |
| rs1265235 | 6 | 4445680 | | A | 1.06x-5 |
| rs2414660 | 15 | 58262633 | | T | 1.37x-5 |
| rs12589623 | 14 | 47934734 | | A | 0.00002 |
| rs583338 | 18 | 39126230 | | G | 0.00003 |
| rs279542 | 3 | 9910772 | #IL17RE::2 | G | 0.00003 |
| rs279553 | 3 | 9909604 | #IL17RE::2 | G | 0.00003 |
| rs2774089 | 13 | 111885503 | | G | 0.00004 |
| rs1421780 | 5 | 174335276 | | A | 0.00005 |
| rs4907674 | 13 | 111900673 | | G | 0.00005 |
| rs7676106 | 4 | 190343908 | | C | 0.00006 |
| rs9949324 | 18 | 74181100 | | A | 0.00007 |
| rs4518023 | 20 | 39454182 | | A | 0.00008 |
| rs7704939 | 5 | 78109953 | #ARSB | A | 0.00008 |
| rs4544214 | 15 | 30719195 | #ARHGAP11A::2 | T | 0.00009 |
| rs6072343 | 20 | 39401601 | #LPIN3 | A | 0.00009 |
| rs965118 | 7 | 150809355 | #RHEB | A | 0.0001 |
| rs1109089 | 7 | 150800951 | #RHEB | G | 0.00011 |
| rs2980872 | 8 | 126486101 | | C | 0.00011 |
| rs2025949 | 13 | 92742277 | #GPC6 | G | 0.00012 |
| rs12142266 | 1 | 87070436 | | T | 0.00012 |
| rs9877859 | 3 | 112923378 | #PLCXD2 | C | 0.00012 |
| rs2070439 | 21 | 34933082 | | T | 0.00012 |
| rs12617566 | 2 | 167435454 | | A | 0.00013 |
| rs1958421 | 14 | 83249995 | | G | 0.00013 |
| rs9589698 | 13 | 92722965 | #GPC6 | C | 0.00013 |
| rs7645289 | 3 | 18047811 | | G | 0.00014 |
| rs2664537 | 20 | 39247142 | #ZHX3#PLCG1 | A | 0.00015 |
| rs2228246 | 20 | 39225477 | #PLCG1 | G | 0.00015 |
| rs2088138 | 17 | 44359721 | #SNF8#UBE2Z | T | 0.00015 |
| rs2839178 | 21 | 46503201 | #MCM3AP | A | 0.00016 |
| rs6500497 | 16 | 87338087 | #FLJ40448 | T | 0.00016 |
| rs11258903 | 10 | 14236785 | #FRMD4A | T | 0.00019 |
| rs2244057 | 14 | 61162625 | | C | 0.00019 |
| rs1459841 | 5 | 95936220 | | G | 0.0002 |
| rs7901463 | 10 | 59842383 | | C | 0.0002 |
| rs584762 | 11 | 31827333 | | C | 0.0002 |
| rs761453 | 11 | 31834575 | | A | 0.0002 |
| rs6035712 | 20 | 20773775 | | T | 0.0002 |
| rs554710 | 9 | 135171668 | | T | 0.00021 |
| rs6868846 | 5 | 59164510 | | A | 0.00021 |
| rs1013120 | 17 | 13485943 | | C | 0.00022 |
| rs649867 | 18 | 75501034 | | G | 0.00023 |
| rs6072317 | 20 | 39314930 | #ZHX3 | T | 0.00023 |
| rs4795069 | 17 | 30633359 | | A | 0.00026 |
| rs6948196 | 7 | 150844144 | #RHEB | T | 0.00029 |
| rs13403584 | 2 | 179550306 | | T | 0.00029 |
| rs7685314 | 4 | 190363247 | | A | 0.0003 |
| rs2662090 | 3 | 9066945 | #SRGAP3 | C | 0.0003 |
| rs6500498 | 16 | 87338145 | #FLJ40448 | G | 0.0003 |
| rs2074997 | 7 | 150805218 | #RHEB | G | 0.0003 |
| rs1204798 | 6 | 116650539 | #NT5DC1 | A | 0.00031 |
| rs12143647 | 1 | 228874868 | #COG2 | T | 0.00032 |
| rs3801033 | 7 | 6700627 | #ZNF12 | T | 0.00032 |
| rs10997044 | 10 | 67727448 | #CTNNA3 | A | 0.00035 |
| rs7350731 | 14 | 25179946 | | A | 0.00038 |
| rs9323737 | 14 | 83252414 | | G | 0.00038 |
| rs4881394 | 10 | 5103710 | | C | 0.0004 |
| rs4799159 | 18 | 74381450 | | A | 0.0004 |
| rs2270101 | 7 | 22902528 | | C | 0.00041 |
| rs356999 | 2 | 60665087 | | T | 0.00041 |
| rs618670 | 18 | 74187714 | | A | 0.00042 |
| rs7788668 | 7 | 22947162 | #DRCTNNB1A | A | 0.00045 |
| rs9332172 | 10 | 96721777 | #CYP2C9 | G | 0.00046 |
| rs902923 | 9 | 93373419 | | G | 0.00046 |
| rs4810622 | 20 | 45310632 | #PRKCBP1 | T | 0.00047 |
| rs6873640 | 5 | 123937398 | | C | 0.00047 |
| rs9302822 | 16 | 6482925 | #A2BP1 | A | 0.00047 |
| rs3753151 | 7 | 150815917 | #RHEB | C | 0.00051 |
| rs10490832 | 3 | 74427045 | #CNTN3 | G | 0.00052 |
| rs1545384 | 3 | 74434950 | #CNTN3 | A | 0.00052 |
| rs1884641 | 20 | 5987908 | #C20orf75 | G | 0.00052 |
| rs1958438 | 14 | 83230211 | | T | 0.00053 |
| rs4917639 | 10 | 96715524 | #CYP2C9 | C | 0.00054 |
| rs2279103 | 18 | 75574114 | #CTDP1 | T | 0.00057 |
| rs7307064 | 12 | 21525660 | #RECQL::2 | C | 0.00059 |
| rs6793017 | 3 | 2554126 | #CNTN4 | A | 0.00066 |
| rs523386 | 18 | 75628182 | | C | 0.00072 |
| rs1984151 | 6 | 94585819 | | T | 0.00076 |
| rs7719325 | 5 | 123500474 | | G | 0.00076 |
| rs1454333 | 4 | 92062152 | | A | 0.00078 |
| rs7146332 | 14 | 40847650 | | A | 0.00079 |
| rs11250017 | 8 | 10357428 | | C | 0.00079 |
| rs17175096 | 18 | 60871165 | | C | 0.00084 |
| rs2299965 | 7 | 150823594 | #RHEB | C | 0.00085 |
| rs2299967 | 7 | 150836143 | #RHEB | T | 0.00085 |
| rs720733 | 4 | 138216796 | | A | 0.00085 |
| rs635546 | 18 | 5927731 | | C | 0.00086 |
| rs7550277 | 1 | 239520957 | #RGS7 | C | 0.00086 |
| rs301191 | 3 | 177621532 | | A | 0.00089 |
| rs301193 | 3 | 177622218 | | C | 0.00089 |
| rs12583395 | 13 | 34117777 | | G | 0.00095 |
| rs1037448 | 11 | 60891107 | #CYBASC3 | T | 0.00096 |
| rs4939517 | 11 | 60883433 | #CYBASC3 | C | 0.00096 |
| rs4388301 | 6 | 138324067 | | C | 0.00096 |
| rs8095186 | 18 | 38167177 | | G | 0.00098 |
| rs1891877 | 10 | 23018034 | #PIP5K2A | C | 0.00098 |
| rs2348427 | 4 | 111633847 | #ENPEP | T | 0.00098 |
| rs9480684 | 6 | 107118917 | #RTN4IP1 | A | 0.00099 |

| | | | | | |
|---|---|---|---|---|---|
| ***Table 1:* Results from association analysis** Best empiric associations calculated with Fisher Product Method over both allelic and genotypic tests with p < 0.001 and being significantly associated in both non-TESI comparison groups. The non-TESI comparison groups were 1) all individuals without increase in suicidality independent of baseline suicidality (no increase of the HAM-D item 3 over time; n = 329) and 2) a sub-group of 1), patients rating zero on item 3 throughout treatment (item 3 of the HAM-D = zero at all visits; n = 79; see also example 1, *Psychopathology and phenotype definition*). SNP: reference sequence identification number according to dbSNP; CHR: chromosome number; MAP: physical location of SNP on genome build HG18. | | | | | |

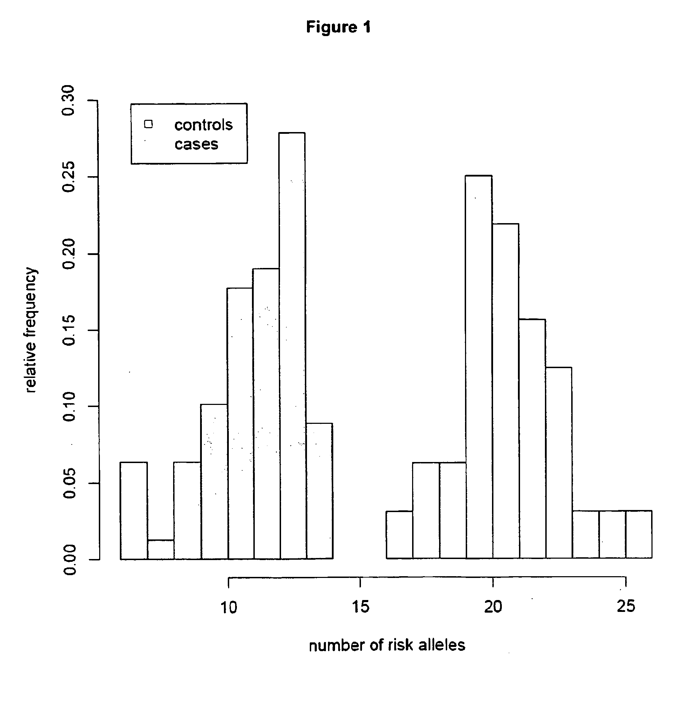

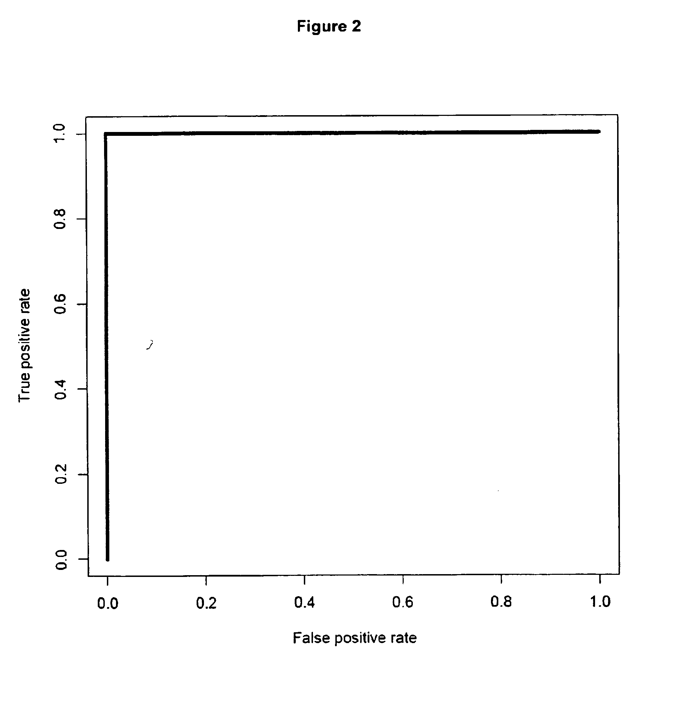

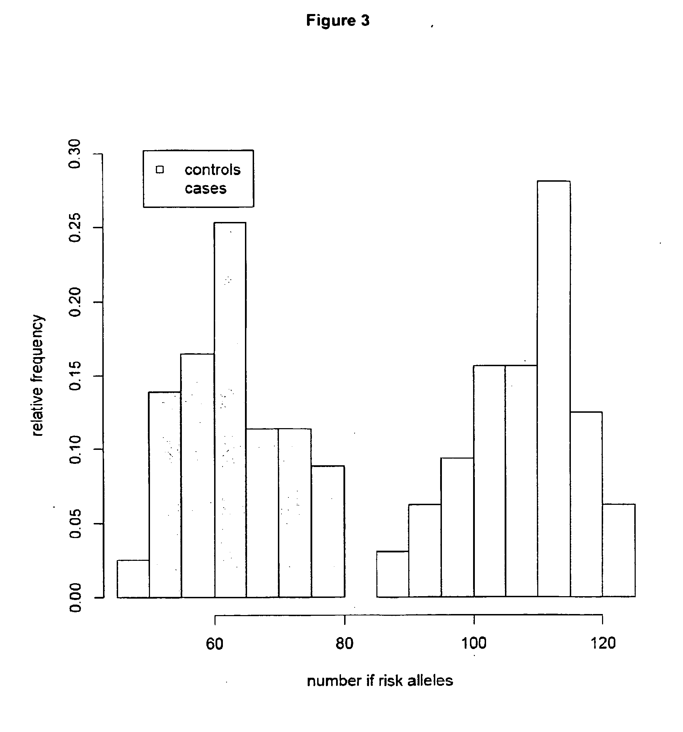

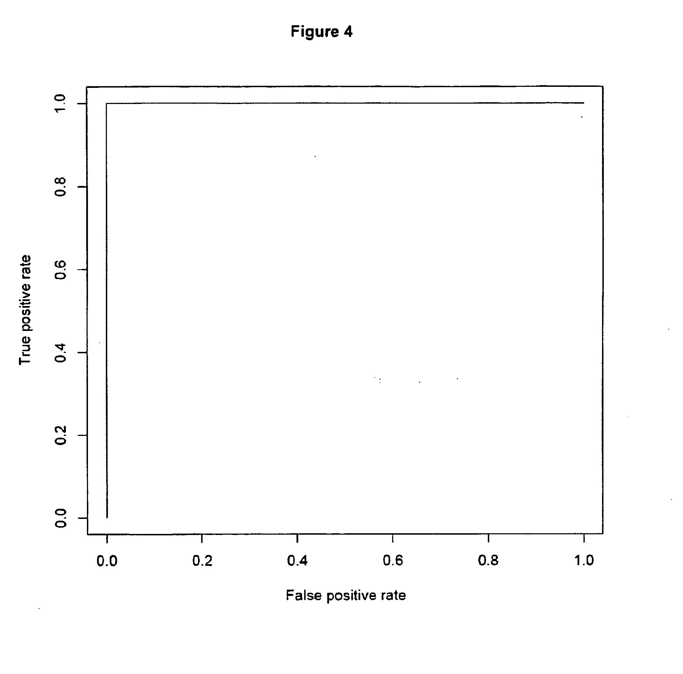

The SNPs according to the invention are characterized by a particularly high predictive value. As shown in the Example as well as in Figures 3 and 4 enclosed herewith, when using all 100 SNPs according to the invention, a perfect discrimination between risk patients and patients which do not exhibit a risk of developing TESI is achieved. It is noteworthy that, according to the invention, a subset of 18 SNPs (see Table 2) has the same discriminatory power; see also Figures 1 and 2. When using the subset of 18 SNPs according to the invention or the respective subsequences thereof as defined herein above, individuals with 16 or more risk alleles are classified as TESI positive. When using 100 SNPs as defined by SEQ ID NOs. 1 to 100 or the respective subsequences thereof as defined herein above, individuals with 85 or more risk alleles are classified as TESI positive. Even single SNPs, in particular those with low P-values, allow excellent prediction.

The SNPs according to the invention are furthermore indicative of possible pathophysiological pathways relevant for TESI. Accordingly, they are indicative of candidate targets for therapeutic intervention. Genes encoding said candidate targets are indicated in Tables 1 to 3.

Said SNPs comprise the SNP defined by SEQ ID NO: 1. This is the SNP being mentioned first in Table 1. When selecting subsets of SNPs, preference is given to subsets comprising SNPs with low p-values. P-values are indicated in Table 1.

Said SNPs consist of or comprise the SNPs defined by SEQ ID NOs: 1, 3, 6, 9, 21, 23, 29, 32, 38, 53, 55, 59, 61, 62, 66, 76, 78 and 94.

A set of four SNPs SEQ ID NOs: 1, 3, 66 and 78 has been shown to correctly predict a risk of developing TESI in 90% of the patients analyzed. The set of 18 SNPs according to the invention provides correct predictions for 100% of the patients analyzed (see Figures 1 and 2 enclosed herewith). Table 2 provides a listing of the 18 SNPs characterized by SEQ ID NOs: 1, 3, 6, 9, 21, 23, 29, 32, 38, 53, 55, 59, 61, 62, 66, 76, 78 and 94.

**Table 2**

| **SNP** | **CHR** | **MAP** | **Gene** | **Closest gene** | **Distance to cl. gene** | **Risk allele** | **P value** |
|---|---|---|---|---|---|---|---|
| rs1630535 | 15 | 58297467 | | ANXA2P3 | -129157 | A | 1.3x-7 |
| rs1265235 | 6 | 4445680 | | Q5G014 | -110164 | A | 1.06x-5 |
| rs583338 | 18 | 39126230 | | SYT4 | 14801 | G | 0.00003 |
| rs2774089 | 13 | 111885503 | | C13orf28 | -193130 | G | 0.00004 |
| rs2025949 | 13 | 92742277 | GPC6 | | | G | 0.00012 |
| rs9877859 | 3 | 112923378 | PLCXD2 | | | C | 0.00012 |
| rs2664537 | 20 | 39247142 | ZHX3 | | | A | 0.00015 |
| rs2839178 | 21 | 46503201 | MCM3AP | | | A | 0.00016 |
| rs584762 | 11 | 31827333 | | PAX6 | 31249 | C | 0.0002 |
| rs1204798 | 6 | 116650539 | NT5DC1 | | | A | 0.00031 |
| rs3801033 | 7 | 6700627 | ZNF12 | | | T | 0.00032 |
| rs4881394 | 10 | 5103710 | | AKR1C3 | -22872 | C | 0.0004 |
| rs2270101 | 7 | 22902528 | | FAM126A | -47256 | C | 0.00041 |
| rs356999 | 2 | 60665087 | | BCL11A | 30951 | T | 0.00041 |
| rs902923 | 9 | 93373419 | ROR2 | | | G | 0.00046 |
| rs2279103 | 18 | 75574114 | CTDP1 | | | T | 0.00057 |
| rs6793017 | 3 | 2554126 | CNTN4 | | | A | 0.00066 |
| rs1037448 | 11 | 60891107 | TMEM138 | | | T | 0.00096 |

| | | | | | | | |
|---|---|---|---|---|---|---|---|
| ***Table 2:* Results from association analysis, subset with 18 SNPs** Best empiric associations calculated with Fisher Product Method over both allelic and genotypic tests with p < 0.001 and being significantly associated in both non-TESI comparison groups. SNP: reference sequence identification number according to dbSNP; CHR: chromosome number; MAP: physical location of SNP on genome build HG18. | | | | | | | |

Subsets of SNPs, i.e. in the present case subsets of less than 100 SNPs, exhibiting optimal predictive power on a given data set may be determined with tools known in the art when provided with the set of 100 SNPs according to the invention. Suitable tools are described in the examples enclosed herewith.

As regards the means and methods for determining presence or absence of a given SNP, numerous suitable methods are known in the art. Any of these methods, either alone or in combination, may be used for determining presence or absence of one or more SNPs according to the invention in a sample. In a preferred embodiment, said determining is effected by allele specific hybridization, allele specific oligonucleotide ligation, primer extension, minisequencing, mass spectroscopy, heteroduplex analysis, single strand conformational polymorphism, denaturing gradient gel electrophoresis, microarray analysis, temperature gradient gel electrophoresis or combinations thereof.

To explain further, in allele specific hybridization a probe is used which is preferably strictly complementary to a region of the target nucleic acid comprising the SNP in question. Hybridization conditions are chosen which allow to distinguish between full complementarity and a single mismatch.

Allele specific oligonucleotide ligation is another method for detecting alleles that differ by a single base. A pair of oligonucleotide probes that hybridize to adjacent segments of the target nucleic acid are used. The oligomer on the 5' side of the pair is an allele specific oligonucleotide in that it is strictly complementary to one specific allele of the target nucleic acid sequence, in the present case to the risk allele. The last base at the 3' end of this oligonucleotide corresponds to the SNP. The oligomer on the 3' side of the pair is the same for both different alleles. In case the risk allele is present, both oligonucleotides hybridize completely and are amenable to ligation with DNA ligase. In case the risk allele is not present, hybridization is not complete and no ligation is possible. Detecting the ligation product corresponds to detecting the risk allele. A variety of suitable detection schemes for the ligation product are known in the art.

In a primer extension assay according to the invention, nucleic acid comprised in the patient sample is hybridized to a primer complementary to the region adjacent to the SNP site. Dideoxyribonucleotides (ddNTPS) and DNA polymerase are added to the mixture and the primer is extended by a single nucleotide. The single nucleotide added is dependent on the allele of the amplified DNA. Primer extension biochemistry can be coupled with a variety of detection schemes, comprising fluorescence, fluorescence polarization (FP), luminescence and mass spectrometry (MS). Primer extension is sometimes also referred to as minisequencing.

Preferably, the hybridization of said primer to said nucleic acid is specific. Means of ensuring specificity of hybridization according to the present invention are known in the art and include stringent hybridization conditions. The term "stringent hybridization conditions", as used in the description of the present invention, is well known to the skilled artisan. Appropriate stringent hybridization conditions for each sequence may be established by a person skilled in the art on well-known parameters such as temperature, composition of the nucleic acid molecules, salt conditions etc.; see, for example, Sambrook et al., "Molecular Cloning, A Laboratory Manual"; CSH Press, Cold Spring Harbor, 1989 or Higgins and Hames (eds.), "Nucleic acid hybridization, a practical approach", IRL Press, Oxford 1985, see in particular the chapter "Hybridization Strategy" by Britten & Davidson, 3 to 15. Stringent hybridization conditions are, for example, conditions comprising overnight incubation at 42° C in a solution comprising: 50% formamide, 5x SSC (750 mM NaCl, 75 mM trisodium citrate), 50 mM sodium phosphate (pH 7.6), 5x Denhardt's solution, 10% dextran sulfate, and 20 micrograms/ml denatured, sheared salmon sperm DNA, followed by washing the filters in 0.1x SSC at about 65°. Other stringent hybridization conditions are for example 0.2 x SSC (0.03 M NaCl, 0.003M Natriumcitrat, pH 7) at 65°C.

Microarrays inter alia provide a miniaturized implementation of a plurality of allele specific hybridization assays in arrayed format. Microarrays for SNP detection are known in the art and available from a variety of manufacturers including Affymetrix. These microarrays include oligonucleotide microarrays.

In a further preferred embodiment, said treatment emergent suicidal ideation occurs or may occur in a patient being administered selective serotonin re-uptake inhibitors, selective noradrenalin re-uptake inhibitors, dual serotonin and noradrenalin re-uptake inhibitors and/or tricyclic antidepressants, and optionally being administered neuroleptics, mood stabilizers and/or benzodiazepines. As explained in the background section herein above, antidepressant drugs in general, and in particular selective serotonin reuptake inhibitors may be causative of TESI.

In a further preferred embodiment, said individual is a child or adolescent. As stated in the background section herein above, children and adolescents are considered to be particularly effected by drug induced TESI.

In a further preferred embodiment, said determining comprises isolating a nucleic acid from said sample.

Means and methods for isolating nucleic acids from a sample taken from an individual are well-known in the art²². Preferred samples according to the invention are samples comprising or consisting of body fluids. Generally speaking, body fluids are liquid components of living organisms. Preferred body fluids include blood, serum, saliva, semen, vaginal secretions as well amniotic, cerebrospinal, synovial, pleural, peritoneal and pericardial fluids. The term "nucleic acid" as used herein includes DNA such as cDNA or genomic DNA, and RNA. It is understood that the term "RNA" as used herein comprises all forms of RNA including mRNA, non-coding RNA, tRNA and rRNA. The term "non-coding RNA" in tum includes siRNA (small interfering RNA), miRNA (micro RNA), rasiRNA (repeat associated RNA), snoRNA (small nucleolar RNA) and snRNA (small nuclear RNA). A preferred RNA is mRNA.

Described is also a kit comprising (a) one or more agents suitable for determining presence or absence of two or more SNPs, said SNPs being defined by SEQ ID NOs: 1 to 100; and (b) optionally a manual with instructions for performing the method of the invention. Agents suitable for determining presence or absence of SNPs are well-known in the art^{23, 24}_{.}

In the kit said agents may be selected from primers and probes for determining presence or absence of one or more SNPs of SEQ ID NOs: 1 to 100. Probes and primers may be used in methods such as allele specific hybridization, allele specific oligonucleotide ligation and primer extension as mentioned herein above.

Said more SNPs may be at least 2, 3, 4, 5, 10, 18, 20, 30, 40, 50, 60, 70, 80, 90 or 100 SNPs.

In the method or the kit of the invention, respectively, instead of a SNP as defined in these claims, a corresponding tagging SNP may be used, wherein said tagging SNP is provided in Table 3. Sequences of the tagging SNPs are presented in the sequence listing (SEQ ID NOs: 101 to 813).

**Table 3**

| SNP | type | Closest gene | distance to gene | Tagged_SNP | Tagged_SNP type | Tagged_SNP_r2 |
|---|---|---|---|---|---|---|
| rs1630535 | INTERGENIC | ANXA2P3 | -129157 | | | |
| | | | | rs1872133 | INTERGENIC | 1 |
| | | | | rs1872132 | INTERGENIC | 1 |
| | | | | rs2054680 | INTERGENIC | 1 |
| | | | | rs336536 | INTERGENIC | 1 |
| | | | | rs163135 | INTERGENIC | 1 |
| | | | | rs12148406 | INTERGENIC | 1 |
| | | | | rs336528 | INTERGENIC | 1 |
| | | | | rs17270048 | INTERGENIC | 1 |
| | | | | rs17270055 | INTERGENIC | 1 |
| | | | | rs338446 | INTERGENIC | 1 |
| | | | | rs7182941 | INTERGENIC | 1 |
| | | | | rs7183074 | INTERGENIC | 1 |
| | | | | rs10851673 | INTERGENIC | 1 |
| | | | | rs12912083 | INTERGENIC | 1 |
| | | | | rs1680195 | INTERGENIC | 1 |
| | | | | rs16942291 | INTERGENIC | 1 |
| | | | | rs12148854 | INTERGENIC | 1 |
| | | | | rs8028748 | INTERGENIC | 1 |
| | | | | rs1680222 | INTERGENIC | 1 |
| | | | | rs1680221 | INTERGENIC | 1 |
| | | | | rs12899914 | INTERGENIC | 0,87 |
| rs4724701 | SYNONYMOUS_CODING | FBXL18 | 0 | | | |
| | | | | rs7808325 | INTRONIC | 0,87 |
| | | | | rs3801058 | INTRONIC | 0,87 |
| | | | | rs7806101 | INTRONIC | 0,9 |
| | | | | rs10216189 | INTRONIC | 1 |
| | | | | rs4724699 | INTRONIC | 0,96 |
| | | | | rs7799970 | INTRONIC | 0,96 |
| | | | | rs4077245 | INTRONIC | 0,96 |
| | | | | rs4560713 | INTRONIC | 1 |
| rs1265235 | INTERGENIC | Q5G014_HUMAN | -110164 | | | |
| | | | | rs1265237 | INTERGENIC | 1 |
| | | | | rs1265234 | INTERGENIC | 1 |
| | | | | rs7774098 | INTERGENIC | 0,84 |
| rs2414660 | INTERGENIC | ANXA2P3 | -163991 | | | |
| | | | | rs338424 | INTERGENIC | 1 |
| | | | | rs2136559 | INTERGENIC | 0,82 |
| | | | | rs7165903 | INTERGENIC | 0,92 |
| | | | | rs7173200 | INTERGENIC | 0,91 |
| | | | | rs7173250 | INTERGENIC | 0,85 |
| | | | | rs900634 | INTERGENIC | 0,9 |
| | | | | rs7166014 | INTERGENIC | 0,88 |
| | | | | rs4775243 | INTERGENIC | 0,92 |
| | | | | rs2136557 | INTERGENIC | 0,84 |
| rs12589623 | INTERGENIC | N/A | -9 | | | |
| | | | | rs1621025 | INTERGENIC | 0,85 |
| | | | | rs1769522 | INTERGENIC | 0,85 |
| | | | | rs11157633 | INTERGENIC | 1 |
| | | | | rs7158518 | INTERGENIC | 0,95 |
| rs279553 | SYNONYMOUS_CODING | JAGN1 | 0 | | | |
| | | | | rs279558 | 5PRIME UTR | 0,89 |
| | | | | rs279542 | 3PRIME UTR | 1 |
| | | | | rs33970214 | INTRONIC | 1 |
| rs279542 | 3PRIME UTR | JAGN1 | 0 | | | |
| | | | | rs279558 | 5PRIME UTR | 0,89 |
| | | | | rs279553 | SYNONYMOUS_CODING | 1 |
| | | | | rs33970214 | INTRONIC | 1 |
| rs583338 | INTERGENIC | SYT4 | 14801 | | | |
| | | | | rs816750 | INTERGENIC | 0,9 |
| | | | | rs310095 | INTERGENIC | 1 |
| | | | | rs310091 | INTERGENIC | 1 |
| | | | | rs310086 | INTERGENIC | 1 |
| | | | | rs310087 | INTERGENIC | 1 |
| | | | | rs310089 | INTERGENIC | 1 |
| | | | | rs618486 | INTERGENIC | 1 |
| rs2774089 | INTERGENIC | SOX1 | 111483 | | | |
| | | | | rs2576497 | INTERGENIC | 0,97 |
| | | | | rs1946770 | INTERGENIC | 0,97 |
| | | | | rs100224 | INTERGENIC | 0,97 |
| | | | | rs188122 | INTERGENIC | 0,97 |
| rs1421780 | INTRONIC | Q6ZNE8_HU MAN | 0 | | | |
| rs4907674 | INTERGENIC | SOX1 | 126653 | | | |
| rs7676106 | INTERGENIC | LOC728856 | -48091 | | | |
| | | | | rs9312392 | INTERGENIC | 1 |
| | | | | rs9312393 | INTERGENIC | 1 |
| | | | | rs7689338 | INTERGENIC | 1 |
| | | | | rs4241859 | INTERGENIC | 1 |
| | | | | rs4241860 | INTERGENIC | 1 |
| | | | | rs4241862 | INTERGENIC | 1 |
| | | | | rs4273547 | INTERGENIC | 1 |
| | | | | rs4282263 | INTERGENIC | 1 |
| | | | | rs6553287 | INTERGENIC | 1 |
| | | | | rs6820247 | INTERGENIC | 0,83 |
| | | | | rs10866319 | INTERGENIC | 0,83 |
| rs9949324 | INTERGENIC | N/A | -9 | | | |
| | | | | rs9949519 | INTERGENIC | 1 |
| | | | | rs611509 | INTERGENIC | 1 |
| | | | | rs592612 | INTERGENIC | 0,92 |
| | | | | rs603942 | INTERGENIC | 1 |
| | | | | rs677233 | INTERGENIC | 1 |
| rs7704939 | 3PRIME UTR | ARSB | 0 | | | |
| rs4518023 | INTERGENIC | CHD6 | -10401 | | | |
| | | | | rs6065347 | INTERGENIC | 1 |
| | | | | rs4810317 | INTERGENIC | 1 |
| rs4544214 | UPSTREAM | ARHGAP11A | 46 | | | |
| | | | | rs11632524 | INTRONIC | 0,81 |
| | | | | rs8037818 | INTRONIC | 1 |
| | | | | rs4643284 | UPSTREAM | 1 |
| rs6072343 | UPSTREAM | LPIN3 | -1372 | | | |
| | | | | rs6065333 | INTERGENIC | 0,92 |
| | | | | rs16985655 | INTERGENIC | 0,92 |
| | | | | rs16985677 | INTRONIC | 0,81 |
| rs965118 | INTRONIC | RHEB | 0 | | | |
| | | | | rs17713386 | DOWNSTREA M | 0,97 |
| | | | | rs4726029 | INTRONIC | 0,97 |
| | | | | rs875588 | INTRONIC | 0,97 |
| | | | | rs1109089 | INTRONIC | 0,96 |
| | | | | rs17713697 | INTRONIC | 0,87 |
| | | | | rs2074998 | INTRONIC | 0,97 |
| | | | | rs2074997 | INTRONIC | 0,97 |
| | | | | rs17713890 | INTRONIC | 0,97 |
| | | | | rs2299965 | INTRONIC | 0,97 |
| | | | | rs2284264 | INTRONIC | 0,97 |
| | | | | rs11772458 | INTRONIC | 1 |
| | | | | rs2299967 | INTRONIC | 0,97 |
| | | | | rs3789817 | INTRONIC | 0,97 |
| | | | | rs758666 | INTRONIC | 0,97 |
| | | | | rs736645 | INTRONIC | 0,97 |
| | | | | rs4298422 | INTRONIC | 0,96 |
| | | | | rs2374261 | INTRONIC | 0,97 |
| | | | | rs12112989 | INTRONIC | 0,97 |
| | | | | rs6980020 | INTRONIC | 0,97 |
| | | | | rs12112134 | INTRONIC | 0,97 |
| | | | | rs6948196 | INTRONIC | 0,97 |
| | | | | rs6943752 | INTRONIC | 0,87 |
| | | | | rs7794922 | UPSTREAM | 0,81 |
| rs1109089 | INTRONIC | RHEB | 0 | | | |
| | | | | rs17713386 | DOWNSTREA M | 1 |
| | | | | rs4726029 | INTRONIC | 1 |
| | | | | rs875588 | INTRONIC | 1 |
| | | | | rs7805967 | INTRONIC | 0,83 |
| | | | | rs17713697 | INTRONIC | 0,9 |
| | | | | rs2074998 | INTRONIC | 1 |
| | | | | rs2074997 | INTRONIC | 1 |
| | | | | rs17713890 | INTRONIC | 1 |
| | | | | rs2299961 | INTRONIC | 0,83 |
| | | | | rs965118 | INTRONIC | 0,96 |
| | | | | rs3753151 | INTRONIC | 0,83 |
| | | | | rs2299965 | INTRONIC | 1 |
| | | | | rs2284264 | INTRONIC | 1 |
| | | | | rs11772458 | INTRONIC | 1 |
| | | | | rs2299967 | INTRONIC | 1 |
| | | | | rs3789817 | INTRONIC | 1 |
| | | | | rs758666 | INTRONIC | 1 |
| | | | | rs736645 | INTRONIC | 1 |
| | | | | rs4298422 | INTRONIC | 1 |
| | | | | rs2374261 | INTRONIC | 1 |
| | | | | rs12112989 | INTRONIC | 1 |
| | | | | rs6980020 | INTRONIC | 1 |
| | | | | rs12112134 | INTRONIC | 1 |
| | | | | rs6948196 | INTRONIC | 1 |
| | | | | rs6943752 | INTRONIC | 0,89 |
| | | | | rs7794922 | UPSTREAM | 0,86 |
| rs2980872 | INTERGENIC | TRIB1 | -25643 | | | |
| | | | | rs2954006 | INTERGENIC | 0,93 |
| rs12142266 | INTERGENIC | SEP15_HUM AN | -30281 | | | |
| rs9877859 | INTRONIC | PHLDB2 | 0 | | | |
| | | | | rs4682298 | INTRONIC | 0,84 |
| rs2025949 | INTRONIC | GPC6 | 0 | | | |
| | | | | rs7338831 | INTRONIC | 0,91 |
| | | | | rs9561295 | INTRONIC | 0,94 |
| | | | | rs9589698 | INTRONIC | 0,9 |
| | | | | rs9523990 | INTRONIC | 0,9 |
| | | | | rs9523991 | INTRONIC | 0,94 |
| | | | | rs4142598 | INTRONIC | 0,95 |
| | | | | rs9523998 | INTRONIC | 1 |
| | | | | rs9561304 | INTRONIC | 1 |
| | | | | rs956036 | INTRONIC | 0,95 |
| | | | | rs1475449 | INTRONIC | 0,95 |
| rs2070439 | INTERGENIC | RCAN1 | 23780 | | | |
| rs12617566 | INTERGENIC | XIRP2 | -32784 | | | |
| rs9589698 | INTRONIC | GPC6 | 0 | | | |
| | | | | rs7338831 | INTRONIC | 1 |
| | | | | rs9561295 | INTRONIC | 1 |
| | | | | rs9523990 | INTRONIC | 1 |
| | | | | rs9523991 | INTRONIC | 1 |
| | | | | rs4142598 | INTRONIC | 1 |
| | | | | rs9523998 | INTRONIC | 0,91 |
| | | | | rs2025949 | INTRONIC | 0,9 |
| | | | | rs9561304 | INTRONIC | 0,91 |
| | | | | rs956036 | INTRONIC | 0,95 |
| | | | | rs1475449 | INTRONIC | 0,89 |
| rs1958421 | INTERGENIC | N/A | -9 | | | |
| | | | | rs1958438 | INTERGENIC | 0,92 |
| | | | | rs10139321 | INTERGENIC | 0,92 |
| | | | | rs10132490 | INTERGENIC | 0,89 |
| | | | | rs10143458 | INTERGENIC | 0,91 |
| | | | | rs9671273 | INTERGENIC | 0,92 |
| | | | | rs1952534 | INTERGENIC | 0,92 |
| | | | | rs1958436 | INTERGENIC | 0,91 |
| | | | | rs11159605 | INTERGENIC | 0,95 |
| | | | | rs7359138 | INTERGENIC | 0,8 |
| | | | | rs1958435 | INTERGENIC | 0,92 |
| | | | | rs1958434 | INTERGENIC | 0,91 |
| | | | | rs1958433 | INTERGENIC | 0,92 |
| | | | | rs2149684 | INTERGENIC | 0,87 |
| | | | | rs2183203 | INTERGENIC | 0,91 |
| | | | | rs1958432 | INTERGENIC | 0,91 |
| | | | | rs2149683 | INTERGENIC | 0,92 |
| | | | | rs8010141 | INTERGENIC | 0,92 |
| | | | | rs1958430 | INTERGENIC | 0,92 |
| | | | | rs1958429 | INTERGENIC | 0,92 |
| | | | | rs1958428 | INTERGENIC | 0,92 |
| | | | | rs1958426 | INTERGENIC | 1 |
| | | | | rs1958425 | INTERGENIC | 0,92 |
| | | | | rs1958424 | INTERGENIC | 1 |
| | | | | rs1958423 | INTERGENIC | 1 |
| | | | | rs1958422 | INTERGENIC | 1 |
| | | | | rs10132081 | INTERGENIC | 1 |
| | | | | rs10132130 | INTERGENIC | 1 |
| | | | | rs10147226 | INTERGENIC | 1 |
| | | | | rs1958420 | INTERGENIC | 1 |
| | | | | rs10873350 | INTERGENIC | 1 |
| | | | | rs10140080 | INTERGENIC | 1 |
| | | | | rs9323737 | INTERGENIC | 1 |
| | | | | rs4545730 | INTERGENIC | 1 |
| | | | | rs1952530 | INTERGENIC | 1 |
| | | | | rs1952529 | INTERGENIC | 1 |
| | | | | rs12431659 | INTERGENIC | 1 |
| | | | | rs12434622 | INTERGENIC | 1 |
| | | | | rs10141403 | INTERGENIC | 1 |
| | | | | rs9323738 | INTERGENIC | 1 |
| | | | | rs9323739 | INTERGENIC | 1 |
| | | | | rs1952528 | INTERGENIC | 0,92 |
| | | | | rs1958418 | INTERGENIC | 1 |
| | | | | rs877800 | INTERGENIC | 1 |
| | | | | rs2372550 | INTERGENIC | 0,91 |
| | | | | rs1952545 | INTERGENIC | 1 |
| | | | | rs1952544 | INTERGENIC | 1 |
| | | | | rs10747302 | INTERGENIC | 1 |
| | | | | rs4899838 | INTERGENIC | 0,92 |
| | | | | rs11159610 | INTERGENIC | 0,9 |
| | | | | rs11159611 | INTERGENIC | 0,92 |
| | | | | rs7494095 | INTERGENIC | 0,92 |
| | | | | rs10132137 | INTERGENIC | 0,92 |
| | | | | rs4899839 | INTERGENIC | 0,92 |
| | | | | rs10135095 | INTERGENIC | 0,92 |
| | | | | rs2888357 | INTERGENIC | 0,92 |
| | | | | rs4143911 | INTERGENIC | 0,92 |
| | | | | rs1958458 | INTERGENIC | 0,91 |
| | | | | rs2372553 | INTERGENIC | 0,92 |
| | | | | rs10147836 | INTERGENIC | 0,87 |
| | | | | rs10139812 | INTERGENIC | 0,91 |
| | | | | rs1958456 | INTERGENIC | 0,92 |
| | | | | rs1958455 | INTERGENIC | 0,92 |
| | | | | rs2372523 | INTERGENIC | 0,87 |
| | | | | rs2372524 | INTERGENIC | 0,92 |
| | | | | rs10143348 | INTERGENIC | 0,92 |
| | | | | rs9323740 | INTERGENIC | 0,82 |
| | | | | rs10130591 | INTERGENIC | 0,87 |
| | | | | rs1958449 | INTERGENIC | 0,87 |
| rs7645289 | INTERGENIC | TBC1D5 | 290409 | | | |
| | | | | rs4132218 | INTERGENIC | 1 |
| | | | | rs7627583 | INTERGENIC | 1 |
| | | | | rs9859212 | INTERGENIC | 0,93 |
| | | | | rs6790525 | INTERGENIC | 0,94 |
| | | | | rs4688898 | INTERGENIC | 0,94 |
| | | | | rs9861146 | INTERGENIC | 0,84 |
| | | | | rs2203242 | INTERGENIC | 0,84 |
| rs2088138 | 3PRIME UTR | UBE2Z | 0 | | | |
| rs2228246 | N/A | N/A | -9 | | | |
| rs2664537 | 3PRIME UTR | ZHX3 | 0 | | | |
| | | | | rs2228246 | NON_SYNONYMOUS_CODIN G | 0,82 |
| | | | | rs6072299 | DOWNSTREAM | 0,93 |
| | | | | rs6072302 | 3PRIME UTR | 0,82 |
| | | | | rs7261917 | INTRONIC | 0,82 |
| | | | | rs6065325 | INTRONIC | 0,82 |
| | | | | rs6072317 | INTRONIC | 0,82 |
| | | | | rs6065326 | INTRONIC | 0,94 |
| | | | | rs6072320 | INTRONIC | 0,94 |
| | | | | rs6072321 | INTRONIC | 0,82 |
| | | | | rs6072322 | INTRONIC | 0,82 |
| rs6500497 | 3PRIME UTR | Q8N7R2_HU MAN | 0 | | | |
| | | | | rs7185630 | 3PRIME UTR | 0,87 |
| | | | | rs2242166 | 3PRIME UTR | 0,87 |
| | | | | rs2242164 | UPSTREAM | 0,87 |
| | | | | rs6500498 | 3PRIME UTR | 1 |
| rs2839178 | INTRONIC | MCM3AP | 0 | | | |
| rs11258903 | INTRONIC | FRMD4A | 0 | | | |
| | | | | rs11258902 | INTRONIC | 1 |
| | | | | rs10159816 | INTRONIC | 1 |
| rs2244057 | INTERGENIC | HIF1A | -69366 | | | |
| | | | | rs2253980 | INTERGENIC | 0,94 |
| | | | | rs2246928 | INTERGENIC | 0,95 |
| | | | | rs1006529 | INTERGENIC | 1 |
| | | | | rs698028 | INTERGENIC | 0,84 |
| rs1459841 | INTERGENIC | CAST | -87312 | | | |
| rs7901463 | INTERGENIC | TFAM | 12223 | | | |
| | | | | rs10763541 | INTERGENIC | 1 |
| | | | | rs2081692 | INTERGENIC | 0,87 |
| | | | | rs7903523 | INTERGENIC | 0,87 |
| | | | | rs2101237 | INTERGENIC | 0,93 |
| | | | | rs2086784 | INTERGENIC | 0,87 |
| | | | | rs2393422 | INTERGENIC | 1 |
| | | | | rs1427219 | INTERGENIC | 0,87 |
| | | | | rs919166 | INTERGENIC | 1 |
| | | | | rs919167 | INTERGENIC | 1 |
| | | | | rs10826187 | INTERGENIC | 0,89 |
| | | | | rs10826188 | INTERGENIC | 1 |
| | | | | rs6481390 | INTERGENIC | 1 |
| | | | | rs7071862 | INTERGENIC | 1 |
| | | | | rs7896213 | INTERGENIC | 1 |
| | | | | rs7900673 | INTERGENIC | 1 |
| | | | | rs7916849 | INTERGENIC | 1 |
| | | | | rs10763545 | INTERGENIC | 1 |
| | | | | rs4948298 | INTERGENIC | 1 |
| | | | | rs4948299 | INTERGENIC | 0,87 |
| | | | | rs4948513 | INTERGENIC | 1 |
| | | | | rs2893777 | INTERGENIC | 0,81 |
| | | | | rs7084955 | INTERGENIC | 1 |
| | | | | rs7085387 | INTERGENIC | 1 |
| | | | | rs7073985 | INTERGENIC | 1 |
| | | | | rs7077653 | INTERGENIC | 1 |
| | | | | rs16912268 | INTERGENIC | 1 |
| | | | | rs10826193 | INTERGENIC | 1 |
| | | | | rs2114562 | INTERGENIC | 0,87 |
| | | | | rs2114563 | INTERGENIC | 0,92 |
| | | | | rs1013473 | INTERGENIC | 0,85 |
| | | | | rs2128909 | INTERGENIC | 0,9 |
| | | | | rs1030432 | INTERGENIC | 1 |
| | | | | rs10509091 | INTERGENIC | 1 |
| | | | | rs7077537 | INTERGENIC | 1 |
| | | | | rs4948301 | INTERGENIC | 1 |
| | | | | rs1125139 | INTERGENIC | 0,87 |
| | | | | rs1896248 | INTERGENIC | 0,86 |
| | | | | rs2114561 | INTERGENIC | 1 |
| rs584762 | INTERGENIC | PAX6 | 31249 | | | |
| | | | | rs585972 | INTERGENIC | 1 |
| | | | | rs604518 | INTERGENIC | 1 |
| | | | | rs685428 | INTERGENIC | 1 |
| | | | | rs624732 | INTERGENIC | 1 |
| | | | | rs761453 | INTERGENIC | 1 |
| | | | | rs2440250 | INTERGENIC | 1 |
| | | | | rs594462 | INTERGENIC | 0,95 |
| | | | | rs687947 | INTERGENIC | 0,95 |
| | | | | rs2473858 | INTERGENIC | 0,9 |
| | | | | rs677874 | INTERGENIC | 0,9 |
| rs761453 | INTERGENIC | PAX6 | 38491 | | | |
| | | | | rs585972 | INTERGENIC | 1 |
| | | | | rs604518 | INTERGENIC | 1 |
| | | | | rs685428 | INTERGENIC | 1 |
| | | | | rs624732 | INTERGENIC | 1 |
| | | | | rs584762 | INTERGENIC | 1 |
| | | | | rs2440250 | INTERGENIC | 1 |
| | | | | rs594462 | INTERGENIC | 0,95 |
| | | | | rs687947 | INTERGENIC | 0,95 |
| | | | | rs2473858 | INTERGENIC | 0,9 |
| | | | | rs677874 | INTERGENIC | 0,9 |
| rs6035712 | INTERGENIC | C20orf74 | 132505 | | | |
| | | | | rs6047077 | INTERGENIC | 0,91 |
| | | | | rs6035711 | INTERGENIC | 0,96 |
| rs6868846 | INTERGENIC | hsa-mir-582 | 129225 | | | |
| | | | | rs256353 | INTERGENIC | 1 |
| | | | | rs173945 | INTERGENIC | 1 |
| | | | | rs256348 | INTERGENIC | 0,96 |
| | | | | rs159608 | INTERGENIC | 0,96 |
| rs554710 | UPSTREAM | SURF6 | -15721 | | | |
| rs1013120 | INTERGENIC | HS3ST3A1 | 39975 | | | |
| | | | | rs968026 | INTERGENIC | 0,95 |
| | | | | rs11078172 | INTERGENIC | 1 |
| | | | | rs12952261 | INTERGENIC | 0,9 |
| rs649867 | INTRONIC | Q8N7E7_HU MAN | 0 | | | |
| rs6072317 | INTRONIC | ZHX3 | 0 | | | |
| | | | | rs6072268 | INTRONIC | 0,82 |
| | | | | rs17264110 | INTRONIC | 0,82 |
| | | | | rs6072269 | INTRONIC | 0,82 |
| | | | | rs6072275 | INTRONIC | 0,82 |
| | | | | rs6072286 | INTRONIC | 1 |
| | | | | rs2228246 | NON_SYNONYMOUS_CODING | 1 |
| | | | | rs6072299 | DOWNSTREAM | 1 |
| | | | | rs6072300 | 3PRIME UTR | 0,94 |
| | | | | rs6072302 | 3PRIME UTR | 1 |
| | | | | rs2664537 | 3PRIME UTR | 0,82 |
| | | | | rs7261917 | INTRONIC | 1 |
| | | | | rs6065325 | INTRONIC | 1 |
| | | | | rs6065326 | INTRONIC | 1 |
| | | | | rs6072320 | INTRONIC | 1 |
| | | | | rs6072321 | INTRONIC | 1 |
| | | | | rs6072322 | INTRONIC | 1 |
| | | | | rs8121001 | INTRONIC | 0,88 |
| | | | | rs17181845 | UPSTREAM | 0,94 |
| | | | | rs6072338 | UPSTREAM | 0,94 |
| rs4795069 | INTERGENIC | SLFN5 | 14839 | | | |
| rs13403584 | INTERGENIC | CCDC141 | 92384 | | | |
| | | | | rs10171173 | INTERGENIC | 0,86 |
| | | | | rs2200826 | INTERGENIC | 0,9 |
| | | | | rs10497528 | INTERGENIC | 0,81 |
| | | | | rs7574599 | INTERGENIC | 0,9 |
| | | | | rs6747725 | INTERGENIC | 0,9 |
| | | | | rs924800 | INTERGENIC | 0,96 |
| | | | | rs10930847 | INTERGENIC | 0,96 |
| | | | | rs1847420 | INTERGENIC | 0,96 |
| | | | | rs4894072 | INTERGENIC | 0,96 |
| rs6948196 | INTRONIC | RHEB | 0 | | | |
| | | | | rs17713386 | DOWNSTREAM | 1 |
| | | | | rs4726029 | INTRONIC | 1 |
| | | | | rs875588 | INTRONIC | 1 |
| | | | | rs1109089 | INTRONIC | 1 |
| | | | | rs7805967 | INTRONIC | 0,82 |
| | | | | rs17713697 | INTRONIC | 0,9 |
| | | | | rs2074998 | INTRONIC | 1 |
| | | | | rs2074997 | INTRONIC | 1 |
| | | | | rs17713890 | INTRONIC | 1 |
| | | | | rs2299961 | INTRONIC | 0,82 |
| | | | | rs965118 | INTRONIC | 0,97 |
| | | | | rs3753151 | INTRONIC | 0,82 |
| | | | | rs2299965 | INTRONIC | 1 |
| | | | | rs2284264 | INTRONIC | 1 |
| | | | | rs11772458 | INTRONIC | 1 |
| | | | | rs2299967 | INTRONIC | 1 |
| | | | | rs3789817 | INTRONIC | 1 |
| | | | | rs758666 | INTRONIC | 1 |
| | | | | rs736645 | INTRONIC | 1 |
| | | | | rs4298422 | INTRONIC | 1 |
| | | | | rs2374261 | INTRONIC | 1 |
| | | | | rs12112989 | INTRONIC | 1 |
| | | | | rs6980020 | INTRONIC | 1 |
| | | | | rs12112134 | INTRONIC | 1 |
| | | | | rs6943752 | INTRONIC | 0,9 |
| | | | | rs7794922 | UPSTREAM | 0,84 |
| rs2662090 | INTRONIC | SRGAP3 | 0 | | | |
| | | | | rs2670000 | INTRONIC | 0,97 |
| rs7685314 | INTERGENIC | LOC728856 | -28752 | | | |
| | | | | rs6820247 | INTERGENIC | 0,83 |
| | | | | rs7437007 | INTERGENIC | 1 |
| | | | | rs10866319 | INTERGENIC | 0,83 |
| rs2074997 | INTRONIC | RHEB | 0 | | | |
| | | | | rs17713386 | DOWNSTREAM | 1 |
| | | | | rs4726029 | INTRONIC | 1 |
| | | | | rs875588 | INTRONIC | 1 |
| | | | | rs1109089 | INTRONIC | 1 |
| | | | | rs7805967 | INTRONIC | 0,81 |
| | | | | rs17713697 | INTRONIC | 0,9 |
| | | | | rs2074998 | INTRONIC | 1 |
| | | | | rs17713890 | INTRONIC | 1 |
| | | | | rs2299961 | INTRONIC | 0,81 |
| | | | | rs965118 | INTRONIC | 0,97 |
| | | | | rs3753151 | INTRONIC | 0,81 |
| | | | | rs2299965 | INTRONIC | 1 |
| | | | | rs2284264 | INTRONIC | 1 |
| | | | | rs11772458 | INTRONIC | 1 |
| | | | | rs2299967 | INTRONIC | 1 |
| | | | | rs3789817 | INTRONIC | 1 |
| | | | | rs758666 | INTRONIC | 1 |
| | | | | rs736645 | INTRONIC | 1 |
| | | | | rs4298422 | INTRONIC | 1 |
| | | | | rs2374261 | INTRONIC | 1 |
| | | | | rs12112989 | INTRONIC | 1 |
| | | | | rs6980020 | INTRONIC | 1 |
| | | | | rs12112134 | INTRONIC | 1 |
| | | | | rs6948196 | INTRONIC | 1 |
| | | | | rs6943752 | INTRONIC | 0,9 |
| | | | | rs7794922 | UPSTREAM | 0,84 |
| rs6500498 | 3PRIME UTR | Q8N7R2_HUMAN | 0 | | | |
| | | | | rs7185630 | 3PRIME UTR | 0,87 |
| | | | | rs2242166 | 3PRIME UTR | 0,87 |
| | | | | rs2242164 | UPSTREAM | 0,87 |
| | | | | rs6500497 | 3PRIME UTR | 1 |
| rs1204798 | INTRONIC | NT5DC1 | 0 | | | |
| | | | | rs1204842 | INTRONIC | 1 |
| | | | | rs1204843 | INTRONIC | 1 |
| | | | | rs742930 | INTRONIC | 1 |
| | | | | rs7770203 | INTRONIC | 1 |
| | | | | rs1211388 | INTRONIC | 0,83 |
| | | | | rs1204851 | INTRONIC | 1 |
| | | | | rs1209221 | INTRONIC | 1 |
| | | | | rs1204782 | INTRONIC | 1 |
| | | | | rs1204783 | INTRONIC | 1 |
| | | | | rs1204784 | INTRONIC | 0,88 |
| | | | | rs1204785 | INTRONIC | 0,83 |
| | | | | rs1204786 | INTRONIC | 0,94 |
| | | | | rs1204788 | INTRONIC | 1 |
| | | | | rs1204789 | INTRONIC | 0,88 |
| | | | | rs1204794 | INTRONIC | 0,83 |
| | | | | rs1204797 | INTRONIC | 0,88 |
| | | | | rs1204799 | INTRONIC | 0,83 |
| | | | | rs1204800 | INTRONIC | 1 |
| | | | | rs1204801 | INTRONIC | 1 |
| | | | | rs1204802 | INTRONIC | 0,82 |
| | | | | rs1209223 | INTRONIC | 1 |
| | | | | rs1204807 | INTRONIC | 1 |
| | | | | rs1204814 | INTRONIC | 1 |
| | | | | rs926829 | INTRONIC | 1 |
| | | | | rs1204817 | INTRONIC | 0,83 |
| | | | | rs1204818 | INTRONIC | 0,82 |
| rs12143647 | INTRONIC | COG2 | 0 | | | |
| | | | | rs4846999 | INTRONIC | 0,81 |
| | | | | rs12564388 | INTRONIC | 0,81 |
| | | | | rs1887492 | INTRONIC | 0,82 |
| | | | | rs11122569 | INTRONIC | 0,86 |
| | | | | rs11122570 | INTRONIC | 0,82 |
| | | | | rs3736983 | INTRONIC | 0,82 |
| | | | | rs12041242 | INTRONIC | 0,84 |
| | | | | rs12045973 | INTRONIC | 0,84 |
| rs3801033 | INTRONIC | ZNF12 | 0 | | | |
| | | | | rs3801034 | INTRONIC | 0,85 |
| | | | | rs7798471 | INTRONIC | 0,8 |
| rs10997044 | INTRONIC | CTNNA3 | 0 | | | |
| | | | | rs1876334 | INTRONIC | 0,84 |
| | | | | rs10822782 | INTRONIC | 0,95 |
| | | | | rs10822783 | INTRONIC | 0,95 |
| | | | | rs1911479 | INTRONIC | 0,95 |
| | | | | rs10997021 | INTRONIC | 0,95 |
| | | | | rs10822784 | INTRONIC | 0,95 |
| | | | | rs4609495 | INTRONIC | 0,95 |
| | | | | rs4745897 | INTRONIC | 0,95 |
| | | | | rs10762045 | INTRONIC | 0,95 |
| | | | | rs10997032 | INTRONIC | 0,95 |
| | | | | rs12763572 | INTRONIC | 0,95 |
| | | | | rs10822791 | INTRONIC | 0,91 |
| | | | | rs10762046 | INTRONIC | 0,91 |
| | | | | rs10822792 | INTRONIC | 0,95 |
| | | | | rs9804181 | INTRONIC | 0,95 |
| | | | | rs10997035 | INTRONIC | 0,95 |
| | | | | rs10997037 | INTRONIC | 0,95 |
| | | | | rs10822794 | INTRONIC | 0,95 |
| | | | | rs10762051 | INTRONIC | 0,91 |
| | | | | rs12357109 | INTRONIC | 0,95 |
| | | | | rs1911485 | INTRONIC | 0,81 |
| | | | | rs1911486 | INTRONIC | 1 |
| | | | | rs10997048 | INTRONIC | 1 |
| | | | | rs10822801 | INTRONIC | 0,95 |
| | | | | rs4523585 | INTRONIC | 0,95 |
| rs7350731 | INTERGENIC | N/A | -9 | | | |
| rs9323737 | INTERGENIC | N/A | -9 | | | |
| | | | | rs1958438 | INTERGENIC | 0,92 |
| | | | | rs10139321 | INTERGENIC | 0,92 |
| | | | | rs10132490 | INTERGENIC | 0,89 |
| | | | | rs10143458 | INTERGENIC | 0,91 |
| | | | | rs9671273 | INTERGENIC | 0,92 |
| | | | | rs1952534 | INTERGENIC | 0,92 |
| | | | | rs1958436 | INTERGENIC | 0,91 |
| | | | | rs11159605 | INTERGENIC | 0,95 |
| | | | | rs7359138 | INTERGENIC | 0,8 |
| | | | | rs1958435 | INTERGENIC | 0,92 |
| | | | | rs1958434 | INTERGENIC | 0,91 |
| | | | | rs1958433 | INTERGENIC | 0,92 |
| | | | | rs2149684 | INTERGENIC | 0,87 |
| | | | | rs2183203 | INTERGENIC | 0,91 |
| | | | | rs1958432 | INTERGENIC | 0,91 |
| | | | | rs2149683 | INTERGENIC | 0,92 |
| | | | | rs8010141 | INTERGENIC | 0,92 |
| | | | | rs1958430 | INTERGENIC | 0,92 |
| | | | | rs1958429 | INTERGENIC | 0,92 |
| | | | | rs1958428 | INTERGENIC | 0,92 |
| | | | | rs1958426 | INTERGENIC | 1 |
| | | | | rs1958425 | INTERGENIC | 0,92 |
| | | | | rs1958424 | INTERGENIC | 1 |
| | | | | rs1958423 | INTERGENIC | 1 |
| | | | | rs1958422 | INTERGENIC | 1 |
| | | | | rs10132081 | INTERGENIC | 1 |
| | | | | rs10132130 | INTERGENIC | 1 |
| | | | | rs10147226 | INTERGENIC | 1 |
| | | | | rs1958421 | INTERGENIC | 1 |
| | | | | rs1958420 | INTERGENIC | 1 |
| | | | | rs10873350 | INTERGENIC | 1 |
| | | | | rs10140080 | INTERGENIC | 1 |
| | | | | rs4545730 | INTERGENIC | 1 |
| | | | | rs1952530 | INTERGENIC | 1 |
| | | | | rs1952529 | INTERGENIC | 1 |
| | | | | rs12431659 | INTERGENIC | 1 |
| | | | | rs12434622 | INTERGENIC | 1 |
| | | | | rs10141403 | INTERGENIC | 1 |
| | | | | rs9323738 | INTERGENIC | 1 |
| | | | | rs9323739 | INTERGENIC | 1 |
| | | | | rs1952528 | INTERGENIC | 0,92 |
| | | | | rs1958418 | INTERGENIC | 1 |
| | | | | rs877800 | INTERGENIC | 1 |
| | | | | rs2372550 | INTERGENIC | 0,91 |
| | | | | rs1952545 | INTERGENIC | 1 |
| | | | | rs1952544 | INTERGENIC | 1 |
| | | | | rs10747302 | INTERGENIC | 1 |
| | | | | rs4899838 | INTERGENIC | 0,92 |
| | | | | rs11159610 | INTERGENIC | 0,9 |
| | | | | rs11159611 | INTERGENIC | 0,92 |
| | | | | rs7494095 | INTERGENIC | 0,92 |
| | | | | rs10132137 | INTERGENIC | 0,92 |
| | | | | rs4899839 | INTERGENIC | 0,92 |
| | | | | rs10135095 | INTERGENIC | 0,92 |
| | | | | rs2888357 | INTERGENIC | 0,92 |
| | | | | rs4143911 | INTERGENIC | 0,92 |
| | | | | rs1958458 | INTERGENIC | 0,91 |
| | | | | rs2372553 | INTERGENIC | 0,92 |
| | | | | rs10147836 | INTERGENIC | 0,87 |
| | | | | rs10139812 | INTERGENIC | 0,91 |
| | | | | rs1958456 | INTERGENIC | 0,92 |
| | | | | rs1958455 | INTERGENIC | 0,92 |
| | | | | rs2372523 | INTERGENIC | 0,87 |
| | | | | rs2372524 | INTERGENIC | 0,92 |
| | | | | rs10143348 | INTERGENIC | 0,92 |
| | | | | rs9323740 | INTERGENIC | 0,82 |
| | | | | rs10130591 | INTERGENIC | 0,87 |
| | | | | rs1958449 | INTERGENIC | 0,87 |
| rs4881394 | INTERGENIC | AKR1C3 | -22872 | | | |
| | | | | rs4525119 | INTERGENIC | 0,8 |
| | | | | rs4881388 | INTERGENIC | 0,8 |
| | | | | rs10795234 | INTERGENIC | 0,96 |
| | | | | rs7074522 | INTERGENIC | 0,96 |
| | | | | rs10795241 | INTERGENIC | 0,8 |
| | | | | rs2096422 | INTERGENIC | 0,85 |
| rs4799159 | INTERGENIC | SALL3 | -459812 | | | |
| | | | | rs185750 | INTERGENIC | 0,85 |
| | | | | rs2578210 | INTERGENIC | 0,85 |
| | | | | rs12456117 | INTERGENIC | 0,93 |
| | | | | rs7506508 | INTERGENIC | 0,93 |
| | | | | rs9952440 | INTERGENIC | 1 |
| rs356999 | INTERGENIC | BCL11A | 30951 | | | |
| | | | | rs357003 | INTERGENIC | 1 |
| | | | | rs357002 | INTERGENIC | 1 |
| | | | | rs356998 | INTERGENIC | 1 |
| rs2270101 | INTERGENIC | FAM126A | -47256 | | | |
| | | | | rs1001027 | INTERGENIC | 0,96 |
| | | | | rs1001026 | INTERGENIC | 0,96 |
| | | | | rs13227316 | INTERGENIC | 0,96 |
| | | | | rs11765548 | INTERGENIC | 0,96 |
| | | | | rs12700402 | INTERGENIC | 0,96 |
| rs618670 | INTERGENIC | N/A | -9 | | | |
| rs7788668 | DOWNSTREA M | FAM126A | -2622 | | | |
| | | | | rs2286497 | INTERGENIC | 0,92 |
| | | | | rs2033670 | INTERGENIC | 0,92 |
| | | | | rs13227654 | INTERGENIC | 0,83 |
| | | | | rs11561822 | INTERGENIC | 0,96 |
| | | | | rs11764386 | DOWNSTREAM | 1 |
| | | | | rs10488277 | 3PRIME UTR | 0,96 |
| | | | | rs2286493 | 3PRIME UTR | 0,96 |
| | | | | rs2286491 | INTRONIC | 1 |
| | | | | rs2286490 | INTRONIC | 1 |
| | | | | rs11772725 | INTRONIC | 0,95 |
| | | | | rs11772749 | INTRONIC | 1 |
| | | | | rs11764613 | INTRONIC | 0,83 |
| | | | | rs11768060 | INTRONIC | 0,82 |
| | | | | rs17147527 | INTRONIC | 1 |
| | | | | rs17147529 | INTRONIC | 0,85 |
| | | | | rs11762669 | INTRONIC | 0,88 |
| | | | | rs13225964 | INTRONIC | 0,83 |
| | | | | rs11771543 | INTERGENIC | 0,96 |
| | | | | rs17370052 | INTERGENIC | 0,96 |
| | | | | rs9647996 | INTERGENIC | 0,96 |
| | | | | rs12674362 | INTERGENIC | 0,81 |
| | | | | rs13230424 | INTERGENIC | 0,84 |
| | | | | rs10950935 | INTERGENIC | 0,88 |
| rs902923 | INTRONIC | ROR2 | 0 | | | |
| | | | | rs10991949 | INTERGENIC | 0,88 |
| | | | | rs768056 | INTERGENIC | 0,94 |
| | | | | rs1412466 | INTERGENIC | 0,88 |
| | | | | rs16907585 | INTERGENIC | 0,89 |
| | | | | rs7855644 | INTERGENIC | 0,89 |
| | | | | rs1492679 | INTERGENIC | 1 |
| | | | | rs2131301 | DOWNSTREAM | 1 |
| | | | | rs9409427 | INTRONIC | 1 |
| rs9332172 | INTRONIC | CYP2C9 | 0 | | | |
| | | | | rs17521564 | INTERGENIC | 1 |
| | | | | rs7893293 | INTERGENIC | 0,9 |
| | | | | rs2860905 | INTRONIC | 0,9 |
| | | | | rs4086116 | INTRONIC | 1 |
| | | | | rs4917639 | INTRONIC | 1 |
| | | | | rs1934963 | INTRONIC | 1 |
| | | | | rs4918797 | DOWNSTREA M | 1 |
| rs6873640 | INTERGENIC | ZNF608 | -56628 | | | |
| | | | | rs7725329 | INTERGENIC | 0,97 |
| rs9302822 | INTERGENIC | Q8N9J9_HU MAN | 114376 | | | |
| | | | | rs17539244 | INTERGENIC | 0,84 |
| | | | | rs1019191 | INTERGENIC | 0,84 |
| | | | | rs17442566 | INTERGENIC | 0,84 |
| | | | | rs17442866 | INTERGENIC | 0,81 |
| | | | | rs8050684 | INTERGENIC | 0,81 |
| | | | | rs17540392 | INTERGENIC | 0,81 |
| rs4810622 | INTRONIC | ZMYND8 | 0 | | | |
| | | | | rs4809630 | INTRONIC | 0,93 |
| | | | | rs13038759 | INTRONIC | 0,82 |
| | | | | rs761021 | INTRONIC | 0,82 |
| | | | | rs6124987 | INTRONIC | 1 |
| | | | | rs3803941 | INTRONIC | 1 |
| | | | | rs6124990 | INTRONIC | 0,92 |
| rs3753151 | INTRONIC | RHEB | 0 | | | |
| | | | | rs17713386 | DOWNSTREA M | 0,84 |
| | | | | rs4726029 | INTRONIC | 0,82 |
| | | | | rs875588 | INTRONIC | 0,82 |
| | | | | rs1109089 | INTRONIC | 0,83 |
| | | | | rs7805967 | INTRONIC | 1 |
| | | | | rs2074998 | INTRONIC | 0,84 |
| | | | | rs2074997 | INTRONIC | 0,81 |
| | | | | rs17713890 | INTRONIC | 0,82 |
| | | | | rs2299961 | INTRONIC | 1 |
| | | | | rs2299962 | INTRONIC | 0,9 |
| | | | | rs2299965 | INTRONIC | 0,82 |
| | | | | rs2284264 | INTRONIC | 0,82 |
| | | | | rs2299967 | INTRONIC | 0,84 |
| | | | | rs3789817 | INTRONIC | 0,82 |
| | | | | rs758666 | INTRONIC | 0,82 |
| | | | | rs736645 | INTRONIC | 0,82 |
| | | | | rs4298422 | INTRONIC | 0,84 |
| | | | | rs2374261 | INTRONIC | 0,82 |
| | | | | rs12112989 | INTRONIC | 0,82 |
| | | | | rs6980020 | INTRONIC | 0,82 |
| | | | | rs12112134 | INTRONIC | 0,81 |
| | | | | rs6948196 | INTRONIC | 0,82 |
| rs10490832 | SYNONYMOUS_CODING | CNTN3 | 0 | | | |
| rs1545384 | INTRONIC | CNTN3 | 0 | | | |
| | | | | rs1809043 | INTRONIC | 0,9 |
| | | | | rs2197742 | INTRONIC | 0,95 |
| rs1884641 | INTERGENIC | C20orf75 | 5233 | | | |
| | | | | rs6117052 | UPSTREAM | 1 |
| | | | | rs6076925 | INTERGENIC | 0,81 |
| rs1958438 | INTERGENIC | N/A | -9 | | | |
| | | | | rs10139321 | INTERGENIC | 1 |
| | | | | rs10132490 | INTERGENIC | 1 |
| | | | | rs10143458 | INTERGENIC | 1 |
| | | | | rs9671273 | INTERGENIC | 1 |
| | | | | rs1952534 | INTERGENIC | 1 |
| | | | | rs1958436 | INTERGENIC | 1 |
| | | | | rs11159605 | INTERGENIC | 1 |
| | | | | rs7359138 | INTERGENIC | 0,9 |
| | | | | rs1958435 | INTERGENIC | 1 |
| | | | | rs1958434 | INTERGENIC | 1 |
| | | | | rs1958433 | INTERGENIC | 1 |
| | | | | rs2149684 | INTERGENIC | 0,96 |
| | | | | rs2183203 | INTERGENIC | 1 |
| | | | | rs1958432 | INTERGENIC | 1 |
| | | | | rs2149683 | INTERGENIC | 1 |
| | | | | rs8010141 | INTERGENIC | 1 |
| | | | | rs1958430 | INTERGENIC | 1 |
| | | | | rs1958429 | INTERGENIC | 1 |
| | | | | rs1958428 | INTERGENIC | 1 |
| | | | | rs1958426 | INTERGENIC | 0,92 |
| | | | | rs1958425 | INTERGENIC | 1 |
| | | | | rs1958424 | INTERGENIC | 0,92 |
| | | | | rs1958423 | INTERGENIC | 0,92 |
| | | | | rs1958422 | INTERGENIC | 0,92 |
| | | | | rs10132081 | INTERGENIC | 0,92 |
| | | | | rs10132130 | INTERGENIC | 0,91 |
| | | | | rs10147226 | INTERGENIC | 0,91 |
| | | | | rs1958421 | INTERGENIC | 0,92 |
| | | | | rs1958420 | INTERGENIC | 0,92 |
| | | | | rs10873350 | INTERGENIC | 0,92 |
| | | | | rs10140080 | INTERGENIC | 0,92 |
| | | | | rs9323737 | INTERGENIC | 0,92 |
| | | | | rs4545730 | INTERGENIC | 0,92 |
| | | | | rs1952530 | INTERGENIC | 0,92 |
| | | | | rs1952529 | INTERGENIC | 0,92 |
| | | | | rs12431659 | INTERGENIC | 0,91 |
| | | | | rs12434622 | INTERGENIC | 0,95 |
| | | | | rs10141403 | INTERGENIC | 0,91 |
| | | | | rs9323738 | INTERGENIC | 0,92 |
| | | | | rs9323739 | INTERGENIC | 0,92 |
| | | | | rs1952528 | INTERGENIC | 0,84 |
| | | | | rs1958418 | INTERGENIC | 0,92 |
| | | | | rs877800 | INTERGENIC | 0,91 |
| | | | | rs2372550 | INTERGENIC | 0,82 |
| | | | | rs1952545 | INTERGENIC | 0,92 |
| | | | | rs1952544 | INTERGENIC | 0,92 |
| | | | | rs10747302 | INTERGENIC | 0,95 |
| | | | | rs4899838 | INTERGENIC | 1 |
| | | | | rs11159610 | INTERGENIC | 1 |
| | | | | rs11159611 | INTERGENIC | 1 |
| | | | | rs7494095 | INTERGENIC | 1 |
| | | | | rs10132137 | INTERGENIC | 1 |
| | | | | rs4899839 | INTERGENIC | 1 |
| | | | | rs10135095 | INTERGENIC | 1 |
| | | | | rs2888357 | INTERGENIC | 1 |
| | | | | rs4143911 | INTERGENIC | 1 |
| | | | | rs1958458 | INTERGENIC | 1 |
| | | | | rs2372553 | INTERGENIC | 1 |
| | | | | rs10147836 | INTERGENIC | 0,95 |
| | | | | rs10139812 | INTERGENIC | 1 |
| | | | | rs1958456 | INTERGENIC | 1 |
| | | | | rs1958455 | INTERGENIC | 1 |
| | | | | rs2372523 | INTERGENIC | 0,95 |
| | | | | rs2372524 | INTERGENIC | 1 |
| | | | | rs10143348 | INTERGENIC | 1 |
| | | | | rs9323740 | INTERGENIC | 0,91 |
| | | | | rs10130591 | INTERGENIC | 0,95 |
| | | | | rs12894765 | INTERGENIC | 0,87 |
| | | | | rs7157077 | INTERGENIC | 0,87 |
| | | | | rs4904138 | INTERGENIC | 0,87 |
| | | | | rs1952541 | INTERGENIC | 0,87 |
| | | | | rs1958451 | INTERGENIC | 0,87 |
| | | | | rs12882544 | INTERGENIC | 0,87 |
| | | | | rs1958449 | INTERGENIC | 0,95 |
| | | | | rs1952538 | INTERGENIC | 0,87 |
| rs4917639 | INTRONIC | CYP2C9 | 0 | | | |
| | | | | rs17521564 | INTERGENIC | 1 |
| | | | | rs7893293 | INTERGENIC | 0,91 |
| | | | | rs2860905 | INTRONIC | 0,91 |
| | | | | rs4086116 | INTRONIC | 1 |
| | | | | rs9332172 | INTRONIC | 1 |
| | | | | rs1934963 | INTRONIC | 1 |
| | | | | rs4918797 | DOWNSTREA M | 1 |
| rs2279103 | NON_SYNON YMOUS_CODI NG | CTDP1 | 0 | | | |
| | | | | rs3809939 | INTRONIC | 0,86 |
| | | | | rs8098133 | INTRONIC | 0,95 |
| | | | | rs3859315 | INTRONIC | 1 |
| | | | | rs3859316 | INTRONIC | 1 |
| | | | | rs898619 | INTRONIC | 1 |
| | | | | rs3809936 | INTRONIC | 1 |
| | | | | rs3786235 | INTRONIC | 1 |
| | | | | rs8087647 | INTRONIC | 1 |
| | | | | rs8084175 | INTRONIC | 1 |
| | | | | rs9953991 | INTRONIC | 0,82 |
| | | | | rs9946977 | 3PRIME UTR | 1 |
| | | | | rs12605690 | DOWNSTREAM | 0,82 |
| | | | | rs554659 | INTERGENIC | 0,82 |
| | | | | rs551017 | INTERGENIC | 0,82 |
| | | | | rs665138 | INTERGENIC | 0,82 |
| | | | | rs523386 | INTERGENIC | 1 |
| | | | | rs652717 | INTERGENIC | 0,82 |
| | | | | rs496036 | INTERGENIC | 1 |
| | | | | rs576937 | INTERGENIC | 0,81 |
| | | | | rs621490 | INTERGENIC | 0,95 |
| rs7307064 | INTRONIC | RECQL | 0 | | | |
| | | | | rs7307519 | INTRONIC | 1 |
| | | | | rs7976409 | INTRONIC | 1 |
| | | | | rs7976415 | INTRONIC | 1 |
| | | | | rs7956315 | INTRONIC | 1 |
| | | | | rs17627102 | INTRONIC | 0,85 |
| | | | | rs7310464 | INTRONIC | 0,91 |
| | | | | rs1061626 | 5PRIME UTR | 0,86 |
| rs6793017 | INTRONIC | CNTN4 | 0 | | | |
| | | | | rs6763008 | INTRONIC | 0,88 |
| | | | | rs6774320 | INTRONIC | 0,95 |
| rs523386 | INTERGENIC | Q6ZVY3_HU MAN | 10572 | | | |
| | | | | rs3809939 | INTRONIC | 0,86 |
| | | | | rs8098133 | INTRONIC | 0,95 |
| | | | | rs3859315 | INTRONIC | 1 |
| | | | | rs3859316 | INTRONIC | 1 |
| | | | | rs898619 | INTRONIC | 1 |
| | | | | rs3809936 | INTRONIC | 1 |
| | | | | rs3786235 | INTRONIC | 1 |
| | | | | rs8087647 | INTRONIC | 1 |
| | | | | rs2279103 | NON_SYNONYMOUS_CODING | 1 |
| | | | | rs8084175 | INTRONIC | 1 |
| | | | | rs9953991 | INTRONIC | 0,82 |
| | | | | rs9946977 | 3PRIME UTR | 1 |
| | | | | | | |
| | | | | rs12605690 | DOWNSTREAM | 0,82 |
| | | | | rs554659 | INTERGENIC | 0,82 |
| | | | | rs551017 | INTERGENIC | 0,82 |
| | | | | rs665138 | INTERGENIC | 0,82 |
| | | | | rs652717 | INTERGENIC | 0,82 |
| | | | | rs496036 | INTERGENIC | 1 |
| | | | | rs576937 | INTERGENIC | 0,81 |
| | | | | rs621490 | INTERGENIC | 0,95 |
| rs7719325 | INTERGENIC | ZNF608 | -493552 | | | |
| | | | | rs17151230 | INTERGENIC | 0,95 |
| | | | | rs17151248 | INTERGENIC | 1 |
| rs1984151 | INTERGENIC | EPHA7 | 399827 | | | |
| | | | | rs2325528 | INTERGENIC | 1 |
| | | | | rs9452447 | INTERGENIC | 1 |
| | | | | rs9294577 | INTERGENIC | 1 |
| | | | | rs2152543 | INTERGENIC | 1 |
| | | | | rs4707810 | INTERGENIC | 1 |
| | | | | rs6904831 | INTERGENIC | 0,96 |
| | | | | rs2000361 | INTERGENIC | 0,95 |
| | | | | rs7765999 | INTERGENIC | 0,85 |
| | | | | rs6925802 | INTERGENIC | 0,83 |
| | | | | rs9294582 | INTERGENIC | 0,85 |
| | | | | rs7752145 | INTERGENIC | 0,88 |
| | | | | rs1238901 | INTERGENIC | 0,85 |
| | | | | rs1219036 | INTERGENIC | 0,82 |
| | | | | rs1219037 | INTERGENIC | 0,81 |
| | | | | rs2248260 | INTERGENIC | 0,85 |
| | | | | rs2801552 | INTERGENIC | 0,82 |
| | | | | rs2801556 | INTERGENIC | 0,82 |
| | | | | rs2633619 | INTERGENIC | 0,82 |
| | | | | rs2801559 | INTERGENIC | 0,82 |
| | | | | rs1601856 | INTERGENIC | 0,82 |
| rs1454333 | INTERGENIC | TMSL3 | 82867 | | | |
| | | | | rs17017774 | INTERGENIC | 0,93 |
| | | | | rs1377917 | INTERGENIC | 0,93 |
| rs11250017 | INTERGENIC | Q6ZVI4_HUMAN | -12575 | | | |
| | | | | rs10095339 | DOWNSTREAM | 0,83 |
| | | | | rs10103466 | DOWNSTREA M | 0,96 |
| rs7146332 | INTERGENIC | LRFN5 | -298872 | | | |
| | | | | rs8005889 | INTERGENIC | 0,97 |
| | | | | rs4128092 | INTERGENIC | 0,96 |
| | | | | rs7161171 | INTERGENIC | 0,97 |
| | | | | rs6572078 | INTERGENIC | 0,88 |
| | | | | rs3866730 | INTERGENIC | 1 |
| | | | | rs6572079 | INTERGENIC | 1 |
| | | | | rs8019332 | INTERGENIC | 1 |
| | | | | rs4128087 | INTERGENIC | 1 |
| | | | | rs11628088 | INTERGENIC | 1 |
| | | | | rs12892392 | INTERGENIC | 1 |
| | | | | rs1431032 | INTERGENIC | 1 |
| rs17175096 | INTERGENIC | N/A | -9 | | | |
| | | | | rs12960630 | INTERGENIC | 0,89 |
| | | | | rs17260406 | INTERGENIC | 0,94 |
| | | | | rs17074095 | INTERGENIC | 1 |
| rs720733 | INTERGENIC | PCDH18 | -443488 | | | |
| | | | | rs6825957 | INTERGENIC | 1 |
| | | | | rs1914603 | INTERGENIC | 1 |
| | | | | rs10857200 | INTERGENIC | 1 |
| | | | | rs10857201 | INTERGENIC | 1 |
| | | | | rs6825255 | INTERGENIC | 1 |
| | | | | rs6853959 | INTERGENIC | 1 |
| | | | | rs4864386 | INTERGENIC | 1 |
| rs2299965 | INTRONIC | RHEB | 0 | | | |
| | | | | rs17713386 | DOWNSTREAM | 1 |
| | | | | rs4726029 | INTRONIC | 1 |
| | | | | rs875588 | INTRONIC | 1 |
| | | | | rs1109089 | INTRONIC | 1 |
| | | | | rs7805967 | INTRONIC | 0,82 |
| | | | | rs17713697 | INTRONIC | 0,9 |
| | | | | rs2074998 | INTRONIC | 1 |
| | | | | rs2074997 | INTRONIC | 1 |
| | | | | rs17713890 | INTRONIC | 1 |
| | | | | rs2299961 | INTRONIC | 0,82 |
| | | | | rs965118 | INTRONIC | 0,97 |
| | | | | rs3753151 | INTRONIC | 0,82 |
| | | | | rs2284264 | INTRONIC | 1 |
| | | | | rs11772458 | INTRONIC | 1 |
| | | | | rs2299967 | INTRONIC | 1 |
| | | | | rs3789817 | INTRONIC | 1 |
| | | | | rs758666 | INTRONIC | 1 |
| | | | | rs736645 | INTRONIC | 1 |
| | | | | rs4298422 | INTRONIC | 1 |
| | | | | rs2374261 | INTRONIC | 1 |
| | | | | rs12112989 | INTRONIC | 1 |
| | | | | rs6980020 | INTRONIC | 1 |
| | | | | rs12112134 | INTRONIC | 1 |
| | | | | rs6948196 | INTRONIC | 1 |
| | | | | rs6943752 | INTRONIC | 0,9 |
| | | | | rs7794922 | UPSTREAM | 0,84 |
| rs2299967 | INTRONIC | RHEB | 0 | | | |
| | | | | rs17713386 | DOWNSTREAM | 1 |
| | | | | rs4726029 | INTRONIC | 1 |
| | | | | rs875588 | INTRONIC | 1 |
| | | | | rs1109089 | INTRONIC | 1 |
| | | | | rs7805967 | INTRONIC | 0,84 |
| | | | | rs17713697 | INTRONIC | 0,9 |
| | | | | rs2074998 | INTRONIC | 1 |
| | | | | rs2074997 | INTRONIC | 1 |
| | | | | rs17713890 | INTRONIC | 1 |
| | | | | rs2299961 | INTRONIC | 0,84 |
| | | | | rs965118 | INTRONIC | 0,97 |
| | | | | rs3753151 | INTRONIC | 0,84 |
| | | | | rs2299965 | INTRONIC | 1 |
| | | | | rs2284264 | INTRONIC | 1 |
| | | | | rs11772458 | INTRONIC | 1 |
| | | | | rs3789817 | INTRONIC | 1 |
| | | | | rs758666 | INTRONIC | 1 |
| | | | | rs736645 | INTRONIC | 1 |
| | | | | rs4298422 | INTRONIC | 1 |
| | | | | rs2374261 | INTRONIC | 1 |
| | | | | rs12112989 | INTRONIC | 1 |
| | | | | rs6980020 | INTRONIC | 1 |
| | | | | rs12112134 | INTRONIC | 1 |
| | | | | rs6948196 | INTRONIC | 1 |
| | | | | rs6943752 | INTRONIC | 0,9 |
| | | | | rs7794922 | UPSTREAM | 0,84 |
| rs7550277 | INTRONIC | RGS7 | 0 | | | |
| | | | | rs11589937 | INTRONIC | 0,86 |
| | | | | rs1110128 | INTRONIC | 0,86 |
| | | | | rs6702310 | INTRONIC | 1 |
| | | | | rs11583779 | INTRONIC | 1 |
| | | | | rs11584248 | INTRONIC | 1 |
| | | | | rs1996805 | INTRONIC | 1 |
| | | | | rs11587158 | INTRONIC | 1 |
| | | | | rs11584771 | INTRONIC | 1 |
| | | | | rs10926454 | INTRONIC | 1 |
| | | | | rs7539742 | INTRONIC | 1 |
| | | | | rs6429256 | INTRONIC | 1 |
| | | | | rs6684760 | INTRONIC | 1 |
| rs635546 | INTERGENIC | L3MBTL4 | -16980 | | | |
| rs301191 | INTERGENIC | N/A | -9 | | | |
| | | | | rs177295 | INTERGENIC | 1 |
| | | | | rs301193 | INTERGENIC | 1 |
| | | | | rs2062246 | INTERGENIC | 1 |
| | | | | rs4857681 | INTERGENIC | 1 |
| | | | | rs4857686 | INTERGENIC | 1 |
| | | | | rs11710431 | INTERGENIC | 1 |
| | | | | rs4857698 | INTERGENIC | 1 |
| | | | | rs13091297 | INTERGENIC | 1 |
| | | | | rs9798968 | INTERGENIC | 0,86 |
| | | | | rs11922088 | INTERGENIC | 1 |
| rs301193 | INTERGENIC | N/A | -9 | | | |
| | | | | rs177295 | INTERGENIC | 1 |
| | | | | rs301191 | INTERGENIC | 1 |
| | | | | rs2062246 | INTERGENIC | 1 |
| | | | | rs4857681 | INTERGENIC | 1 |
| | | | | rs4857686 | INTERGENIC | 1 |
| | | | | rs11710431 | INTERGENIC | 1 |
| | | | | rs4857698 | INTERGENIC | 1 |
| | | | | rs13091297 | INTERGENIC | 1 |
| | | | | rs9798968 | INTERGENIC | 0,86 |
| | | | | rs11922088 | INTERGENIC | 1 |
| rs12583395 | INTERGENIC | NBEA | -296678 | | | |
| rs4388301 | INTERGENIC | TNFAIP3 | 77933 | | | |
| rs4939517 | INTRONIC | CYBASC3 | 0 | | | |
| | | | | rs7111608 | INTRONIC | 1 |
| | | | | rs11230659 | INTRONIC | 1 |
| | | | | rs6591651 | INTRONIC | 1 |
| | | | | rs1037448 | INTRONIC | 1 |
| | | | | rs10750955 | UPSTREAM | 1 |
| | | | | rs3741265 | SYNONYMOUS_CODING | 0,94 |
| | | | | rs10897158 | INTRONIC | 0,94 |
| | | | | rs921635 | INTRONIC | 0,94 |
| | | | | rs3018727 | INTRONIC | 0,94 |
| | | | | rs3019198 | SYNONYMOUS_CODING | 0,93 |
| | | | | rs2860519 | INTRONIC | 0,93 |
| | | | | rs3809083 | INTRONIC | 0,94 |
| | | | | rs2943807 | INTRONIC | 0,94 |
| | | | | rs2943806 | INTRONIC | 0,87 |
| | | | | rs896831 | INTRONIC | 0,87 |
| | | | | rs879647 | INTRONIC | 0,87 |
| | | | | rs3017602 | INTRONIC | 0,87 |
| | | | | rs2943805 | INTRONIC | 0,87 |
| | | | | rs17702 | 3PRIME UTR | 0,87 |
| | | | | rs1377456 | DOWNSTREAM | 0,87 |
| | | | | rs720888 | DOWNSTREAM | 0,84 |
| | | | | rs720891 | DOWNSTREAM | 0,87 |
| | | | | rs6591654 | DOWNSTREAM | 0,87 |
| | | | | rs6591655 | INTERGENIC | 0,87 |
| | | | | rs729404 | INTERGENIC | 0,87 |
| | | | | rs729347 | INTERGENIC | 0,87 |
| | | | | rs3018729 | INTERGENIC | 0,87 |
| | | | | rs2924436 | INTERGENIC | 0,87 |
| | | | | rs11230701 | INTERGENIC | 0,87 |
| | | | | rs3019186 | INTERGENIC | 0,87 |
| | | | | rs896829 | INTERGENIC | 0,87 |
| | | | | rs748902 | INTERGENIC | 0,94 |
| | | | | rs730338 | INTERGENIC | 0,85 |
| | | | | rs2943800 | UPSTREAM | 0,94 |
| | | | | rs2957860 | UPSTREAM | 0,93 |
| | | | | rs2924441 | UPSTREAM | 0,94 |
| | | | | rs2924446 | DOWNSTREA M | 0,87 |
| | | | | rs3017605 | DOWNSTREA M | 0,81 |
| | | | | rs2100388 | INTERGENIC | 0,87 |
| rs1037448 | INTRONIC | TMEM138 | 0 | | | |
| | | | | rs7111608 | INTRONIC | 1 |
| | | | | rs11230659 | INTRONIC | 1 |
| | | | | rs6591651 | INTRONIC | 1 |
| | | | | rs4939517 | INTRONIC | 1 |
| | | | | rs10750955 | UPSTREAM | 1 |
| | | | | rs3741265 | SYNONYMOUS_CODING | 0,94 |
| | | | | rs10897158 | INTRONIC | 0,94 |
| | | | | rs921635 | INTRONIC | 0,94 |
| | | | | rs3018727 | INTRONIC | 0,94 |
| | | | | rs3019198 | SYNONYMOUS_CODING | 0,94 |
| | | | | rs2860519 | INTRONIC | 0,94 |
| | | | | rs3809083 | INTRONIC | 0,94 |
| | | | | rs2943807 | INTRONIC | 0,94 |
| | | | | rs2943806 | INTRONIC | 0,87 |
| | | | | rs896831 | INTRONIC | 0,88 |
| | | | | rs879647 | INTRONIC | 0,87 |
| | | | | rs3017602 | INTRONIC | 0,88 |
| | | | | rs2943805 | INTRONIC | 0,88 |
| | | | | rs17702 | 3PRIME UTR | 0,87 |
| | | | | rs1377456 | DOWNSTREA M | 0,88 |
| | | | | rs720888 | DOWNSTREA M | 0,84 |
| | | | | rs720891 | DOWNSTREA M | 0,88 |
| | | | | rs6591654 | DOWNSTREA M | 0,88 |
| | | | | rs6591655 | INTERGENIC | 0,88 |
| | | | | rs729404 | INTERGENIC | 0,88 |
| | | | | rs729347 | INTERGENIC | 0,87 |
| | | | | rs3018729 | INTERGENIC | 0,88 |
| | | | | rs2924436 | INTERGENIC | 0,88 |
| | | | | rs11230701 | INTERGENIC | 0,88 |
| | | | | rs3019186 | INTERGENIC | 0,88 |
| | | | | rs896829 | INTERGENIC | 0,88 |
| | | | | rs748902 | INTERGENIC | 0,94 |
| | | | | rs730338 | INTERGENIC | 0,86 |
| | | | | rs2943800 | UPSTREAM | 0,94 |
| | | | | rs2957860 | UPSTREAM | 0,94 |
| | | | | rs2924441 | UPSTREAM | 0,94 |
| | | | | rs2924446 | DOWNSTREA M | 0,88 |
| | | | | rs3017605 | DOWNSTREA M | 0,82 |
| | | | | rs2100388 | INTERGENIC | 0,88 |
| rs2348427 | INTRONIC | ENPEP | 0 | | | |
| | | | | rs1126483 | NON_SYNONYMOUS_CODING | 1 |
| | | | | rs12503640 | INTRONIC | 1 |
| | | | | rs2881913 | INTRONIC | 1 |
| | | | | rs2348429 | INTRONIC | 1 |
| | | | | rs17551888 | INTRONIC | 1 |
| | | | | rs6842486 | INTRONIC | 1 |
| | | | | rs10015807 | INTRONIC | 1 |
| | | | | rs12506732 | INTRONIC | 0,96 |
| | | | | rs2348431 | INTRONIC | 0,96 |
| | | | | rs6813802 | INTRONIC | 0,96 |
| | | | | rs3796889 | INTRONIC | 0,96 |
| | | | | rs3796888 | INTRONIC | 1 |
| | | | | rs2348433 | INTRONIC | 1 |
| | | | | rs1448808 | INTRONIC | 0,8 |
| rs1891877 | INTRONIC | PIP4K2A | 0 | | | |
| rs8095186 | INTERGENIC | PIK3C3 | 251736 | | | |
| | | | | rs346457 | INTERGENIC | 0,92 |
| rs9480684 | INTRONIC | AIM1 | 0 | | | |
| | | | | rs1770728 | INTRONIC | 0,93 |
| | | | | rs1770731 | INTRONIC | 0,91 |
| | | | | rs1770732 | INTRONIC | 0,93 |
| | | | | rs1676016 | INTRONIC | 0,93 |
| | | | | rs2297971 | INTRONIC | 0,93 |
| | | | | rs2066202 | INTRONIC | 0,86 |
| | | | | rs2054366 | INTRONIC | 1 |
| | | | | rs2615206 | INTRONIC | 1 |
| | | | | rs2615207 | INTRONIC | 1 |
| | | | | rs2642469 | INTRONIC | 1 |
| | | | | rs1037955 | INTRONIC | 1 |
| | | | | rs965347 | INTRONIC | 1 |
| | | | | rs4946764 | INTRONIC | 1 |
| | | | | rs9486398 | INTRONIC | 1 |
| | | | | rs9486399 | INTRONIC | 1 |
| | | | | rs9480685 | INTRONIC | 1 |
| | | | | rs9486403 | INTRONIC | 1 |
| | | | | rs9480686 | INTRONIC | 1 |
| | | | | rs9486404 | INTRONIC | 1 |
| | | | | rs3747790 | 3PRIME UTR | 0,93 |
| | | | | rs13319 | 3PRIME UTR | 1 |
| | | | | rs9320182 | 3PRIME UTR | 1 |
| | | | | rs9320183 | 3PRIME UTR | 1 |
| | | | | rs9486410 | SYNONYMOUS_CODING | 1 |
| | | | | rs9486411 | INTRONIC | 1 |
| | | | | rs9486414 | INTRONIC | 1 |
| | | | | rs9486415 | INTRONIC | 1 |
| | | | | rs6926307 | INTRONIC | 1 |
| | | | | rs6926670 | INTRONIC | 1 |
| | | | | rs6926833 | INTRONIC | 1 |
| | | | | rs6907164 | INTRONIC | 1 |
| | | | | rs17067368 | INTRONIC | 1 |
| | | | | rs9480689 | INTRONIC | 1 |
| | | | | rs4523123 | INTRONIC | 1 |
| | | | | rs7741101 | INTRONIC | 1 |
| | | | | rs7776287 | INTRONIC | 1 |
| | | | | rs7776294 | INTRONIC | 1 |
| | | | | rs12523774 | INTRONIC | 0,92 |
| | | | | rs6903754 | INTRONIC | 1 |
| | | | | rs4945759 | INTRONIC | 1 |
| | | | | rs9689006 | INTRONIC | 1 |
| | | | | rs7770930 | INTRONIC | 1 |

| | | | | | | |
|---|---|---|---|---|---|---|
| ***Table 3:* Subset of 100 associated SNPs with tagged SNPs (r² > 0.8).** SNP: reference sequence identification number according to dbSNP; type: location/function of SNP; CHR: chromosome number; MAP: physical location of SNP on genome build HG18. tagged SNP r²: Linkage disequilibrium (LD) in the observed regions was assessed by calculating r² according to Hill et al. 1968.²⁵ | | | | | | |

It is known in the art that two or more alleles, polymorphisms or SNPs may be associated in a non-random manner. Such SNPs are also referred to as being in linkage disequilibrium and are inherited together. A genomic region comprising a group of SNPs being in linkage disequilibrium may also be referred to as haplotype block. SNPs being located within the same haplotype block may also be referred to as "tagged SNPs". Each of the SNPs, provided in Table 1 may be replaced with a corresponding tagged SNP from Table 3 for the purpose of the present invention. Corresponding tagged SNPs for a given SNP provided in column 1 of Table 3 are those tagged SNPs which are listed in column 5 of Table 3 subsequent to the given SNP. For example, SNPs rs1872133, rs1872132, until SNP rs12899914 are tagged SNPs corresponding to SNP rs1630535. Preferred tagged SNPs are those with a correlation coefficient (r2 value) of 1. The risk alleles of the tagged SNPs are those alleles which co-occur with the risk allele of the respective SNP as listed in Table 1. "Co-occurrence" in this context refers to those alleles of a group of SNPs within a haplotype block which occur together. Determining co-occurrence is within the skills of the skilled person.

The figures show:
**Figure 1**
   Distribution of risk alleles in cases and controls applying the subset of 18 SNPs according to the invention (see Table 2).
**Figure 2**
   Receiver Operating Characteristics (ROC) curve of the number of risk alleles as a predictor applying the subset of 18 SNPs according to the invention. The area under the curve is 1.0 hence yielding a perfect classification.
**Figure 3**
   Distribution of risk alleles in cases and controls applying SNPs as defined by SEQ ID NOs: 1 to 100.
**Figure 4**
   Receiver Operating Characteristics (ROC) curve of the number of risk alleles as a predictor applying SNPs as defined by SEQ ID NOs: 1 to 100. The area under the curve is 1.0 hence yielding a perfect classification.

The following examples illustrate the invention but should not be construed as being limiting.

### Example 1

### Methods

### MARS sample - patient recruitment

We recruited 397 patients aged 18 to 75 years who were admitted to the hospital of the Max Planck Institute of Psychiatry (MPI), Munich, Germany, for treatment of a depressive disorder presenting with a unipolar depressive episode (85.7%), bipolar disorder (12.4%, 6.1% bipolar I and 6.3% bipolar II), or other primary diagnosis with current depression (0.5% dysthymia, 0.7% adjustment disorder). Patients were diagnosed by psychiatrists according to the Diagnostic and Statistical Manual of Mental Disorders (DSM) IV criteria and were included in the study within the first three days after in-patient admission. Patients with depressive disorder due to a general medical or neurological condition were excluded, as were patients with a lifetime diagnosis of intravenous drug abuse and depressive symptoms secondary to alcohol or substance abuse or dependency. We recorded ethnicity using a self-report sheet for nationality, first language and ethnicity of the patient and of all four grandparents. All individuals included were Caucasian, 85.1 % were of German origin. The study was approved by the local ethics committee, and written informed consent was obtained from all subjects.

The study is designed as a naturalistic pharmacogenetic study (Munich Antidepressant Response Signature (MARS) project) to reveal genetic biomarkers specific for depressive disorder, clinical outcome and severe side effects. All patients are treated with antidepressants according to doctor's choice. Concomitant psychotropic medication with mood stabilizers, neuroleptics, benzodiazepines and hypnotics is allowed. For all patients plasma concentrations of antidepressants were monitored to assure clinical effective drug levels.

### Psychopathology and phenotype definition

Severity of depressive symptoms was assessed at admission by trained raters using the 21-item Hamilton Depression Rating Scale (HAM-D) ^{26, 27}. Patients fulfilling the criteria for at least a moderate to severe depressive episode (HAM-D >= 14) were eligible. Ratings were performed within five days of admission and then weekly until discharge. The severity of suicidal ideation was rated by item 3 "suicide" of the HAM-D, with 0 "absent"; 1 "subject feels life is not worth living"; 2 "wishes he/she were dead or any thoughts of possible death to self'; 3 "suicidal ideas or gestures" and 4 "attempts at suicide". TESI was defined as an increase of suicidal thoughts in patients without suicidal ideation (item 3 of the HAM-D = zero) at hospital admission (n = 32). The non-TESI comparison groups were 1) all individuals without increase in suicidality independent of baseline suicidality (no increase of the HAM-D item 3 over time; n = 329) and 2) a sub-group of 1), patients rating zero on item 3 throughout treatment (item 3 of the HAM-D = zero at all visits; n = 79). In the STAR*D trial the suicide item (item number 12) of the Quick Inventory of Depression Symptomatology-Self Report (QIDS-SR) was used to define TESI. Both scales should identify similar patients as the QIDS-SR has been shown to correlate well with the HAM-D ²⁸⁻³¹. The observation period for TESI was the first 12 weeks following in-patient admission, congruent with the observation period used in Laje et al., 2007 ³².

### DNA preparation

After enrolment in the study 40 ml of EDTA blood were drawn from each patient. DNA was extracted from fresh blood using the Puregene® whole blood DNA-extraction kit (Gentra Systems Inc; MN).

### Statistical Analysis of genetic associations

Exact tests on Hardy-Weinberg equilibrium (HWE) were performed for all SNPs ³³. SNPs with a minor allele frequency below 2.5 %, with a call rate of less than 98 %, or displaying HWE deviation at an error level of below 10⁻⁵ were excluded from the analysis. Final analysis was done in 371.335 SNPs. Both allelic and genotypic association tests were applied. To avoid false positive associations due to small cell sizes we used permutation-based p-value estimates (100,000 permutations, in 4 best SNPs 100,000,000 permutations) in addition to asymptotic p-values. If not otherwise specified, permutation-based p-values are reported. Genetic association was tested using the WG-permer, a C++ based statistical program for rapid permutation (http://www.wg-permer.org) Phenotypic analyses were performed using SPSS version 16.0.

### Results

The comparisons of disease-related and sociodemographic variables between TESI positive and negative patients using both the broader (comparison group 1) and more restricted definition (comparison group 2) are listed in Table 4. Except for age at onset there were no significant differences among these groups. 59.3 % of patients with TESI developed this side effect within the first 2 weeks after hospital admission. Current medication at the onset of suicidal ideation were SSRIs in 37.5 %, tricyclic antidepressants in 34.3 %, venlafaxine or duloxetine as dual serotonin and noradrenalin reuptake inhibitors in 25 %, mirtazapine in 28 % and reboxetine as a selective noradrenalin reuptake inhibitor in 12.5 %. Antidepressant monotherapy was administered to 62.5 % of the patients, while 37.5 % received combination therapy. There were no significant differences in psychotropic medication among the three groups (Table 5).

**Table 4**

| **Characteristics of MARS sample** | **TESI** n =32 8.1 % | **Non-TESI (1)** n=329 82.6% | **Non-TESI (2)** n=79 19.9% | χ**² or** t-test |
|---|---|---|---|---|
| Male | 43.8 % | 44.8 % | 49.5 % | NS |
| Female | 56.3 % | 55.2 % | 50.5 % | NS |
| Age (mean +/- SD) | 47.1 (16.1) | 49.3 (14.1) | 50.8 (14.3) | NS |
| Employment status *employed* | 62.9 % | 63.4 % | 61.1 % | NS |
| *retired* | 25.7 % | 24.1 % | 23.4 % | NS |
| *unemployed* | 11.4 % | 12.5% | 15.5% | NS |
| Living with partner / children / | 54.3 % | 60.9 % | 64.5 % | NS |
| personnel | 45.7 % | 39.1 % | 35.5 % | NS |
| Single | | | | |
| Age at onset (mean. +/- SD) | 32.6 (16.0) | 37.3 (15.4) | 40.6 (15.6) | * |
| HAM-D at inclusion (mean. +/- SD) | 24.5 (7.1) | 26.6 (6.6) | 22.8 (6.5) | NS |
| Previous episodes (mean. +/- SD) | 3.2 (4.0) | 2.8 (5.3) | 2.7 (5.5) | NS |
| Recurrent depression | 56.3 % | 58.2 % | 60.4 % | NS |
| Psychotic features | 18.8 % | 11.9 % | 13.9 % | NS |
| Illness duration in years (mean. +/- SD) | 13.9 (15.4) | 10.6 (10.5) | 10.3 (10.1) | NS |
| Duration of current episode in weeks (mean. +/- SD) | 38.0 (58.4) | 39.1 (69.3) | 28.4 (21.7) | NS |
| Family history of depression | 46.9 % | 46.6 % | 40.5 % | NS |
| History of attempted suicide | 18.8 % | 28.7 % | 16.2 % | NS |

| | | | | |
|---|---|---|---|---|
| ***Table 4:* Sociodemographic and clinical features** of patients developing TESI compared with patients without worsening of suicidal thoughts under psychopharmacological treatment (1) and with patients completely lacking suicidal ideation (2). * p = 0.012 TESI vs. Non-TESI (2). NS = not significant. | | | | |

**Table 5**

| **Medication** | **TESI** n=32 | **Non-TESI (1)** n=79 | **Non-TESI (2)** n=329 | t-test |
|---|---|---|---|---|
| SSRI | 37.5 % | 37.8 % | 35.7 % | NS |
| Tricyclic antidepressants | 34.4 % | 20.4 % | 22.9 % | NS |
| SNRI | 25 % | 19.4 % | 19.2 % | NS |
| Mirtazapine | 28 % | 35.7 % | 34 % | NS |
| NRI | 12.5 % | 2 % | 4 % | NS |
| Neuroleptics | 21.9 % | 17.3 % | 22 % | NS |
| Mood stabilizers | 46.9 % | 35.7 % | 29.9 % | NS |
| Benzodiazepines | 50 % | 20.4 % | 30 % | * # |

| | | | | |
|---|---|---|---|---|
| ***Table 5:* Psychotropic medication at onset of TESI compared with medication of control group** Medication was according to doctor's choice. A combination of two or more antidepressants was also allowed (TESI=37.5%; Non-TESI (1) = 23.5%; Non-TESI (2) = 24.4 %; no significant difference). * TESI vs Non-TESI (1) p < 0.05; # TESI vs Non-TESI (2) p < 0.05. NS = not significant | | | | |

Provided are 100 SNPs, which were significantly associated with TESI with an empiric p-value < 0.001 comparing to both non-TESI groups (see Table 1). SNP rs1630535 showed a p value of 1.3x10⁻⁷, OR 10.535, Cl 4.2-26.4 comparing to narrow non-TESI group, and p value of 2.45x10⁻⁷, OR 4.16, Cl 2.3-7.4 comparing to broader non-TESI group. Fisher Product Method over all 100 SNPs revealed a significant association comparing TESI vs non-TESI(2) with allelic p=1x10⁻⁵, genotypic p=1x10⁻⁵ and combination of allelic and genotypic p=1x10⁻⁵. A discriminant analysis patients using this subset of SNPs revealed a 100 % probability to classify TESI vs non-TESI cases correctly, no matter if narrow or broader non-TESI definition was applied (narrow definition, 32 vs 79: chi square = 273 and df = 91; broader definition, 32 vs 329: Chi square =429 and df = 96). Furthermore, the distribution of the number of risk alleles present in each individual yielded two separate groups, with TESI cases having 85 and more risk alleles while non-TESI individuals having 80 and less risk ailles (see Figure 3).

**Table 6: Sequences of the SNPs of SEQ ID NOs: 1 to 100; in no particular order. The polymorphic position is indicated by square brackets, for example [A/G] in case of rs 6793017**

| SNP rs# | Sequence |
|---|---|
| rs67 9301 7 | |
| rs12 6523 5 | |
| rs48 8139 4 | |
| rs47 2470 1 | |
| rs63 5546 | |
| rs18 8464 1 | |
| rs93 0282 2 | |
| rs38 0103 3 | |
| rs26 6209 0 | |
| rs27 9553 | |
| rs27 9542 | |
| rs11 2500 17 | |
| rs10 1312 0 | |
| rs11 2589 03 | |
| rs76 4528 9 | |
| rs60 3571 2 | |
| rs73 0706 4 | |
| rs22 7010 1 | |
| rs77 8866 8 | |
| rs18 9187 7 | |
| rs73 5073 1 | |
| rs47 9506 9 | |
| rs45 4421 4 | |
| rs58 4762 | |
| rs76 1453 | |
| rs12 5833 95 | |
| rs20 7043 9 | |
| rs80 9518 6 | |
| rs58 3338 | |
| rs22 2824 6 | |
| rs26 6453 7 | |
| rs60 7231 7 | |
| rs60 7234 3 | |
| rs45 1802 3 | |
| rs71 4633 2 | |
| rs20 8813 8 | |
| rs48 1062 2 | |
| rs28 3917 8 | |
| rs12 5896 23 | |
| rs24 1466 0 | |
| rs16 3053 5 | |
| rs68 6884 6 | |
| rs79 0146 3 | |
| rs35 6999 | |
| rs17 1750 96 | |
| rs49 3951 7 | |
| rs10 3744 8 | |
| rs22 4405 7 | |
| rs10 9970 44 | |
| rs99 4932 4 | |
| rs61 8670 | |
| rs47 9915 9 | |
| rs10 4908 32 | |
| rs15 4538 4 | |
| rs64 9867 | |
| rs22 7910 3 | |
| rs52 3386 | |
| rs77 0493 9 | |
| rs19 5843 8 | |
| rs19 5842 1 | |
| rs93 2373 7 | |
| rs12 1422 66 | |
| rs65 0049 7 | |
| rs65 0049 8 | |
| rs14 5433 3 | |
| rs95 8969 8 | |
| rs20 2594 9 | |
| rs90 2923 | |
| rs19 8415 1 | |
| rs14 5984 1 | |
| rs49 1763 9 | |
| rs93 3217 2 | |
| rs94 8068 4 | |
| rs23 4842 7 | |
| rs27 7408 9 | |
| rs49 0767 4 | |
| rs98 7785 9 | |
| rs12 0479 8 | |
| rs77 1932 5 | |
| rs68 7364 0 | |
| rs29 8087 2 | |
| rs55 4710 | |
| rs72 0733 | |
| rs43 8830 1 | |
| rs11 0908 9 | |
| rs20 7499 7 | |
| rs96 5118 | |
| rs37 5315 1 | |
| rs22 9996 5 | |
| rs22 9996 7 | |
| rs69 4819 6 | |
| rs12 6175 66 | |
| rs14 2178 0 | |
| rs30 1191 | |
| rs30 1193 | |
| rs13 4035 84 | |
| rs76 7610 6 | |
| rs76 8531 4 | |
| rs12 1436 47 | |
| rs75 5027 7 | |

### Further references

**1.** Kessler RC, Berglund P, Demler O, Jin R, Merikangas KR, Walters EE. Lifetime prevalence and age-of-onset distributions of DSM-IV disorders in the National Comorbidity Survey Replication. Arch Gen Psychiatry. Jun 2005;62(6):593-602.
**2.** Nierenberg AA, Gray SM, Grandin LD. Mood disorders and suicide. J Clin Psychiatry. 2001;62 Suppl 25:27-30.
**3.** Teicher MH, Glod C, Cole JO. Emergence of intense suicidal preoccupation during fluoxetine treatment. Am J Psychiatry. Feb 1990;147(2):207-210.
**4.** Masand P, Gupta S, Dewan M. Suicidal ideation related to fluoxetine treatment. N Engl J Med. Feb 7 1991;324(6):420.
**5.** Wirshing WC, Van Putten T, Rosenberg J, Marder S, Ames D, Hicks-Gray T. Fluoxetine, akathisia, and suicidality: is there a causal connection? Arch Gen Psychiatry. Jul 1992;49(7):580-581.
**6.** Licinio J, Wong ML. Depression, antidepressants and suicidality: a critical appraisal. Nat Rev Drug Discov. Feb 2005;4(2):165-171.
**7.** Hall WD, Mant A, Mitchell PB, Rendle VA, Hickie IB, McManus P. Association between antidepressant prescribing and suicide in Australia, 1991-2000: trend analysis. Bmj. May 10 2003;326(7397):1008.
**8.** Morgan OW, Griffiths C, Majeed A. Association between mortality from suicide in England and antidepressant prescribing: an ecological study. BMC Public Health. Dec 21 2004;4:63.
**9.** Nakagawa A, Grunebaum MF, Ellis SP, et al. Association of suicide and antidepressant prescription rates in Japan, 1999-2003. J Clin Psychiatry. Jun 2007;68(6):908-916.
**10.** Angst J, Angst F, Gerber-Werder R, Gamma A. Suicide in 406 mood-disorder patients with and without long-term medication: a 40 to 44 years' follow-up. Arch Suicide Res. 2005;9(3):279-300.
**11.** Perlis RH, Beasley CM, Jr., Wines JD, Jr., et al. Treatment-associated suicidal ideation and adverse effects in an open, multicenter trial of fluoxetine for major depressive episodes. Psychother Psychosom. 2007;76(1):40-46.
**12.** Jick H, Kaye JA, Jick SS. Antidepressants and the risk of suicidal behaviors. Jama. Jul 21 2004;292(3):338-343.
**13.** Hammad TA, Laughren T, Racoosin J. Suicidality in Pediatric Patients Treated With Antidepressant Drugs 10.1001/archpsyc.63.3.332. Arch Gen Psychiatry. March 1, 2006 2006;63(3):332-339.
**14.** Vitiello B, Swedo S. Antidepressant Medications in Children 10.1056/NEJMp038248. N Engl J Med. April 8, 2004 2004;350(15):1489-1491.
**15.** Marshall E. ANTIDEPRESSANTS AND CHILDREN: Buried Data Can Be Hazardous to a Company's Health 10.1126/science.304.5677.1576. Science. June 11, 2004 2004;304(5677):1576-1577.
**16.** Libby AM, Brent DA, Morrato EH, Orton HD, Allen R, Valuck RJ. Decline in Treatment of Pediatric Depression After FDA Advisory on Risk of Suicidality With SSRIs 10.1176/appi.ajp.164.6.884. Am J Psychiatry. June 1, 2007 2007;164(6):884-891.
**17.** Valuck RJ, Libby AM, Orton HD, Morrato EH, Allen R, Baldessarini RJ. Spillover Effects on Treatment of Adult Depression in Primary Care After FDA Advisory on Risk of Pediatric Suicidality With SSRIs. Am J Psychiatry. August 1, 2007 2007;164(8):1198-1205.
**18.** Gibbons RD, Brown CH, Hur K, et al. Early evidence on the effects of regulators' suicidality warnings on SSRI prescriptions and suicide in children and adolescents. Am J Psychiatry. Sep 2007;164(9):1356-1363.
**19.** Brent DA, Mann JJ. Family genetic studies, suicide, and suicidal behavior. Am J Med Genet C Semin Med Genet. Feb 15 2005;133(1):13-24.
**20.** Statham DJ, Heath AC, Madden PA, et al. Suicidal behaviour: an epidemiological and genetic study. Psychol Med. Jul 1998;28(4):839-855.
**21.** Sherry ST, Ward MH, Kholodov M, et al. dbSNP: the NCBI database of genetic variation. Nucleic Acids Res. Jan 1 2001;29(1):308-311.
**22.** Sambrook J, Fritsch EF, Maniatis T. Molecular Cloning: A Laboratory Manual. New York: Cold Spring Harbor Laboratory Press; 1989.
**23.** Kim S, Misra A. SNP genotyping: technologies and biomedical applications. Annu Rev Biomed Eng. 2007;9:289-320.
**24.** Wang L, Luhm R, Lei M. SNP and mutation analysis. Adv Exp Med Biol. 2007;593:105-116.
**25.** Hill WG, Robertson A. Linkage disequilibrium in finite populations. Theoretical and Applied Genetics. 1968;38:226-231.
**26.** Hamilton M. A rating scale for depression. J Neurol Neurosurg Psychiatry. Feb 1960;23:56-62.
**27.** Hamilton M. Development of a rating scale for primary depressive illness. Br J Soc Clin Psychol. Dec 1967;6(4):278-296.
**28.** Trivedi MH, Rush AJ, Ibrahim HM, et al. The Inventory of Depressive Symptomatology, Clinician Rating (IDS-C) and Self-Report (IDS-SR), and the Quick Inventory of Depressive Symptomatology, Clinician Rating (QIDS-C) and Self-Report (QIDS-SR) in public sector patients with mood disorders: a psychometric evaluation. Psychol Med. Jan 2004;34(1):73-82.
**29.** Rush AJ, Trivedi MH, Ibrahim HM, et al. The 16-ltem Quick Inventory of Depressive Symptomatology (QIDS), clinician rating (QIDS-C), and self-report (QIDS-SR): a psychometric evaluation in patients with chronic major depression. Biol Psychiatry. Sep 1 2003;54(5):573-583.
**30.** Rush AJ, Trivedi MH, Carmody TJ, et al. Self-reported depressive symptom measures: sensitivity to detecting change in a randomized, controlled trial of chronically depressed, nonpsychotic outpatients. Neuropsychopharmacology. Feb 2005;30(2):405-416.
**31.** Rush AJ, Bernstein IH, Trivedi MH, et al. An evaluation of the quick inventory of depressive symptomatology and the hamilton rating scale for depression: a sequenced treatment alternatives to relieve depression trial report. Biol Psychiatry. Mar 15 2006;59(6):493-501.
**32.** Laje G, Paddock S, Manji H, et al. Genetic Markers of Suicidal Ideation Emerging During Citalopram Treatment of Major Depression. Am J Psychiatry. October 1, 2007 2007;164(10):1530-1538.
**33.** Wigginton JE, Cutler DJ, Abecasis GR. A note on exact tests of Hardy-Weinberg equilibrium. Am J Hum Genet. May 2005;76(5):887-893.

## Claims

1. A method of diagnosing a predisposition for or the occurrence of treatment emergent suicidal ideation in an individual, the method comprising determining the presence or absence of SNPs selected from the SNPs defined by SEQ ID NOs: 1 to 100 in a sample obtained from a patient, wherein presence of the respective SNPs is/are indicative of said predisposition for or said occurrence of treatment emergent suicidal ideation, wherein said SNPs consist of or comprise the SNPs defined by SEQ ID NOs: 1, 3, 6, 9, 21, 23, 29, 32, 38, 53, 55, 59, 61, 62, 66, 76, 78 and 94.

2. The method of claim 1, wherein all 100 SNPs are used.

3. The method of any one of the preceding claims, wherein said determining is effected by allele specific hybridization, allele specific oligonucleotide ligation, primer extension, minisequencing, mass spectroscopy, heteroduplex analysis, single strand conformational polymorphism, denaturing gradient gel electrophoresis, oligonucleotide microarray analysis, temperature gradient gel electrophoresis or combinations thereof.

4. The method of any one of the preceding claims, wherein said treatment emergent suicidal ideation occurs or may occur in a patient being administered selective serotonin re-uptake inhibitors, selective noradrenalin re-uptake inhibitors, dual serotonin and noradrenalin re-uptake inhibitors and/or tricyclic antidepressants, and optionally being administered neuroleptics, mood stabilizers and/or benzodiazepines.

5. The method of any one of the preceding claims, wherein said individual is a child or adolescent.

6. The method of any one of the preceding claims, wherein said determining comprises isolating a nucleic acid from said sample.

## Patentansprüche

1. Verfahren zum Diagnostizieren einer Veranlagung für oder des Auftretens von behandlungsbedingten Suizidgedanken in einem Individuum, wobei das Verfahren das Bestimmen der Anwesenheit oder Abwesenheit von SNPs in einer von einem Patienten erhaltenen Probe umfasst, wobei die SNPs durch die SEQ ID NOs. 1 bis 100 definiert sind, wobei die Anwesenheit des jeweiligen SNPs auf die Veranlagung für oder das Auftreten von behandlungsbedingten Suizidgedanken hinweist, wobei die SNPs aus den durch die SEQ ID NOs. 1, 3, 6, 9, 21, 23, 29, 32, 38, 53, 55, 59, 61, 62, 66, 76, 78 und 94 definierten SNPs bestehen oder diese umfassen.

2. Verfahren nach Anspruch 1, wobei alle 100 SNPs verwendet werden.

3. Verfahren nach einem der vorangegangenen Ansprüche, wobei das Bestimmen mittels allelspezifischer Hybridisierung, allelspezifischer Oligonukleotidligierung, Primerverlängerung, Minisequenzierung, Massenspektrometrie, Heteroduplexanalyse, Einzelstrangkonformationspolymorphismus, denaturierender Gradientengelelektrophorese, Oligonukleotidmikroarrayanalyse, Temperaturgradientengelelektrophorese oder Kombinationen davon durchgeführt wird.

4. Verfahren nach einem der vorangegangenen Ansprüche, wobei die behandlungsbedingten Suizidgedanken in einem Patienten auftreten oder auftreten können, dem selektive Serotoninwiederaufnahme-Inhibitoren, selektive Noradrenalinwiederaufnahme-Inhibitoren, duale Serotonin- und Noradrenalinwiederaufnahme-Inhibitoren und/oder trizyklische Antidepressiva und gegebenenfalls Neuroleptika, Stimmungsstabilisierer und/oder Benzodiazepine verabreicht werden.

5. Verfahren nach einem der vorangegangenen Ansprüche, wobei das Individuum ein Kind oder ein Jugendlicher ist.

6. Verfahren nach einem der vorangegangenen Ansprüche, wobei das Bestimmen das Isolieren einer Nukleinsäure aus der Probe umfasst.

## Revendications

1. Procédé pour diagnostiquer une prédisposition ou l'apparition chez un individu d'une idéation suicidaire consécutive à un traitement, le procédé comprenant la détermination de la présence ou de l'absence de SNPs choisis parmi les SNPs définis par les SEQ ID NO : 1 à 100 dans un échantillon obtenu à partir d'un patient, dans lequel la présence des SNPs respectifs est indicative de ladite prédisposition ou de ladite apparition d'une idéation suicidaire consécutive à un traitement, dans lequel lesdits SNPs consistent en, ou comprennent, les SNPs définis par les SEQ ID NOs: 1, 3, 6, 9, 21, 23, 29, 32, 38, 53, 55, 59, 61, 62, 66, 76, 78 et 94.

2. Procédé selon la revendication 1, dans lequel la totalité des 100 SNPs est utilisée.

3. Procédé selon l'une quelconque des revendications précédentes, dans lequel ladite détermination est effectuée par hybridation allèle-spécifique, ligation d'oligonucléotides allèle-spécifiques, extension d'amorces, miniséquençage, spectroscopie de masse, analyse d'hétéroduplexes, polymorphisme conformationnel simple brin, électrophorèse sur gel en gradient dénaturant, analyse de puce d'oligonucléotides, électrophorèse sur gel en gradient de température, ou les combinaisons de ceux-ci.

4. Procédé selon l'une quelconque des revendications précédentes, dans lequel ladite idéation suicidaire consécutive à un traitement apparaît, ou peut apparaître, chez un patient auquel est administré des inhibiteurs sélectifs de la recapture de la sérotonine, des inhibiteurs sélectifs de la recapture de la noradrénaline, des inhibiteurs doubles de la recapture de la sérotonine et de la noradrénaline et/ou des antidépresseurs tricycliques, et optionnellement auquel est administré des neuroleptiques, des stabilisateurs de l'humeur et/ou des benzodiazépines.

5. Procédé selon l'une quelconque des revendications précédentes, dans lequel ledit individu est un enfant ou un adolescent.

6. Procédé selon l'une quelconque des revendications précédentes, dans lequel ladite détermination comprend l'isolement d'un acide nucléique à partir dudit échantillon.


### SEQUENCE LISTING

<110> Max-Planck-Gesellschaft zur Förderung der Wissenschaften e.V.
<120> Means and methods for diagnosing predisposition for treatment emergent suicidal ideation (TESI)
<130> P2377 EP
<160> 813
<170> PatentIn version 3.3
<210> 1
   <211> 401
   <212> DNA
   <213> Artificial sequence
<220>
   <221> source
   <223> /note="Description of artificial sequence: rs1630535"
<220>
   <221> variation
   <222> 101
   <223> /replace="c" /replace="g" /replace="t"
<220>
   <221> variation
   <222> 125
   <223> /replace="c" /replace="g" /replace="t"
<220>
   <221> variation
   <222> 145
   <223> /replace="c" /replace="g" /replace="t"
<220>
   <221> variation
   <222> 201
   <223> /replace="g"
<400> 1
   tgacactgaa aaacccactc agtcacaggg ttaagaaact aataaaatta ctatttttca     60
   agtccttagt acatatttag catgattcta ggcatggcac aggcagaaga aaagtataaa    120
   atatagcttc tacatataac ttacagtttt actaaggaga tgattgggta aagacagtcc    180
   attcaaatgc cataatcaac agtgcatagt caagggccag ccaccaagca gctgggagag    240
   agaacattga gctaatattt ggaataaacc aggtttgcca gtcaattatc agaagtacct    300
   ggttggtaag gagtcctgca gagaagagaa gttgttttct gacagcctta agcagttgat    360
   catgcctaaa gacaacatta tcaatgtcca ctaggctctt t                        401
<210> 2
   <211> 401
   <212> DNA
   <213> Artificial sequence
<220>
   <221> source
   <223> /note="Description of artificial sequence: rs4724701"
<220>
   <221> variation
   <222> 93
   <223> /replace="c" /replace="g" /replace="t"
<220>
   <221> variation
   <222> 201
   <223> /replace="g"
<400> 2
   ccctggttcc accgcaggcg aagtcaagat ccaaagcaaa gtcgaggaca cagtgaccgt     60
   gtttttaaaa ccgccatgaa ccagggcgga ccacgacacc cagatgagag ccgtgaggac    120
   agcactgaga ataatcactt agggctttat ttactgacac aagaggactc cacggtgtag    180
   tgttacattt ttaaaaatca aattacaaaa caatatgaga accctatctc cagttcattc    240
   tccagggaaa gttggcagca ttcactgttg ggaaaccttc acttaaaaat aggttgatgt    300
   tggccaggcg ttgtggctca cgcctgtaat cccagcactt tgggaggctg aggtaagcgg    360
   atcacctgag gtcaggagtt agagaccagc ctgggcaaca t                        401
<210> 3
   <211> 401
   <212> DNA
   <213> Artificial sequence
<220>
   <221> source
   <223> /note="Description of artificial sequence: rs1265235"
<220>
   <221> variation
   <222> 18
   <223> /replace="c" /replace="g" /replace="t"
<220>
   <221> variation
   <222> 201
   <223> /replace="g"
<400> 3
   actgcatctg cgtagacatg tcatccagta tactcatgcg atgtcactca tctgccccct     60
   cctacataaa tgatacacaa aaaagtcaac aatcagagaa acccaagctc ctggatgtca    120
   acatccactt ctgcttggag ttggttcctt tcatggagac atacacccct cgccacacac    180
   ctgccacctc cctgaccctc acaatccctg gcatgagaag ctccaccttg tcaagagcag    240
   acattctgaa aggctgaagc caggaacctt aaaggaaaca ttctgccaag tccaccacgt    300
   aggatcaatg gagatggtgg tagtgtggtt attggcttac ccagctctgg gtgtagagtg    360
   ctttggggag gaggaaggaa gaagagctga tcctgccttc t                        401
<210> 4
   <211> 401
   <212> DNA
   <213> Artificial sequence
<220>
   <221> source
   <223> /note="Description of artificial sequence: rs2414660"
<220>
   <221> variation
   <222> 60
   <223> /replace="c" /replace="g" /replace="t"
<220>
   <221> variation
   <222> 201
   <223> /replace="c"
<400> 4
   tagttttatt ttcaaaacag gaacatccgg cagaatctca gctttcattt tgaattcaga 60
   gttcccgctt catccattat tcatacacac tgcactttcc tggtttgtga ttttactaaa 120
   tttcttcatt tgaattatgg ccttgtggtc aacagcgggc aatatattat tcttatataa 180
   tttttaaata caaaggcaaa tggcacggaa caattagaat cgatggcttg ggagtctggc 240
   actcagatgt cataggcaaa tgctgttttc ttgactttaa cctatgggtt ccacttcatg 300
   tgggttgtat ttttcataca tcaggagttt ttataaattt cctattcaat ccagcagccc 360
   tactttgttc gcaaaatata agcaaaacaa atatgtcagg g 401
<210> 5
   <211> 401
   <212> DNA
   <213> Artificial sequence
<220>
   <221> source
   <223> /note="Description of artificial sequence: rs12589623"
<220>
   <221> variation
   <222> 201
   <223> /replace="c"
<220>
   <221> variation
   <222> 276
   <223> /replace="c" /replace="g" /replace="t"
<400> 5
   aatcgtagca acaaaacaaa ggcaattcca aattcaaaaa aatcattttt aaattctgcg 60
   tctcatcatg cattctgatt atgtattagt aaaaatatga aatcccacct gggcttaatg 120
   ctaactaaaa ttttaaatat tatcactact ttacattttt taaaaagaat aaaacatttt 180
   aaaaagcatt atgtaagaaa agcatggagt cagatttgag ttgggaactg ttattatgct 240
   ggttctttag gctgttgttt tctaagggaa ctaaaaaact caattttctt atcctctttg 300
   ggtgagtggt gctgtaagtt tgaagactaa tgtaaggctc tagagtcaga aagtatttta 360
   ttccatacct aatttttcca ctatgttttg ttattcattt t 401
<210> 6
   <211> 401
   <212> DNA
   <213> Artificial sequence
<220>
   <221> source
   <223> /note="Description of artificial sequence: rs583338"
<220>
   <221> variation
   <222> 161
   <223> /replace="c" /replace="g" /replace="t"
<220>
   <221> variation
   <222> 201
   <223> /replace="a"
<400> 6
   tactttccta tcatcatcta cctttgggat attatgtctt tcttagtaaa gtgtcgctac     60
   aagacttttg tccatttttt attgaaatgt ctgtctttta aacattgatt tttctttaaa    120
   tattttggat acaattccta tgtcaggatt atacattgca aatatactct ctaacttcta    180
   ccctgtgcct tgcctttttt gttctcaagt catctgtaat ttgcattctt ccgtcttttt    240
   tcccgtcaat gttatcagga attgtatacc tggagagtat aaaccatata ctctctaagc    300
   acatttagag ctactttctg tagatcttga tatgacatgt tatcattatc attcagttca    360
   caatattttc ctctaatttc tttttttaaa ttttattatt a                        401
<210> 7
   <211> 401
   <212> DNA
   <213> Artificial sequence
<220>
   <221> source
   <223> /note="Description of artificial sequence: rs279542"
<220>
   <221> variation
   <222> 201
   <223> /replace="c"
<220>
   <221> variation
   <222> 309
   <223> /replace="c" /replace="g" /replace="t"
<220>
   <221> variation
   <222> 320
   <223> /replace="c" /replace="g" /replace="t"
<400> 7
   gtgtgttgct tgaaaggtgt ggccttagac actaggagat gatgggtaga agctccttga 60
   gaaacctagc agtggccata gagaagtaga ctgggtatgg ggaacctaag tcatagttgt 120
   aacctacagc tgattggtaa gaactatggg ccagacattt aatctctcca tctatgaaat 180
   agtccattct catttatcta gcttaggtta tgggctcttc ttttagttgg atattcacat 240
   ttatctttca gtgttaactg ggtatcagtt atgagtctac tactgtataa tggcatccaa 300
   aagaatttaa aagtacctca gtttataggg atttacaatt cagtagaaac ttgttattca 360
   tgtgaaacag tgaacataat atctaatgat gtggagcaag t 401
<210> 8
   <211> 401
   <212> DNA
   <213> Artificial sequence
<220>
   <221> source
   <223> /note="Description of artificial sequence: rs279553"
<220>
   <221> variation
   <222> 53
   <223> /replace="c" /replace="g" /replace="t"
<220>
   <221> variation
   <222> 201
   <223> /replace="a"
<220>
   <221> variation
   <222> 378
   <223> /replace="c" /replace="g" /replace="t"
<400> 8
   tagctaatgt tgttgcgggg aaaggagaga aggcccaaga gagagggcaa aaagctcagc 60
   aaatacgggt attcccactg atagggcatg gccacctgat catgtgacaa gagcctcagg 120
   tgtcccacgc tcatcttagc aaccagcagc agccatatga ccagatgtac gtagatcagc 180
   ttcttgattt catacttgag ggtcacactg tggggcagag cagagggaga agtatctttc 240
   aaatttttct taccaccaac tatatgccag acaactgtac tggacactgg gatacaaatt 300
   tgaataaaac agcctagaag ccggacacaa agaacagagg aggaaaaagt aattagaaaa 360
   tgaagtgaaa aagttctagt aaatatatgt tcccagatat a 401
<210> 9
   <211> 401
   <212> DNA
   <213> Artificial sequence
<220>
   <221> source
   <223> /note="Description of artificial sequence: rs2774089"
<220>
   <221> variation
   <222> 18
   <223> /replace="c" /replace="g" /replace="t"
<220>
   <221> variation
   <222> 201
   <223> /replace="t"
<400> 9
   ctttccctgg gtgtgggaac tgccggtgtg ggaactggct gctggcgtgg gaactggctg     60
   ctggtgtggg aacggctgct tgtcccagtg gtgcccacct gtgtccacac ttcaaggcca    120
   cctgggcctg gagatgtcac taagggttga cccggagacg ccgacctcag ggacctggga    180
   ggctgcacac tggccgtgtt ggaagtgacc ctcatgtcac ccaggtcaag tgccacgggc    240
   cccacacatc ctcttcttcc ctgtctccca gctgcaggcc cggactggat ggctttggct    300
   aaggaaccaa cgcaggtgga gaaggaggct gacgatggag cccaggcact ccagccttga    360
   aggaggagaa cctgaccctg agcctgcatg gcagccgtgc g                         401
<210> 10
   <211> 401
   <212> DNA
   <213> Artificial sequence
<220>
   <221> source
   <223> /note="Description of artificial sequence: rs1421780"
<220>
   <221> variation
   <222> 107
   <223> /replace="c" /replace="g" /replace="t"
<220>
   <221> variation
   <222> 114
   <223> /replace="c" /replace="g" /replace="t"
<220>
   <221> variation
   <222> 201
   <223> /replace="g"
<400> 10
   ctatattctt cccctattct acccatctac cttccttttc ttaccccagc tactccaaag 60
   aaagaaaaat ggccagtttg gtcagatatc aagaccttta caggaaaatg gttagccaat 120
   ggagggatca gggccaccaa gtgtcagaaa agctattctg ctttctacct ccctctgtca 180
   aagtcacctg tgcaagactt acctcttggg gctaggacag ctggcatctc tccagtgggg 240
   cacaggtata ttaggggctc agtacccatt cagtcccagc cagccctctt ccctctgacc 300
   cggcctaaag cccacttccc tcctacttgc aagagtggct tattaagggt cccaagattt 360
   gaaaacagga agaatgcagg agccctttat acctcagtct t 401
<210> 11
   <211> 401
   <212> DNA
   <213> Artificial sequence
<220>
   <221> source
   <223> /note="Description of artificial sequence: rs4907674"
<220>
   <221> variation
   <222> 66
   <223> /replace="c" /replace="g" /replace="t"
<220>
   <221> variation
   <222> 201
   <223> /replace="t"
<400> 11
   gtgaactagt acacttgttt taccagaagt cacacaggat ccttaaagca tcctcattct 60
   ttctcagtac aggttctgga tggaggtctc atgagggatt cgggcagagg ctgatgaaaa 120
   cataaaacag gccagtctcc agattcaagg gagagctgga gacagcattc atagcttgac 180
   caggcagctt tgaccaggtg ggacaaagga agcccagatg tcccagtcta ttcatgtttc 240
   tatcacaaaa tgcctttgaa ttagtaattt ataaacaata gaaatttatt tcttatcgtt 300
   ctggaggccg gaaagtccac gatgaagaca ccagctggtt cccagccggt gggtgaggac 360
   ccagcctctg cttccaagat ggcacctctg ggctgcatcc t 401
<210> 12
   <211> 401
   <212> DNA
   <213> Artificial sequence
<220>
   <221> source
   <223> /note="Description of artificial sequence: rs7676106"
<220>
   <221> variation
   <222> 12
   <223> /replace="c" /replace="g" /replace="t"
<220>
   <221> variation
   <222> 54
   <223> /replace="c" /replace="g" /replace="t"
<220>
   <221> variation
   <222> 254
   <223> /replace="c" /replace="g" /replace="t"
<220>
   <221> variation
   <222> 101
   <223> /replace="c" /replace="g" /replace="t"
<220>
   <221> variation
   <222> 201
   <223> /replace="t"
<400> 12
   cagaatttct aatgctatac tttcttgagg taattttaag aaaggtttcc ttaacattta     60
   cccaaatttc atacacagac acacaccaaa acaaaaacaa aacaaaaact gaatgaatat    120
   ttttgtgctg atacaaaccg tcacaaagga agtaatgatg ggatttgtct acattaacct    180
   gacatactgg tgaaaataac cccattgttc attatttgaa ttaggcaata atttggagaa    240
   attcttgaag aggacataaa ggagaccgca gatacgtgtg ggctgcctgt gaaaggcatg    300
   ctgctttgta aatattcaca ctaaactttc aaacacaaga gtgatactac cctagcaaaa    360
   cctaaggttc tccagggagg caggtgacta gttccacttt t                         401
<210> 13
   <211> 401
   <212> DNA
   <213> Artificial sequence
<220>
   <221> source
   <223> /note="Description of artificial sequence: rs9949324"
<220>
   <221> variation
   <222> 48
   <223> /replace="c" /replace="g" /replace="t"
<220>
   <221> variation
   <222> 99
   <223> /replace="c" /replace="g" /replace="t"
<220>
   <221> variation
   <222> 201
   <223> /replace="g"
<220>
   <221> variation
   <222> 401
   <223> /replace="c" /replace="g" /replace="t"
<400> 13
   gtggagtttc tctgttttcc gagacggctt actcggcgcc tagtgagacg gtgcttctgt     60
   ttttgtctct actccgtgtt ctgagtgcac ccatttatac ccagatagcg tttatactta    120
   cagaggagac aggcgaacac taaaacacag cagaaatatg acgaaacacc ctgcaacctt    180
   gtttcttttg agttcctatc actcacttca atctattcta aaaataaagg acttgtcagg    240
   cctaatgata agggagagtt ttagggaaat aaagagagct gaactgacac agacataata    300
   atgtcaagat ataaatcaca gagtccttct gccattttca ttatctgcct cggaggatta    360
   cggtgcttgc tataaatgag acgctatggg gactctgccc a                        401
<210> 14
   <211> 401
   <212> DNA
   <213> Artificial sequence
<220>
   <221> source
   <223> /note="Description of artificial sequence: rs4518023"
<220>
   <221> variation
   <222> 117
   <223> /replace="c" /replace="g" /replace="t"
<220>
   <221> variation
   <222> 201
   <223> /replace="c"
<220>
   <221> variation
   <222> 293
   <223> /replace="c" /replace="g" /replace="t"
<400> 14
   tgtgaggcta aaggctccca cgcagcatgg ggcatgggga gcctgaatgt aagccccagg     60
   gcaggaagtt ttgcctattt gctcaccgtg taatctccag catctagaat agcataatag    120
   gcctttggta aatatctgtt gaatggaagg actaaaatta gttctggaag tgtctgggga    180
   tggggcttaa gttacctctg aagggtctcc aaggctaaat ccttttcctg agttagaaaa 240
   acagggctgt tctcactgaa gttttggcct tcagagtgtg atgaggagtg caaaggggca 300
   cggggcagct tcaaggacag ggctgggacc acagagggtg agaaaagggc acaagggagg 360
   aggggtgctc ccaagatggc agcccctaga gatctgggct g 401
<210> 15
   <211> 401
   <212> DNA
   <213> Artificial sequence
<220>
   <221> source
   <223> /note="Description of artificial sequence: rs7704939"
<220>
   <221> variation
   <222> 201
   <223> /replace="c"
<400> 15
   gaggatgcag aaggaacatc tgggctcagc tatttcatgc tgtcggccat acacttccaa 60
   gtgggaggtg atggcagttt ggtcagctgt ttgccaaccc aggagttggg taaaggttgg 120
   taagagatgt tattagtttt agcaccattt ccttccaata gtctcatttt aatacaatac 180
   aggttagtag tgaccagaaa atattgctac tgaattgaag gatgactata caaggactta 240
   tatcagatta tttgttgctt ttagtttcta gtgtcccata aagtatagaa atggaaattg 300
   ctgacaagag agtacctttg gtaggcacag ttccttttct ccccccactg gagaggacgc 360
   tacaaccttg ctatagagtg tcctgtgact gcccagtgat g 401
<210> 16
   <211> 401
   <212> DNA
   <213> Artificial sequence
<220>
   <221> source
   <223> /note="Description of artificial sequence: rs4544214"
<220>
   <221> variation
   <222> 201
   <223> /replace="a"
<220>
   <221> variation
   <222> 218
   <223> /replace="c" /replace="g" /replace="t"
<220>
   <221> variation
   <222> 395
   <223> /replace="c" /replace="g" /replace="t"
<400> 16
   taaaaatgca ttaacgtctt tttatatcca tcaagggaag gatgaaatgt tgaatttgaa     60
   gactaattca gtaagaagtc ctaggggttt aactgtacat actacctgaa ctggcttttc    120
   tgagagatga atcaataatg aaacatgtct gttttaaaaa ctaccacatg tgactcctat    180
   ttttgttagc tgaaagctgc tatacggagt attacagaaa tgtgaaggtg actagcttga    240
   aggtagggta actagagagc cagaaaagtt ttgttttaaa cttgatttaa tgcgttttta    300
   ttttttctta tacaaaatag agataatgtt gctaacttca tggaatattt gaggaaatga   360
   tatgaaagtg tctggacgtg cagtaacctc atggattctt c                        401
<210> 17
   <211> 401
   <212> DNA
   <213> Artificial sequence
<220>
   <221> source
   <223> /note="Description of artificial sequence: rs6072343"
<220>
   <221> variation
   <222> 85
   <223> /replace="c" /replace="g" /replace="t"
<220>
   <221> variation
   <222> 177
   <223> /replace="c" /replace="g" /replace="t"
<220>
   <221> variation
   <222> 201
   <223> /replace="g"
<400> 17
   caaccaactg gagaggtttt taaactccca gtatccgggg catcagtatt cgtctagggc     60
   acagtgattc taacacacag tcaaagttga gaaccactta tttaattcat cctgtaaatc    120
   ctcacaatag aatcctgcaa ggtagctttt atcaccacct ttgccgatga aattgaagct    180
   caaagaggta aattgatttc agcatctgta gccccaacct gtgacctctc cttccttcct    240
   tctcgggctc ccttcaggga tttcctgatg ttacagaaaa tggtatgtat gacgctgccc    300
   tcctggccac cttgacatag ctctgctccc aacagggctc ttctgagata gcagccagac    360
   actacctggg atcaggactg atactaaatg acttctgcca a                        401
<210> 18
   <211> 401
   <212> DNA
   <213> Artificial sequence
<220>
   <221> source
   <223> /note="Description of artificial sequence: rs965118"
<220>
   <221> variation
   <222> 201
   <223> /replace="t"
<400> 18
   tccaagagct gctttataaa ttggatgcat acgggggctg gttctaggat taagactggt     60
   agtagtaggg taaaatgatt aaggggcaaa ttcttttaaa agtaatctat agtaaatttg    120
   ttcactggct tccacatttc aggagtgaaa ttgcacgcag tgaattgctg catagcggta    180
   tttgttagca agttctattt actacttagt agtttatgtc ggtacatcca taccactatg    240
   gtttaatagt ggtttaattg cccacctagg aatgatgatg aacatggtta aatacagatt    300
   atttttagat tgcatgaaat attttggttt tgcccaattt tagggtgaga acatataccc    360
   taaatgaatt ttggggtaag aatgtgaagt atcaaaaaaa a                        401
<210> 19
   <211> 401
   <212> DNA
   <213> Artificial sequence
<220>
   <221> source
   <223> /note="Description of artificial sequence: rs1109089"
<220>
   <221> variation
   <222> 201
   <223> /replace="a"
<400> 19
   ttctgttcat tagacgcacg tcactaggtc catgcaggga gatgacacac agtgtggcca     60
   ctaggagagc aggagatctg ggagccggct ccaagctgcc tgccacacag tcctagttct    120
   ttcagctgtt tctcacgtga cagggtactg tatttttgcc caagtggcga cacttttaaa    180
   gataggcaaa tacagcatgc ggaggaaacc gtggttctgt aatgcaaccg caggtcctta    240
   ggacacccat atcccatttg ttgacccaca gcgagcttgt aggggggtta aatttcattt    300
   ttgtttttat caaatgacag cacacatcgt ttaaaatagc acagtgtgta attcattcaa    360
   caagctaact tattctgaag cttgtgatgc aatcatgaac a                        401
<210> 20
   <211> 401
   <212> DNA
   <213> Artificial sequence
<220>
   <221> source
   <223> /note="Description of artificial sequence: rs2980872"
<220>
   <221> variation
   <222> 201
   <223> /replace="t"
<220>
   <221> variation
   <222> 205
   <223> /replace="c" /replace="g" /replace="t"
<400> 20
   gggggacacg caaatgtgtc tctaagtcag ggcaccctct gtgtgcacag agccatcgag     60
   ggcactcgcc tggctggacc ccagggaagg aaatgggatg gcttagtgga gcagcccagt    120
   gtgacttccc agagctgact tcccaaaaca tgccacgtcc tcatcaaata aaatcaaacc    180
   caaacccctc taggctgctg cgccagacct tgcttactct aacctcatct caccatcatg    240
   ctccagccat gccttcttct ctatgtaata gtgctgggca ctttctgcca caggaccttt    300
   gcacacgcag agtcctcttc caggggcctc ttcctcacca cccacccctc tgcttctccc    360
   caccaactcc tacttcccct tcaggtttca gctacagtcc c                        401
<210> 21
   <211> 401
   <212> DNA
   <213> Artificial sequence
<220>
   <221> source
   <223> /note="Description of artificial sequence: rs2025949"
<220>
   <221> variation
   <222> 99
   <223> /replace="c" /replace="g" /replace="t"
<220>
   <221> variation
   <222> 201
   <223> /replace="a"
<400> 21
   gacatctcaa tttccaccct cttcataaaa taaacaaaca cacaaacttt gaaatacaat     60
   aaagctatta gttaaccact ggcaagtttg ctacaaaaag gcttatcaat gatgaatttg    120
   ccttttgatt tatttatcat tgtgaaatac tgtcaaccct catttacaaa ttagaattcc    180
   taaaccaaac tggtaaaact gagattctta aattgaacaa aactattaaa tgctatactg    240
   tagcattaaa tatttacagc attttaactt ttatattttt gtatgtaaca ctgaaaattt    300
   ttataatggc ttttgttcta atttgcatat tgaatataca aacgctgaac aggagagaaa    360
   ttaaaagaag tgatgtcttt tccaagttaa aatctctctg g                        401
<210> 22
   <211> 401
   <212> DNA
   <213> Artificial sequence
<220>
   <221> source
   <223> /note="Description of artificial sequence: rs12142266"
<220>
   <221> variation
   <222> 201
   <223> /replace="c"
<220>
   <221> variation
   <222> 293
   <223> /replace="c" /replace="g" /replace="t"
<400> 22
   tctctccacc aaaaggaacc aggcctcctt ggagaaatgg ctgattccag gattggggca     60
   gggaaattac aagatgagtc tagactatct atctttttat ggccaaaaat aaggaggtgt    120
   tcaagcaaga tgaggacatg ttaaaaggac acaaaaggca gcttgaaggg gctcccactg    180
   gtcaaatctg caacaattca tacatcaaaa aatgccagta gtaataaagc ataatctatt    240
   gaactaaata agaaacccat gagtccacat tggtataaat aaatgaatta ttacataaag    300
   ggggaggaga gaaacttcct ttgtgtatta gaaagcctac taataaatgt aaaaggagtt    360
   atagagttag aaaatcacca tttggcatca gtaataatta t                        401
<210> 23
   <211> 401
   <212> DNA
   <213> Artificial sequence
<220>
   <221> source
   <223> /note="Description of artificial sequence: rs9877859"
<220>
   <221> variation
   <222> 201
   <223> /replace="t"
<400> 23
   caacaagaga tagatatgca tgtttgctgc atgccagata tcttttattt aattctacag     60
   ctttttccag aatggctgcc aggtagagca cctgcatcat tttggcaaag tcctctttgg    120
   agtggggtct tcaagcctga tcttggaatt gcgttttatt attgtctgcc taggactcag    180
   gtctgcactg tgggacacaa cgctaagtgg aaggcccctt atccatggca ccatggaagc    240
   aggcgcagga tgcatgggaa actgcaatgg cattaaagga ggaagctcca agtggatttg    300
   gagccattct ataactcctt gaaaacaaga ctacctcggg gatatttagg atgactcgtg    360
   tgtcaccaag tgagaaatac ctagtgttgg aacatttcca g                        401
<210> 24
   <211> 401
   <212> DNA
   <213> Artificial sequence
<220>
   <221> source
   <223> /note="Description of artificial sequence: rs2070439"
<220>
   <221> variation
   <222> 201
   <223> /replace="g"
<400> 24
   tcagctgctt gggaggctga catgggagga tcccttgagc ccaggagttt gaggttagag     60
   tgagctgtgg tcgtgccttt gcattccagc ctgggcaaca acgagagacc ctgtttcaaa    120
   accaaaaggg agaatggatt accacagggg ctcgacatga tcaagtcagt ttagtaggaa    180
   cgacatgtga ggcatggccc tgtcacaggc atgaaggttc agaatatagc ctggagtaca    240
   catgtatccc tctgggccag gcagaggctt cacatcttgg tgaaaggcct ggtcgcactt    300
   tctctgggtt tcagatggac tgttaatgca aagttgtttt tgtgtttttg caaatcccag    360
   gagctggtgg atggggatgg tgtgaccctg ttggaacctg c                        401
<210> 25
   <211> 401
   <212> DNA
   <213> Artificial sequence
<220>
   <221> source
   <223> /note="Description of artificial sequence: rs12617566"
<220>
   <221> variation
   <222> 201
   <223> /replace="g"
<400> 25
   acaactaaat gtcagtaagc ctggaaaaca acagagctct agggagttca gctgcaggag 60
   ttcatggact tttactctaa tgatcttcta ttacctcagc tagcaactta ttaagtttct 120
   gcatatctta gttcaaattc tcaaaaggga atctgattaa ttcagattag tttatggatt 180
   agttcctctg gggttggata acttctcttg gctcaatcag ccatgtcagg ggaatgacat 240
   tgctaatgaa ggataaacaa caggaaacaa gatatttctc tagtagagat atttatgatg 300
   ctggtccata tttatttaca tgtaaatgta taatataaaa caaactacat ggatattaat 360
   aaaatgtaat taccttattc taagacagta tatttataag c 401
<210> 26
   <211> 401
   <212> DNA
   <213> Artificial sequence
<220>
   <221> source
   <223> /note="Description of artificial sequence: rs1958421"
<220>
   <221> variation
   <222> 80
   <223> /replace="c" /replace="g" /replace="t"
<220>
<211> variation
   <222> 201
   <223> /replace="a"
<220>
   <221> variation
   <222> 312
   <223> /replace="c" /replace="g" /replace="t"
<400> 26
   ttatctttca gtaaattcct aatgaaataa caaagtgtat tgggttatct ccaactgccg 60
   taccatacta ctctctaaaa atagcaagtc aaaaatgact gactttgcca atatcctgag 120
   attctacaga catctcagtg aaggcagagg atggtgggga gagtggcatt tggctcagct 180
   tcagaactgg cactgtctcc gtgataaagg gatcctgtta acttctgttt tccacctcct 240
   gacagttgac attttaatgt gtgtcaggaa aatccatcaa catgctacct ctctcaaacc 300
   caggatttcg cattgggatc agtaggaatc tgatcagagc taacacaagc cctccttctc 360
   attaaagcat accccattcc cattgttgac tgtcctaatt t 401
<210> 27
   <211> 401
   <212> DNA
   <213> Artificial sequence
<220>
   <221> source
   <223> /note="Description of artificial sequence: rs9589698"
<220>
   <221> variation
   <222> 201
   <223> /replace="t"
<400> 27
   atatatataa cttagaaaat gtttgaatgg tatgattaga atggagtcat agtgttttgc     60
   aaaacatgga ttgttgaaat acaaaaatcc aggttttgaa gattattaaa taattcatgt    120
   aagatatcag agtggagctc caacaatttg ttctactctt atttggatat aatgtctgtg    180
   agtgattccg taaatgaggt cataaatatt catctatttt atcactggca atagctgaga    240
   atctctgaac agacagtgga ccacagaatt cagaccctct atgcctgtgc ctatccattg    300
   acttatccaa ctttatttcc atgttagata ccagcagttc agaaacattt gtatttccca    360
   gctgcggaaa cagaattatg cttctttcta ttaggatttg a                        401
<210> 28
   <211> 401
   <212> DNA
   <213> Artificial sequence
<220>
   <221> source
   <223> /note="Description of artificial sequence: rs7645289"
<220>
   <221> variation
   <222> 201
   <223> /replace="a"
<220>
   <221> variation
   <222> 342
   <223> /replace="c" /replace="g" /replace="t"
<400> 28
   aagtcacttg ctttttgttt ttttttcagt ctccagattg aataggcaga ctgcccaaat 60
   ctccctttct ttcatttttt cactctctct gtcagaagaa gggggcatta taggacagta 120
   ttattaggct agatctttgc cttgttttag tgtcttatcc actggcccct tcatagggtt 180
   gggaaatggt ttcataagat ggtagaaagg gtggcatagg tacttgcagt atggctaaca 240
   gttgttcacc atgtagcttt agatatctta gacccagttc ccagctgtgt ctgccttagt 300
   cactctggtc ctcgtcttcc tcagtatctc ctctatgagg taggcatcca tgcaattcac 360
   tccaattgct tgtacattct tacattaagt tttgggggat c 401
<210> 29
   <211> 401
   <212> DNA
   <213> Artificial sequence
<220>
   <221> source
   <223> /note="Description of artificial sequence: rs2664537"
<220>
   <221> variation
   <222> 201
   <223> /replace="g"
<220>
   <221> variation
   <222> 206
   <223> /replace="c" /replace="g" /replace="t"
<400> 29
   ctgtgtctat gaatatgact atgaactctg aactatttta tccattggaa ggtaaaggaa 60
   aggtcctaca ttgtgggagg aagaactgat gagaacccca tcttgcttgc tgcttgcttg 120
   gtggtgggca ggccgagggt agcggcaggc tctgggctgt ctgcgaggat tctggaagct 180
   ctcccaggtg cccagcagcc aggcaaggga acggcatgtg gcagcagaga gtcgggtttg 240
   gctcttccac gtggcaggcg gtttcccaga ctggccagtc cttcacatta atcagatcaa 300
   attcagtctg tttctgagaa gaaaacacat gcctgtcact ctacggcagc tgccaccacc 360
   tgccccccag gcagcctggt ccttgctata ccagggtggc a 401
<210> 30
   <211> 401
   <212> DNA
   <213> Artificial sequence
<220>
   <221> source
   <223> /note="Description of artificial sequence: rs2228246"
<220>
   <221> variation
   <222> 60
   <223> /replace="c" /replace="g" /replace="t"
<220>
   <221> variation
   <222> 140
   <223> /replace="c" /replace="g" /replace="t"
<220>
   <221> variation
   <222> 201
   <223> /replace="a"
<220>
   <221> variation
   <222> 374
   <223> /replace="c" /replace="g" /replace="t"
<400> 30
   tttgccgagt gtcccttcct gagttccagc agttccttct tgactaccag ggggtatgga 60
   tgggctgaca ttggcccagg ctggtaggtt gtggggggct gggctcatcc ctgactggag 120
   gcttctctca tcccctgcca tccctacccc atcaggagct gtgggctgtt gatcgcctcc 180
   aggtgcagga gttcatgctc ggcttcctcc gagacccctt acgagagatc gaggagccat 240
   acttcttcct ggatgaggtg agcccgatgt ttcacccatt ttttgtcaag agaatgagta 300
   ggggtgacca ggaccccacc cgggctccag gagctagacg ctccttaggg atgccacctt 360
   gtttctacct actatgcacc ttgcccaccc ccagttggga c 401
<210> 31
   <211> 401
   <212> DNA
   <213> Artificial sequence
<220>
   <221> source
   <223> /note="Description of artificial sequence: rs2088138"
<220>
   <221> variation
   <222> 201
   <223> /replace="c"
<400> 31
   gtgtcacaga cagcccccac cccaagctcc cttccaggta tccccataaa tcatttgggc 60
   acactccctc ttggaattgc aatttccatc ttccacttat ccctaaggag ctggccctgt 120
   ggagaggtgt gttggttgtt ttgtttttgc caaaggccct cgctctaggt gctcctgtaa 180
   aggtaccttg gcccctgata tgggatgaat ggatgaacga agccccagat gctccagtgc 240
   caaggaggtc agaccgggaa caggccccca ctcccacacc ctgggatcgg agcttgcttg 300
   gttcttgcca tcttgcagga tgacttcgag tctggaggga gatacacagt gcctctccat 360
   ccctggggtg gggggaaggg attctgctgg gactcttgag t 401
<210> 32
   <211> 401
   <212> DNA
   <213> Artificial sequence
<220>
   <221> source
   <223> /note="Description of artificial sequence: rs2839178"
<220>
   <221> variation
   <222> 201
   <223> /replace="g"
<220>
   <221> variation
   <222> 328
   <223> /replace="c" /replace="g" /replace="t"
<400> 32
   caccctataa atatcagttg acatacagag tagccaccac gtcccaatct aactcctgac 60
   ttttcccctt atattgtatg tgtgtgtgtg tgtgtgtgta atcaagtatt tctgaaatgc 120
   agtataatcc aattagtcta ttttaggtat tcattgctat aacattttcc ctcatccttt 180
   taccactgaa tacagaaact aatagctcat atattttgaa tttgttaaaa gactcttccc 240
   cgtgtgtgtg gattttgcct gtaggaacac aggactcacc gctgtagata cttgcagaag 300
   cactgaagct cctggagggt ctcctttaca gtctggaaga tttcctccac gagaaacaag 360
   tccactaagt gggcacagac atcctcacag caacgggcca c 401
<210> 33
   <211> 401
   <212> DNA
   <213> Artificial sequence
<220>
   <221> source
   <223> /note="Description of artificial sequence: rs6500497"
<220>
   <221> variation
   <222> 201
   <223> /replace="a"
<220>
   <221> variation
   <222> 259
   <223> /replace="c" /replace="g" /replace="t"
<400> 33
   agggctcccg gaggacagga ccttccaggg cctccctgcc ccgaacccat aggtgccagg     60
   cttctcactg ctcacgacct ccgtgccggg ctcatacctg ccgcgccagc ccctcttcag    120
   cagagcacaa gtctggcagc aggagggcac cagccagggg ccccgcgccc ccgcctctgt    180
   acagcagggt caccgtgggg tgacatgaga acaccgaccc agggaatggg ctcctgccac    240
   acctgcctgg gaacctggac tgtgagtcct gctcgctgca ccctctagcc tgacgttgaa    300
   gtaacctcgc cagcggggcc ctctgtggca ctgggcactc agggagagca cgtggcattg    360
   gagaaggtat tctgagtcct acagagaggg acctgcgggg c                        401
<210> 34
   <211> 401
   <212> DNA
   <213> Artificial sequence
<220>
   <221> source
   <223> /note="Description of artificial sequence: rs11258903"
<220>
   <221> variation
   <222> 59
   <223> /replace="c" /replace="g" /replace="t"
<220>
   <221> variation
   <222> 71
   <223> /replace="c" /replace="g" /replace="t"
<220>
   <221> variation
   <222> 95
   <223> /replace="c" /replace="g" /replace="t"
<220>
   <221> variation
   <222> 199
   <223> /replace="c" /replace="g" /replace="t"
<220>
   <221> variation
   <222> 201
   <223> /replace="c"
<400> 34
   aaaattttaa attaaatcca aaaatacata aaattaacaa cgttcaaatt tattgcttaa 60
   ccctgatatt aatatttttt ggctgaaatg aaatagcagt ttttagcatg aatatgtttc 120
   tgactcctct tctgtgggtt cttttgagat tggcatgtct atattgcccc gtgctgggtt 180
   aggagtcaat tctcctatag tttttttcct acccaacctt ctgcaaacct tgaacaatac 240
   agcgagaggc ccttagctta catttggcag aaccacatca tttgttacta agtaaatctc 300
   tagtctttct ttgttggtct gaaagaaata tggcaggcaa aacaatcctg ggccagtact 360
   caactatgaa gtggtcatca ggtggtcccc aatcctctta t 401
<210> 35
   <211> 401
   <212> DNA
   <213> Artificial sequence
<220>
   <221> source
   <223> /note="Description of artificial sequence: rs2244057"
<220>
   <221> variation
   <222> 73
   <223> /replace="c" /replace="g" /replace="t"
<220>
   <221> variation
   <222> 201
   <223> /replace="t"
<400> 35
   ttgcccccca tagtttctga gagggctctt cctggcccac tgctttagtt accactcata 60
   ttcacatctc tcagacttct ctcccacgct ttttctcaag atatgtgaga taaaactcaa 120
   cattttcctt tgtgaacatg cccctctgcc tgtatgcctc atcttattcg atgctaactt 180
   ctctacccag tcaccgaaga caaaaatgca ggcatcataa taatctttct tctttaatta 240
   ttcataccct ctcctagtct ttatcatagt gaattgatta aatagtcatg ctcatttcct 300
   actaaatatt ttttggcatc tctccatcct ctctcctctc actcattagt ttacactttt 360
   tcaccttctg ctaaaaccat tgcaaggcct tcttaactga c 401
<210> 36
   <211> 401
   <212> DNA
   <213> Artificial sequence
<220>
   <221> source
   <223> /note="Description of artificial sequence: rs1459841"
<220>
   <221> variation
   <222> 201
   <223> /replace="a"
<220>
   <221> variation
   <222> 376
   <223> /replace="c" /replace="g" /replace="t"
<400> 36
   tttcctttta tggattgtat ttcatgcatg gagtctaaga actctttgcc tagccctaat      60
   tctcaaattt tccttgtgtt ttcctaaaag ttttgtaatt ttacatttta catttatgtt     120
   tacaatccat ttttggttaa tttttctttg aggcatgaag ttttcttgcc tatgttcatc     180
   tttttggata tggatgtcca gttgctccag gatttgtcaa aaagactgct atcctcccac     240
   cattaaattg tttttgtacc tgtgcaccat acgaaacagc acattcctat gggtttattt     300
   ctgggttctc tattatgctt cattgttaca tgtgtctatc tctctgccca tgccattcca     360
   aataaaccat agaataggtt tgtatattta attaattaat t                         401
<210> 37
   <211> 401
   <212> DNA
   <213> Artificial sequence
<220>
   <221> source
   <223> /note="Description of artificial sequence: rs7901463"
<220>
   <221> variation
   <222> 201
   <223> /replace="t"
<400> 37
   ttaatttatc tctcatttgt gaacactgta atcatttctc tatcccacat ttatttagct      60
   ctcaaagcta tgctatttta ttgtacaatg actaaactac tcaacatgag agatttggga     120
   ggatctttgt ggtcagctta ttattctttt cattaccaca acaacagcac tacaaactct     180
   ttttctaagt gtttctccat ctatgatctc acttttgtgg ctagacctca cctctagccc     240
   ctgggaattt cttctgtgaa cgcagcagga tgacatccct caagtcaaat tccaaagcct     300
   taaagtagat cagcagatca gggcccccta atgactgatt acacacagct ctgaccccac     360
   acttagcccc gagcttgttg ttttctgcag ccttttatcc c                         401
<210> 38
   <211> 401
   <212> DNA
   <213> Artificial sequence
<220>
   <221> source
   <223> /note="Description of artificial sequence: rs584762"
<220>
   <221> variation
   <222> 199
   <223> /replace="c" /replace="g" /replace="t"
<220>
   <221> variation
   <222> 201
   <223> /replace="a"
<400> 38
   gatgttattg aaaaagattg ggaagattat gggtagtaat agagataatt gcaaaggaga      60
   aacaagaaaa aaaattaatg aatcttccat ctcagggaga aataaatggc tttattatgg     120
   ggtcaggagg gatgatccta aaattccctg tccaagagaa aatgcatagt ggtagtttgg     180
   gagttggaga atttggcaac cacaacatcc atgaatcaaa acagctttta agggacaacc     240
   tcaggggatt gcatttctat aaaaggaatg agatgttttt ctttatgctg ctgaaaatgc     300
   agaatcagag ccgactagaa gcacagctag ctcctttgtc attatatgca aaggacaaga     360
   agaaacatga tgttccacta taatagggcc ataggcaaaa a                         401
<210> 39
   <211> 401
   <212> DNA
   <213> Artificial sequence
<220>
   <221> source
   <223> /note="Description of artificial sequence: rs761453"
<220>
   <221> variation
   <222> 79
   <223> /replace="c" /replace="g" /replace="t"
<220>
   <221> variation
   <222> 201
   <223> /replace="g"
<400> 39
   caagtaagta aggatgtgta taaagaccct agcccataaa tggtcatcac taaataggta 60
   gtcattattt atcattggaa tctttatttg tgagctgcaa tggatatgta aataaatata 120
   ggtatgtatg catgatgcac aggtatccac ctgtgtatag cagttgcact atttcccttg 180
   cctaaggaga aactgaaatg atagtttagg ccaagagatg attcagaccc atgtgtatga 240
   ctaattacag tacttaacaa gaaattgtaa taatatccat tggtatagct agaggtatga 300
   gtttctgcta actcaggatc tagtgtcacc ttcaacaaac tccccttatt ctgaatgtat 360
   ctagaagctc taaatgcttt gctggaaact gaaagggtag a 401
<210> 40
   <211> 401
   <212> DNA
   <213> Artificial sequence
<220>
   <221> source
   <223> /note="Description of artificial sequence: rs6035712"
<220>
   <221> variation
   <222> 201
   <223> /replace="g"
<220>
   <221> variation
   <222> 392
   <223> /replace="c" /replace="g" /replace="t"
<400> 40
   tgcccggcct gttcatctgt tttcacacaa ctaaagttgc tcagttgttg tgctggaaag 60
   gacttccctg cccaaaatat accagcaaga ccatcatgca accggagcag gctgtgccac 120
   agaccctggc atgcagaggt gttgtgtaaa cacagaaggg atctgggcaa atgcatgcct 180
   tcatttataa cagataaaaa taaagaagtg tgccagacag tgaggcactg acgcttcaca 240
   ggagggttcc gagagctttc ctccctgcct gaagttcctt ctcaacccaa gtagccacct 300
   tgtcctcgtg tttgtagttc ccctctcaat ccaagcacta tccttggcct gggggggtca 360
   tcatcaccac cagcaagggt cagcactgca gagtgacaat c 401
<210> 41
   <211> 401
   <212> DNA
   <213> Artificial sequence
<220>
   <221> source
   <223> /note="Description of artificial sequence: rs554710"
<220>
   <221> variation
   <222> 132
   <223> /replace="c" /replace="g" /replace="t"
<220>
   <221> variation
   <222> 201
   <223> /replace="c"
<220>
   <221> variation
   <222> 397
   <223> /replace="c" /replace="g" /replace="t"
<400> 41
   cacagagaga ggacttctgt cctgatagac gtcggtgagg actgaatccc cacttacagg     60
   cgtgcacatc agggactgat ggacatcgct gaggaccgat cccagcttac aggcgtgcac    120
   actggaggct cagaagagct gactgtgact cacttccggc ttccccccag gacaaaacct    180
   gcctctcctt ccagactcgc tgacttccct tcatgtcccg ctgtgatgtg cagtccagca    240
   tccttgggtc atgacaccag ctgcactggc acagggaatg agagaatatt cctgaaaatg    300
   aagactaccg cggaaggcag gagcttctaa gctagagtga ctggcagctt caacaggccc    360
   tgctgcacct gaaccagccc ggggcccccc agtgccacgg a                         401
<210> 42
   <211> 401
   <212> DNA
   <213> Artificial sequence
<220>
   <221> source
   <223> /note="Description of artificial sequence: rs6868846"
<220>
   <221> variation
   <222> 201
   <223> /replace="g"
<220>
   <221> variation
   <222> 279
   <223> /replace="c" /replace="g" /replace="t"
<400> 42
   aaagaaatat ggattggcta tcaagggact tccaccacta atcaatattg gacaaagatc 60
   atctcacttc tctgtgtgtc tatgtcttca cttttaaagt gagagagaca ggctgagtgt 120
   ttatactaga aaaagcttta atttcaagga aatctgacta gaatcccaac atggaaaaac 180
   agatgaaaac gggttcattc attgaagcag agatgggggc cggaaggaac gtaaggatga 240
   aatggaggga tgggggaaat ggagaggtca gtgaaggtat gacaagtaga agcagcagtg 300
   gcagtgacat agttaaactg ggaaacaaac aggatgtggc aactggaagg caggctgtct 360
   gaactaggga ttttggaagc agaccggtgt tgcatcttaa g 401
<210> 43
   <211> 401
   <212> DNA
   <213> Artificial sequence
<220>
   <221> source
   <223> /note="Description of artificial sequence: rs1013120"
<220>
   <221> variation
   <222> 137
   <223> /replace="c" /replace="g" /replace="t"
<220>
   <221> variation
   <222> 201
   <223> /replace="a"
<220>
   <221> variation
   <222> 239
   <223> /replace="c" /replace="g" /replace="t"
<400> 43
   caaacttaaa aataacaatt ttcccagaag gccaaatgtt tagggaacat tttcccccag 60
   aggactcagg ggtaaaacga atctgaagac cttaattatt caatgtcccc aagcctctct 120
   tcctaaacgt ggaatgagga tgagaaggaa tcacttacaa gagagagaac tgtgtgctaa 180
   atatcaggag acaagtcgcc caaaattcta ggtaatggta gtaaagtaag aataggcaac 240
   ctaaggtatc aaaggtcaaa cgtggtcccc caaacacctg acctgcaaac ctaggcagat 300
   agcagctcta gagaagaaga aatggctgga gcagagagta cgtggctcag tatattagac 360
   cttatttgcc acatagatga cttccaaaaa tactcttttg g 401
<210> 44
   <211> 401
   <212> DNA
   <213> Artificial sequence
<220>
   <221> source
   <223> /note="Description of artificial sequence: rs649867"
<220>
   <221> variation
   <222> 201
   <223> /replace="a"
<400> 44
   tgtggggtgt ccctgtgtct ccggggtgac ttgggcctcc agagaacccc tggcacaacc     60
   tccagctgaa aacaggaaca aagaaagtga gcttaacatt ccggctgttt cagacaatgt    120
   gtttctcttt gtgggggcaa ggagggcagg gagacacctg aaaccatttt tattcatcag    180
   ctcaagttgg tgggtggcag ggtcatgttg agggaaaaaa gccaaactct gtaaaatatt    240
   tagagaggtt tcttctgagc caaatatgag tggtcaaggc ccgaggcaca gtctcaagag    300
   gtcctgagaa catgcgccca ggttggcttc agacatttag ggggacaaaa gttacaggca    360
   gacgccaatc agtacatgtg aggtgtgcgt ggggttggtc t                        401
<210> 45
   <211> 401
   <212> DNA
   <213> Artificial sequence
<220>
   <221> source
   <223> /note="Description of artificial sequence: rs6072317"
<220>
   <221> variation
   <222> 186
   <223> /replace="c" /replace="g" /replace="t"
<220>
   <221> variation
   <222> 201
   <223> /replace="g"
<400> 45
   aaagtaatct tcagttcttc ctcacccatt acttatatat acaatatatt attataaagg     60
   agacaacaca gaatatagaa ggcagatttc taaggtcccc tattagaggt ttataattct    120
   tttataaaat gttatctttt ctcttcctca acacaacaaa cgaaccccat taaagtcaaa    180
   actgaaaact ctggccagga ttctgttata tgtaagatgc ctccttgttg atgttgctat    240
   agtcctaatt aacagtcgac aggacatttc aagacttctc actcttatca ccagtcctgg    300
   aaaagttgtc tttttctata actgaggaca tggaatccca tctctagggc cacacaggat    360
   aaaccaaagc caaccttgac cttaaggaaa tttgtaagga c                        401
<210> 46
   <211> 401
   <212> DNA
   <213> Artificial sequence
<220>
   <221> source
   <223> /note="Description of artificial sequence: rs4795069"
<220>
   <221> variation
   <222> 201
   <223> /replace="c"
<220>
   <221> variation
   <222> 318
   <223> /replace="c" /replace="g" /replace="t"
<220>
   <221> variation
   <222> 338
   <223> /replace="c" /replace="g" /replace="t"
<400> 46
   ctgggagcta tttaaaaatg tagaatctcg ggccccatcc tagaccttct gaatcagaat     60
   tttagcaata tttccatgtg cttcatatgc acattatatg ttacagtttg agaggcattg    120
   ttctagaagg agctcaaaga ctcagggatg tgagaatatt gaaatggagc tactatgtgc    180
   aacacgcttc gacactaccc acctagccag aatccccgag caagcccagg ggatgcacct    240
   atcaccaagg aattaagaca tgcattggag aggaaggtac tagcagtgct ggagtgaggg    300
   gggaggcaag aagacacaca ggatacaaca tttaaccagg cacccaccct ccggtactga    360
   tcctgaatgc atgagcctga aagtgaatgc ctcctttaat a                        401
<210> 47
   <211> 401
   <212> DNA
   <213> Artificial sequence
<220>
   <221> source
   <223> /note="Description of artificial sequence: rs6948196"
<220>
   <221> variation
   <222> 201
   <223> /replace="c"
<220>
   <221> variation
   <222> 279
   <223> /replace="c" /replace="g" /replace="t"
<400> 47
   tgacaaccag cttttaatta tggtctaatg gtaaactagg ttaccaaagc tctctgtaca     60
   ggtaaaatcc cagtgaaaga tgtaccaggc tgaagacagt ttacgtaaca ctaccatttt    120
   aagttgagtt acttcctatc gcgggactac tccgttgtgg tagaaataac ggaatgtcag    180
   ggagctggta cttttggcca tcacggcaac agttaataca aagcactccc tgaaagccag    240
   actcccagag ttataggtga cgtgtcctta aaagctttaa cattgtaaat accaggaaaa    300
   gtacagggga ctggcaggga gaattgtata gactacctga gaattatatt ttacctattg    360
   gtagttctga caacactcaa aaaaaaagaa aaaaagaaaa c                        401
<210> 48
   <211> 401
   <212> DNA
   <213> Artificial sequence
<220>
   <221> source
   <223> /note="Description of artificial sequence: rs13403584"
<220>
   <221> variation
   <222> 201
   <223> /replace="a"
<220>
   <221> variation
   <222> 215
   <223> /replace="c" /replace="g" /replace="t"
<400> 48
   ttttcactag attcaataaa agctcacgca agtgtaagta taagagtgtt gggcttaatt     60
   atggtttggc ctactatttc cttttacttc atgttatatc tgaaaggaat ttactcatca    120
   tggagaaaat aagctacatt aacactaaaa cacagtgaga aacaaatatt gctccaatgc    180
   tagaaaaaaa gaaaatttcc tgggctcaaa agagatgagt ttcaaggaca gttgacacct    240
   ataccctact tactaagata gagtgaatca gatatagcta taatttggcc tctgtaggtg    300
   tatttcagct gtttcatttc ttttcaggga agaaagatgg tgtatgaaac tttttccaag    360
   atggtctctg aaatgagtta aacttcggtg ttcaaattgt g                        401
<210> 49
   <211> 401
   <212> DNA
   <213> Artificial sequence
<220>
   <221> source
   <223> /note="Description of artificial sequence: rs7685314"
<220>
   <221> variation
   <222> 1
   <223> /replace="c" /replace="g" /replace="t"
<220>
   <221> variation
   <222> 201
   <223> /replace="g"
<400> 49
   attactctag aaagaagata agaattgttt tcatatattt gaagaatttt tacaaggtag      60
   atatcccaaa ccagaaatgt tatataaact aatagctggg taacttcaca caaagttgaa     120
   ataacatgga tatagcacag tttaaagggt gatctttcac attagtcaag aatttttatc     180
   agacagattc atgaatttat agcttttata ataaaaatta tttattaaat ggggttgttt     240
   atagtttgga ctaattgagt tcatagatca agggaaaaag cagaaaaaga ggtttacact     300
   gtgttcttaa aatcttgagt ttttgtgttc ctaaaggaca cattttagtg aaaaaaatgt     360
   ttaaaaattt atctggaaat tagaatgttt aaggcctaaa t                         401
<210> 50
   <211> 401
   <212> DNA
   <213> Artificial sequence
<220>
   <221> source
   <223> /note="Description of artificial sequence: rs2662090"
<220>
   <221> variation
   <222> 87
   <223> /replace="c" /replace="g" /replace="t"
<220>
   <221> variation
   <222> 201
   <223> /replace="a"
<400> 50
   cttcaggtgg ttgaagctta aatgaggcgg acagagatga gctacagagc cccaggctgt      60
   aatttgcgct aaccagtgga tgacctacca aggcatgagt taggcatttg ggattacatc     120
   ttcaacttct tggagcagtg agacacggtg gacagaatat cagcttaccc ctcagctctg     180
   cttttttggg gaattcttgg ccaggagata aaatgagagg acattattgc tggaagcatc    240
   tatagagagc ctctaggaca gtagttacca aactgaagtg tgcatttgaa ccatctggga     300
   agcttgttaa aaatgcaggt tctcagccag gcgcagtggc tcatgcctgt aatcccaaca     360
   ctttgggaac cgaggtgtgc agattgcttg agctcgtgag t                         401
<210> 51
   <211> 401
   <212> DNA
   <213> Artificial sequence
<220>
   <221> source
   <223> /note="Description of artificial sequence: rs6500498"
<220>
   <221> variation
   <222> 143
   <223> /replace="c" /replace="g" /replace="t"
<220>
   <221> variation
   <222> 201
   <223> /replace="c"
<400> 51
   ggcttctcac tgctcacgac ctccgtgccg ggctcatacc tgccgcgcca gcccctcttc     60
   agcagagcac aagtctggca gcaggagggc accagccagg ggccccgcgc ccccgcctct    120
   gtacagcagg gtcaccgtgg ggagacatga gaacaccgac ccagggaatg ggctcctgcc    180
   acacctgcct gggaacctgg gctgtgagtc ctgctcgctg caccctctag cctgacgttg    240
   aagtaacctc gccagcgggg ccctctgtgg cactgggcac tcagggagag cacgtggcat    300
   tggagaaggt attctgagtc ctacagagag ggacctgcgg ggcctgcccc acaggcacgt    360
   ggggctcctg ctggggctgt caaggacccc cgggcactga g                        401
<210> 52
   <211> 401
   <212> DNA
   <213> Artificial sequence
<220>
   <221> source
   <223> /note="Description of artificial sequence: rs2074997"
<220>
   <221> variation
   <222> 201
   <223> /replace="t"
<220>
   <221> variation
   <222> 223
   <223> /replace="c" /replace="g" /replace="t"

<400> 52
   tttcctcaga catactccat agatattaat ggctatattc ttgtgtattc tgttacatca     60
   atcaaaaggt aagactcctg ctgcctgctt gagttgattt gctactcata gataacattg    120
   atccttttaa gggactgtgc tggaattatt aatagtgaca attagaatta ttaggctaca    180
   tgttttttag tgattttctc gtggagcaac tggattgctg ggaaacatgg cccactagat     240
   gttctgttag ttacaccaat aggataaaag gctctttctt acctcaagct acctagaaaa    300
   acagtggttc agcatgatct aagcttttct gctaattgaa atgggaaatg cctaaattaa    360
   ttttactttg tgtgtgtttt taccatatta aggtgaaata g                         401
<210> 53
   <211> 401
   <212> DNA
   <213> Artificial sequence
<220>
   <221> source
   <223> /note="Description of artificial sequence: rs1204798"
<220>
   <221> variation
   <222> 161
   <223> /replace="c" /replace="g" /replace="t"
<220>
   <221> variation
   <222> 201
   <223> /replace="g"
<220>
   <221> variation
   <222> 290
   <223> /replace="c" /replace="g" /replace="t"
<220>
   <221> variation
   <222> 299
   <223> /replace="c" /replace="g" /replace="t"
<400> 53
   ctaactacag atttaaaatc attaattatt gtcatacaaa agttaccaca ttataagact     60
   acactgattg tcaaataaag tgtcttcttc acatttgaag cactctctga aagatgacca    120
   tgacttgcta taatgctgct ttctgtggga cccatgtaac acacaaaatt gttaactctt    180
   caagctaaca aagccagcca atttcaggcc cccaaatttg aagatagatt ttccaaatat    240
   ttccagctgt tagaaactat tgagttcact accaaagcca gcttggtaca tagagtctat    300
   gtgctactct cttttttttc ctgattatgt ttatacttca aagagttgtc aggaagacta    360
   gtactcaccc accttctatt tccagtggaa tgttcacctt a                         401
<210> 54
   <211> 401
   <212> DNA
   <213> Artificial sequence
<220>
   <221> source
   <223> /note="Description of artificial sequence: rs12143647"
<220>
   <221> variation
   <222> 201
   <223> /replace="c"<
   220>
<221> variation
   <222> 380
   <223> /replace="c" /replace="g" /replace="t"
<400> 54
   ctcgaagcag ggcatgaggc cctttttgtt cttgcctggt agctctccac tgtagaaact     60
   ttccctccat agccctggat gctccagcca cacagtgcta gctgcctttc cccagctccc    120
   ttgtactcac gtgcttctgc gtttttcctc tgcctgccct ccccttcctg tgccctttgt    180
   tttcctgtca aactgcctct tcacatgaag ctccagctca gataccacct ccttcaagag    240
   accatccttg accgggcgta gtggctcaca cctgtgatcc cagcactttg ggaggccaag    300
   gcaggtgaat cgcttgaggc caggagttca agaccagcct gggcaacatg acaaaacccc    360
   atcttaaaaa aaaaaaaaga ccattttttc tcctaatgtt c                         401
<210> 55
   <211> 401
   <212> DNA
   <213> Artificial sequence
<220>
   <221> source
   <223> /note="Description of artificial sequence: rs3801033"
<220>
   <221> variation
   <222> 163
   <223> /replace="c" /replace="g" /replace="t"
<220>
   <221> variation
   <222> 201
   <223> /replace="c"
<220>
   <221> variation
   <222> 359
   <223> /replace="c" /replace="g" /replace="t"
<400> 55
   acctgactac tgtacttgct atcatgcaca ttaagtgaaa cgatatcaac cgtaacccct 60
   ataacctgtg ctttccctga acccactgcc aaaatatcaa cagatcaaat gagaaaaaaa 120
   tctgagagac aatacttttc accacctggg atcactatat gcagccaagc taggatgaca 180
   gtcatctaac aaaaatattc tgttgcagga aaagcaagct atttgttgca caaggaggtt 240
   cctaagataa gtccaagtag agagttgttc tggaggaagt gggtctgcac tttacacctt 300
   ctcatgtcac atcaagctgg gagagatagg tcatgatcta tctcttatcc ttgccttaag 360
   ctacgatcac tgtgtgcctg atcaatgccc agcacatttc a 401
<210> 56
   <211> 401
   <212> DNA
   <213> Artificial sequence
<220>
   <221> source
   <223> /note="Description of artificial sequence: rs10997044"
<220>
   <221> variation
   <222> 201
   <223> /replace="g"
<400> 56
   tgtaccaaaa gtaccctgtc ttttgccctc tcttctgaga gaaacattgc atttttctgc 60
   aatatctctt cctcgtgtcc tctatcctac atttatagag tctcaaatat gctcagcctc 120
   aaaattaaga tgctttgagc ttccagaaaa cactacacag ataaaggtgg ctttcaaaag 180
   agatgtttct tattgtctac ataatcatgc agagttaagc agtcactgca acctttcatc 240
   attttctaca ataaggccaa ctttccagat aaccacttag tgaaacaaga atcctgtatt 300
   tctatggtgg agctactgcg ggtctcatcc aacctcctgc catgtttggg ccactgactg 360
   tttgggcact tacgtccagc ttgcgaggat ggagccatgg g 401
<210> 57
   <211> 401
   <212> DNA
   <213> Artificial sequence
<220>
   <221> source
   <223> /note="Description of artificial sequence: rs7350731"
<220>
   <221> variation
   <222> 109
   <223> /replace="c" /replace="g" /replace="t"
<220>
   <221> variation
   <222> 201
   <223> /replace="g"
<400> 57
   tcagcctttc taacctcaca acttacttcc agaccacagc attaatccat gccctttatg      60
   ctccagtaaa atattttaaa gtcccaaaaa gtcttctctg acccctgtat cccccaggat     120
   ttgatatgta tcctttgcac ctcttgtgat atttacctga acatagcact tattgaaatt     180
   gtttaaagtt tttctactca atgaactgta aactcattaa gagaaggaac gatgtgcttt     240
   tgactgttgt ttcccaagca cctctcaagg cagttggcac attaacgtga tcaaccaata     300
   ttagttgagt aaatgaaatg agtcccgggg gactcacact agcttgaggg tcatgggcca     360
   attccaaact cactgtctgt agggggataa tgagtctgat g                         401
<210> 58
   <211> 401
   <212> DNA
   <213> Artificial sequence
<220>
   <221> source
   <223> /note="Description of artificial sequence: rs9323737"
<220>
   <221> variation
   <222> 201
   <223> /replace="a"
<400> 58
   tattccttct catccaaaat ttggttattt aaaatctcac tatttgaact catgaatcaa      60
   catgtttgga tatgtttcca cataaaataa ctcactactt gctatttcaa gttcagggta     120
   aattttttaa aagtgtaaac gatatcctac acctctttat tcaaatttac tatctaaact     180
   tgtgctgtcc aaattcagga ggtaggtagt tgtgggtagc aaggaatact taaagtggat     240
   tgtagctcaa gtcatacaat agcagcattt gttttcacga tccattaata tcatctctag     300
   tttacaacca tcaggagcct tttcatagca atgaactttc ctgtgataac tttgaaaaac     360
   aaaatctaaa tttgtcatgg cctacaatgc aatatatgaa a                         401
<210> 59
   <211> 401
   <212> DNA
   <213> Artificial sequence
<220>
   <221> source
   <223> /note="Description of artificial sequence: rs4881394"
<220>
   <221> variation
   <222> 60
   <223> /replace="c" /replace="g" /replace="t"
<220>
   <221> variation
   <222> 201
   <223> /replace="t"
<400> 59
   atcagtggca gagactctcc ctgctctcat gggctctgtc tattccctct ctacataata 60
   agagtaaaat ccaaagagca tgaggaccaa atcaaatatg acaaagactc cttagaggcg 120
   tgtcagtact ggtaacacct gtttttcaca actcacggtg actaaggcac agaccacccc 180
   acacagtggc acggattctc ctcttgaaca gactttcccc aggctcctct gagtctcttt 240
   tgaaataggc ctcgccttgg tctgtaagga tctgaacaca cccaaacata atttctaaca 300
   gttcaaggct acatcaatag ataaccaagc cctgttaaag agccagcctg agaaaactca 360
   cggcttccaa aagaattcag tatttgttcc agcaatgtct g 401
<210> 60
   <211> 401
   <212> DNA
   <213> Artificial sequence
<220>
   <221> source
   <223> /note="Description of artificial sequence: rs4799159"
<220>
   <221> variation
   <222> 201
   <223> /replace="g"
<400> 60
   tttattcaaa ccagaggaca aggaaaacta ctggatgaac acggttgtct tgggaagaga 60
   cctgcaggaa gctgccccag cagaggtaag cccggtacta acagcttgat tctcataaaa 120
   aggtggcaat aatcgtgaaa gtgtgagaag gaatgtggcg ttatccattt aatcccagaa 180
   ctgaggctgg gagttgtctc acagtgacgt gggccaggcc tctccccagc aagggcgatt 240
   aaagtgaaac cacttaggac atttggtcta tttcagatcc ctgccggtaa ccttttcctt 300
   cttttcacca actcttttca aacttaggtt tttttttccc ttctgtcttt gtattttccc 360
   gtaacatagc cctcggggat gtttacttag atttccctgt t 401
<210> 61
   <211> 401
   <212> DNA
   <213> Artificial sequence
<220>
   <221> source
   <223> /note="Description of artificial sequence: rs2270101"
<220>
   <221> variation
   <222> 3
   <223> /replace="c" /replace="g" /replace="t"
<220>
   <221> variation
   <222> 201
   <223> /replace="t"
<220>
   <221> variation
   <222> 242
   <223> /replace="c" /replace="g" /replace="t"
<400> 61
   acaaaggggc agacactgag acttcagcaa gttaggagtt tgtcagtaaa ttggaaccag     60
   agttgtttct ttctgggcta tgcagttgct agattgctct ctgctttagc cttgctgtct    120
   aattgtcata tgtaggaatc atgcaactgc agaactgtgt accctgtctc ttaaagcagc    180
   tctgtagtgg tcattctgag cgaattaatt taggattagc tcagaaataa ctaatgcctc    240
   cagggtagcc agaattgcag tctgcctatt aacatttgct ttgttccatt gctttagagt    300
   catattcaat ttatcaattt ccctcttcca gtttttggca tggtatcctt ttgtcccttt    360
   ctcctgtatc ataactctca gggagcctag cttgctatgt g                        401
<210> 62
   <211> 401
   <212> DNA
   <213> Artificial sequence
<220>
   <221> source
   <223> /note="Description of artificial sequence: rs356999"
<220>
   <221> variation
   <222> 9
   <223> /replace="c" /replace="g" /replace="t"
<220>
   <221> variation
   <222> 66
   <223> /replace="c" /replace="g" /replace="t"
<220>
   <221> variation
   <222> 201
   <223> /replace="c"
<400> 62
   aggcctaaag cctgactgac aagcagctgt ttgaaggaca ctgtccatcc aaacatggat     60
   tgtacagtgg acatactgtc ttctctggag ttacagaggg agttgggttc attgccaccc    120
   tcattaggtc acagaaatcc caataagata aaatggaaga tgctaccctg cctgaaagtg    180
   cccagcatgg tgcaggatgc tgatgggtac tcaataaatg gactgcaggt tgacttagtc    240
   ccccttccca agtgggcagt ttagattcta agtactttcc agttggctga gcagtttttc    300
   ccaagaagta ggctccttac tgttcctgat ttcttttgtc tatcccatca gaagcaggac    360
   aatcaaggca ggtctaagag agaactcatg gcaggagcag a                         401
<210> 63
   <211> 401
   <212> DNA
   <213> Artificial sequence
<220>
   <221> source
   <223> /note="Description of artificial sequence: rs618670"
<220>
   <221> variation
   <222> 30
   <223> /replace="c" /replace="g" /replace="t"
<220>
   <221> variation
   <222> 70
   <223> /replace="c" /replace="g" /replace="t"
<220>
   <221> variation
   <222> 201
   <223> /replace="g"
<220>
   <221> variation
   <222> 292
   <223> /replace="c" /replace="g" /replace="t"
<400> 63
   attaaatacc agtttatgaa tattgcacca ttagggcatt atcttttgcc agctttcctg 60
   ctcaccacta ccttgtcctt tattccgaat gtggacacat gggtaggagg cctgcaaacc 120
   cgtgatgaca tttctcagtg tgtggggcca tcttggatcc tggcagtgga tgagaaaggc 180
   gtgaaatggc aaggaacggc aatggtcaac tgaacagtgc atggttgtcc gtgggttgag 240
   gaaacgtgat gtccgtggat gttgtttgct cttttccaaa gatgcaaatg cagctgtgaa 300
   tacggaacca caatttgacc taaagacatg tctgcactca catgccaaaa tagtttgtca 360
   tttgctgtcc tttgataatt ccatgtaaaa gactatctca c 401
<210> 64
   <211> 401
   <212> DNA
   <213> Artificial sequence
<220>
   <221> source
   <223> /note="Description of artificial sequence: rs7788668"
<220>
   <221> variation
   <222> 201
   <223> /replace="c"
<400> 64
   cttttttttt ttttttaaga gataagatct cgttatattg cccaggctgg tctcaaattc 60
   ctgggctcga gtgaacctcc tgagtagttg ggactactac tcaccacaca tggcaataaa 120
   gattctaaag ttagaaggac aaagtgatgt tacctaaatt tgcctgtgct tctgatatga 180
   caaataatcc gccactggtg aatggcccat agagctcatc ttcatctttg gatgaatacg 240
   agtggacctg tcatactcct cagccctatg acatgtaaag cacaaagcca ggaatgaggt 300
   cattgtctta tcacttaccc ccaaaagctc tcaatggctc aacttaccta agtcttcctc 360
   ctttattctc ttccttcata tcaaaacttc tgcaaatttt t 401
<210> 65
   <211> 401
   <212> DNA
   <213> Artificial sequence
<220>
   <221> source
   <223> /note="Description of artificial sequence: rs9332172"
<220>
   <221> variation
   <222> 71
   <223> /replace="c" /replace="g" /replace="t"
<220>
   <221> variation
   <222> 201
   <223> /replace="a"
<220>
   <221> variation
   <222> 294
   <223> /replace="c" /replace="g" /replace="t"
<220>
   <221> variation
   <222> 390
   <223> /replace="c" /replace="g" /replace="t"
<400> 65
   attttagcaa aaaaaaatat gctgtgtgac tcagctagct gcaaagagcc tgatgaatgg     60
   aatttttagg aaagcatgga ataagggagt aggaaataaa gtttgggcaa gttggtctac    120
   agcctctgct atacaagcag tatttttttt ctagtactgt actttccagt ttctatgttg    180
   gtaactatat aactatgtga gtaattttga attcactgta atcaaatatg ctggtaaata    240
   atttgtcaga taattgcatc aaatcattcc taggaaaagc acaaccaacc atcagaattt    300
   actattgaaa gcttggaaaa cactgcagtt gacttgtttg gagctgggac agagacgaca    360
   agcacaaccc tgagatatgc tctccttcta ctgctgaagc a                        401
<210> 66
   <211> 401
   <212> DNA
   <213> Artificial sequence
<220>
   <221> source
   <223> /note="Description of artificial sequence: rs902923"
<220>
   <221> variation
   <222> 12
   <223> /replace="c" /replace="g" /replace="t"
<220>
   <221> variation
   <222> 83
   <223> /replace="c" /replace="g" /replace="t"
<220>
   <221> variation
   <222> 201
   <223> /replace="a"
<220>
   <221> variation
   <222> 374
   <223> /replace="c" /replace="g" /replace="t"
<400> 66
   aatgaaatgc aaatgtagta gccagaattt gagcagtcgt tttgatcatg aggtggaagc 60
   aatgtgcaga agatgatggg gaaaaaaagg aatttcaaca gatttttggg agagaaaaat 120
   cagatgatac aactggttta gaagggggaa gaaatttaca atccatggtg cctgtagaga 180
   ttgaggctaa tgggaagaga gtgttctgcc ttgcagaact tgtgaaatgc tcaggaattt 240
   gaggcagtac atacagtggt cagctggatt aatgagggct ggctggagac tgtggaacag 300
   tcagactcta gaacaccttc accctccaca ctcaggcaag tcactcctcc aggtcctcca 360
   accactgccc ctaaagatga gatgagtatt attcactgga g 401
<210> 67
   <211> 401
   <212> DNA
   <213> Artificial sequence
<220>
   <221> source
   <223> /note="Description of artificial sequence: rs4810622"
<220>
   <221> variation
   <222> 201
   <223> /replace="c"
<220>
   <221> variation
   <222> 305
   <223> /replace="c" /replace="g" /replace="t"
<400> 67
   taaaaacaaa tcaagagttg ggccagctgc atggaaggac ccaagaggat tatttaaatg 60
   gcatgccaaa tttcacccat gccacccaat gtcctcacca aacaggcaac ccaacgggag 120
   ctgttgtcct ctggatagcc ccagatgggt ccctaactct agctgctttc tcacctgaag 180
   atgcagagca cccactgaca ttcagaggag cttgctctcc agaggcccag ccatccccca 240
   cttctttaca aatgccctcc tagctttact tacttattca acaaacattc cagagcatct 300
   accaagtgca cagaccaagt accatagaga aggcaaatcc tcataaaacg gcctccctgc 360
   cctcaaggag cttccagtct agattgggag ggacaccccc a 401
<210> 68
   <211> 401
   <212> DNA
   <213> Artificial sequence
<220>
   <221> source
   <223> /note="Description of artificial sequence: rs6873640"
<220>
   <221> variation
   <222> 131
   <223> /replace="c" /replace="g" /replace="t"
<220>
   <221> variation
   <222> 201
   <223> /replace="t"
<400> 68
   tcccctctcc aaaactatga aagctcaggt aacaacaggc aacagcaaaa tcacccgatg     60
   cttgttggag tcctgacaac gttgacaaag tccttggcag gcccttctat tttcatgatc    120
   aatggtacta aagccagaaa caagcctcgc tggctgtgag ctgatctcta ctgtgaaaat    180
   gcacgcacgg tgtacacatt cgtttatatt ctaagcactg aggatgaaat aattctgcac    240
   cgtgtccatt tttgtgctgt taaggcatac ggttgctttg caagcaaaac agtagatctt    300
   tcatttgcca gtgacattcc acaaagtgct tataattcag tgttagctta tcagccgtcc    360
   aatctcccac tccagggaat gtgtcaacca tcaaaaggag c                        401
<210> 69
   <211> 401
   <212> DNA
   <213> Artificial sequence
<220>
   <221> source
   <223> /note="Description of artificial sequence: rs9302822"
<220>
   <221> variation
   <222> 145
   <223> /replace="c" /replace="g" /replace="t"
<220>
   <221> variation
   <222> 201
   <223> /replace="g"
<220>
   <221> variation
   <222> 289
   <223> /replace="c" /replace="g" /replace="t"
<220>
   <221> variation
   <222> 347
   <223> /replace="c" /replace="g" /replace="t"
<400> 69
   aatacctaag tagggtgatt ggaggtcaag gacccttgta ggaatgaagt ggatttgcct     60
   ggatgtaaaa ctacatctaa gatgttatga gtgaggaaga gcccaaaact cagaaatctg    120
   tttgaaaaac tttgttagaa agcaaacata cacatgcaat tgttttacat agagggcaat    180
   actgcaaaag ataatgaagg aactataaac agtgattatc tttgttgtag ctgtgcagta    240
   tgactaagag aggggcaggg aagagatgaa gcttttgctt ttaagattac acctttattc    300
   tttgtgattt tcagcaaaga tgccctattt ggataattaa aaaataatta agagaaaagc    360
   ctccattttg caaacaacct gtgatcactt tacccctgag c                         401
<210> 70
   <211> 401
   <212> DNA
   <213> Artificial sequence
<220>
   <221> source
   <223> /note="Description of artificial sequence: rs3753151"
<220>
   <221> variation
   <222> 91
   <223> /replace="c" /replace="g" /replace="t"
<220>
   <221> variation
   <222> 201
   <223> /replace="t"
<220>
   <221> variation
   <222> 266
   <223> /replace="c" /replace="g" /replace="t"
<400> 70
   taattctact gtctctcatt ttaacctcat gtcaaagtgg aaagctggct ttcttctgac     60
   atcactctcc ttgagcccct ttctccctgc atctttgaat caccagagaa tccatttcca    120
   acctaaaccc ctgaaattct cctaagtaga ctaatagcta tttactagta aatgctacta    180
   accacggtca tagaggtttc caaaatttgg tgttcacaaa gcaagatttg tgttactaaa    240
   gtgtggtgag ggctcttcat tcataaccta ctgagaagtc cacacccagg acgcagtgcc    300
   ttgagaatgc tgcaacacct aagtgcaacc tcccaacttt ctggaaggcg ctttgtcatt    360
   cactaacagt gctaccttga gatggcagaa gtcggaaatt g                         401
<210> 71
   <211> 401
   <212> DNA
   <213> Artificial sequence
<220>
   <221> source
   <223> /note="Description of artificial sequence: rs10490832"
<220>
   <221> variation
   <222> 201
   <223> /replace="t"
<400> 71
   aaaaatttcg gccaaagaca gacttcaaat aagaggtcat tgttaagggt tttaaattca     60
   aggagacaaa gtggcacggt aacacacata cacaatttaa tcggaaaaga aattacctca    120
   tagcccagta aatgtccatt gctcaacttc caaggaatgg tgttccatga aacctcaatt    180
   tctgaggaag ataggctatt ggcagagact tgagatgggg ccactgtagg ctctgttcgg    240
   aaacagaaat tgaaatatga tgataatttt ggcagttagt ttaaaacaca gattaatgtt    300
   cttattttta taaaagcatt ttaacgtatt tgaaaattgt atgaaatatt ctggcaccat    360
   atgttgaaaa tagagacact ggtccatttc cgtttcttag g                        401
<210> 72
   <211> 401
   <212> DNA
   <213> Artificial sequence
<220>
   <221> source
   <223> /note="Description of artificial sequence: rs1545384"
<220>
   <221> variation
   <222> 74
   <223> /replace="c" /replace="g" /replace="t"
<220>
   <221> variation
   <222> 201
   <223> /replace="c"
<220>
   <221> variation
   <222> 401
   <223> /replace="c" /replace="g" /replace="t"
<400> 72
   tttagggagc aaaccaaggt ggtataagag agtagattgc tacaatggaa tgtatggaaa 60
   tataaacaag ttaaaaacca tacatgactg cagtcaaaga tttctgtagc caaataaaca 120
   acaagcttcc tcctgggcat ttatggtttc aaaaataaat aagggcagag attacagtct 180
   cagagtctat ttacttgcac aagggtgaca cttcttttta tatccactgc tcagcaccag 240
   ctcaactctt ttctctggtt ccaacccctt tgcaattatc tgcctgagaa gaagagggac 300
   acatgtgggt atctagtggg agataaatgc agcatgacaa gcggattacc atggttgaaa 360
   cacttttctg aacacactgg agcttaaaaa gcctaaagct a 401
<210> 73
   <211> 401
   <212> DNA
   <213> Artificial sequence
<220>
   <221> source
   <223> /note="Description of artificial sequence: rs1884641"
<220>
   <221> variation
   <222> 201
   <223> /replace="t"
<400> 73
   atggatttta aaaggatctc aaatggtatc ttctaaaatt ctaagcattc tatcaatcct 60
   catgctgaaa tatgacttgg tgagtgacac ttcaaaaaga aaataaacat gcgtctcaac 120
   ccattactac tttatttcct ccagatcatt tcctcacagg cagggcagcc acgtcagtga 180
   acccactcag atatcttggt gtctaccact tggaagcctc ttttctacat tccattttgg 240
   gagagagctg gctcttcccg gagacttgac aacctggtac cgtctcctcc cacaatcacc 300
   ctcccattct tctggcttat ctctggtgtc agagaagaat gtaatcttta ggcgtgtaca 360
   ttctgactcc catgttttgg ggaagacccc tcagcgtagc t 401
<210> 74
   <211> 401
   <212> DNA
   <213> Artificial sequence
<220>
   <221> source
   <223> /note="Description of artificial sequence: rs1958438"
<220>
   <221> variation
   <222> 201
   <223> /replace="c"
<400> 74
   tatcactcca gatgttctgg acagaacaaa gataggctag aatatgtgat gtaatgaatt 60
   aatgaatttt tttatacttc atcagccaac atcaagataa actgtattga actagcatgt 120
   gtccagctga agagtgttta aactgttagt gcaaagctga aacttcaata aacgctagga 180
   aaggatccag gttttattac tggtgataca gtttgagtgg ataaattcta accattttag 240
   ctaaaccctc cagcttcata ttaatcatga agtagaataa agtggggaaa aaaaacaacc 300
   atggggaatg tgaaaataag agtttaaaac tgaagtatta caaaggtcag cttctttatg 360
   agaagacaaa catagctgca ttttaattaa attatgagat t 401
<210> 75
   <211> 401
   <212> DNA
   <213> Artificial sequence
<220>
   <221> source
   <223> /note="Description of artificial sequence: rs4917639"
<220>
   <221> variation
   <222> 201
   <223> /replace="a"
<400> 75
   ttatttcact tagcctaatg ttatgttctc agggttcatt catgtttttg catgtgtcag 60
   atttttcttc ctttctaagg gtgaataata ttccgttgta tgtataaacc aagtttgctt 120
   acccatttat ctgttgatgg gcacttggat tactttcacc ttttgacttt ttgagtaatg 180
   ctattatgaa tatgggtaaa caaatatctc tttgatgtgc tgctttcaat tcttttgggt 240
   ataaataacc caaaagaata acccaccacg cctggttaat tttttgtatc tttagtagaa 300
   actgagtttc accatgttgg ccaggctggt ctcaaactcc tgaccctgtg atccacccac 360
   ctcagcctcc caaagtgctg gaattatagg tgtgagccac g 401
<210> 76
   <211> 401
   <212> DNA
   <213> Artificial sequence
<220>
   <221> source
   <223> /note="Description of artificial sequence: rs2279103"
<220>
   <221> variation
   <222> 160
   <223> /replace="c" /replace="g" /replace="t"
<220>
   <221> variation
   <222> 201
   <223> /replace="c"
<220>
   <221> variation
   <222> 263
   <223> /replace="c" /replace="g" /replace="t"
<400> 76
   acttttcatt attatttttt gtaaattatg cattttcact tgtagaaatc tctttccttg      60
   tggagactca atggtttgca ttattgatga tcgagaagat gtctggaagt ttgcccccaa     120
   tctgataact gtgaagaaat atgtatactt ccagggcaca ggtgatatga atgcgccccc     180
   tgggtcccga gaatctcaga tgagaaagaa aggtgggtaa cctccttcct gattctctag     240
   aagaattcac atttgcttat tgattagctc ttcttatttc ttatctctgt tttgactgct     300
   ataaattcaa gatacacttt ttttatttgt gtttcagtag agattattgg atttatttat     360
   agagtactga aaaacaggat attaggttgt ttcaatttgg g                         401
<210> 77
   <211> 401
   <212> DNA
   <213> Artificial sequence
<220>
   <221> source
   <223> /note="Description of artificial sequence: rs7307064"
<220>
   <221> variation
   <222> 92
   <223> /replace="c" /replace="g" /replace="t"
<220>
   <221> variation
   <222> 98
   <223> /replace="c" /replace="g" /replace="t"
<220>
   <221> variation
   <222> 106
   <223> /replace="c" /replace="g" /replace="t"
<220>
   <221> variation
   <222> 201
   <223> /replace="a"
<220>
   <221> variation
   <222> 360
   <223> /replace="c" /replace="g" /replace="t"
<400> 77
   ttgagaagaa aaagagcagt tcttttgggg gaatttagtt ttggtcatgc taatttagat 60
   acacatttct gataaacaat taggagtata aatttaaagt ttagcagtta gctaattaga 120
   tatgggaata atgttttaca ctttggcagc agatccacag agacaacata agtcatggta 180
   atacttaagc aagggtgata ctcataaggg atcaaaccat ttaggttgaa aatagggcaa 240
   gagtcacaaa tcaggtaact tcagtggcca ccaggtaaca actatgtggc gctatctggt 300
   taagaaaaga ggggccctta atagaaaggc gtatacatcc cgtttaaggc atttaaatca 360
   atttaaaata tataaataaa taaaaacaat gcccataata a 401
<210> 78
   <211> 401
   <212> DNA
   <213> Artificial sequence
<220>
   <221> source
   <223> /note="Description of artificial sequence: rs6793017"
<220>
   <221> variation
   <222> 30
   <223> /replace="c" /replace="g" /replace="t"
<220>
   <221> variation
   <222> 201
   <223> /replace="g"
<400> 78
   ataattgttt gagtcacaga ggttcctaca actccattaa gacagaaggt atagcctaga 60
   gttggtgggt ctgagacagt gtctccaaag tctgaaggat aataactgaa tatagactcc 120
   aaaatttaag gcttcagcat cctcagagtc cttgcatttg tgagagctat gtctgtgatg 180
   ttgccgtttg tcaccaggat actttatgtt gtcttttaag tgttaggtgt ccagatgcag 240
   atcacctgca tcttgcctca tatgtctctc tgaatgtaaa gattttgcct acatctgatg 300
   aaaactgtcc actgttcaca ttatttgtca tatgtatctg cttggagcag agtaagacac 360
   ttctaaggga acttgacatt tcatgaccca ggatatcaga t 401
<210> 79
   <211> 401
   <212> DNA
   <213> Artificial sequence
<220>
   <221> source
   <223> /note="Description of artificial sequence: rs523386"
<220>
   <221> variation
   <222> 50
   <223> /replace="c" /replace="g" /replace="t"
<220>
   <221> variation
   <222> 201
   <223> /replace="t"
<220>
   <221> variation
   <222> 367
   <222> 367
   <223> /replace="c" /replace="g" /replace="t"
<220>
   <221> variation
   <222> 387
   <223> /replace="c" /replace="g" /replace="t"
<400> 79
   gctgaggcca aaacaggcaa tttcccttac tcatgtcctt ggcgtgaaga gcctcgtcct     60
   tgttcagtct tgcaccgaga ggagagctct ctgagctcgt ggccagcagg tttcctgctg    120
   gaagccttac ctggatgctg ggcaaagctg tcagaataaa gttcaaggtg gtcagggctc    180
   tgcagggtcc ctcaagggag cttccctcat ctaaaaacat gattcaacac agtgagcgat    240
   tttcagttgt ggggttccta gcagtgtcta acaagccatt ctggagtcca tcaacatttt    300
   tttttttttt aataaaaaag gtgagatgga attaaggctg gaagtggcgg actttgggag    360
   aaacatacca ggagtctggc ctcctgagat gagcagagct c                        401
<210> 80
   <211> 401
   <212> DNA
   <213> Artificial sequence
<220>
   <221> source
   <223> /note="Description of artificial sequence: rs1984151"
<220>
   <221> variation
   <222> 18
   <223> /replace="c" /replace="g" /replace="t"
<220>
   <221> variation
   <222> 106
   <223> /replace="c" /replace="g" /replace="t"
<220>
   <221> variation
   <222> 201
   <223> /replace="c"
<220>
   <221> variation
   <222> 268
   <223> /replace="c" /replace="g" /replace="t"
<400> 80
   gacaacattc tgatcagatt ttcttttctt ttttatactc ctttaatttt tgcagttttc     60
   tttagattgt gccacctcct ggtctagctg tttctttgtg aatttagtaa taggctctct    120
   gtttgaattc tatggtgtta ttccaaacat tgctgccttc tttttgaagt atgtggctca    180
   catttcacac agatccttta tgatgatttc cttgcatagt gggaagtttt tgacgtgaag    240
   gtttactgga gtcccttggc tttcaagatg gctcagagca gatggtactg ctagagagag    300
   caacaacaca gctttgggct tgaaaatgtt agtgtacaca cgatgctttt tcccctcagg    360
   actggtatga ttgatgggac tttggaacta aaaactgaga a                        401
<210> 81
   <211> 401
   <212> DNA
   <213> Artificial sequence
<220>
   <221> source
   <223> /note="Description of artificial sequence: rs7719325"
<220>
   <221> variation
   <222> 201
   <223> /replace="a"
<400> 81
   gggattctca atccaagtgc tgacattctg gctaaaagag cagtaagaga tggatgatat     60
   aggcacagtc ttggcatgtc tgagccctgt gccagaaggt tatcagccct tttgggggag    120
   ataatatctg cagctttaat tttctctctg actaccaggc ataatgtaaa attaatgtgc    180
   atcatgtcaa ggttatggca gggcattgtt gtctggaagg gaaaactgtt tgctcccccg    240
   ccctccaaac acttgcacat accgtgataa tacattatta aaaagcacat ttctttgtca    300
   ttgtcaatag tggtgagcca gtaaaggata ttttccagca ctcattatgc aggaggaagt    360
   taagagactt ctaaaactca gtcttaaaac aaagaacata c                        401
<210> 82
   <211> 401
   <212> DNA
   <213> Artificial sequence
<220>
   <221> source
   <223> /note="Description of artificial sequence: rs1454333"
<220>
   <221> variation
   <222> 201
   <223> /replace="g"
<400> 82
   taccgatttg caacaaaatt tacttttcat gtacttatga ttctaatcta tctcaaaagt     60
   agataatcaa acttttgtaa ataacctttc aatgatggac aatgttattg ctcctttacc    120
   tattactacc tctccctgtt cttttcttca tcccaatcaa tatgtctaga taaatacatt    180
   tttttctcat ttgaaattta atttggaaaa tgcagccaca tttttactta tctttttcaa    240
   ataattaaaa acttctacct cactcatgta acatcttcct gcatgatcta atctgcatct    300
   aatttttctc tgactagcag cactagatgt tttgtgatta gggaagtcag aagtgatgag    360
   actttaatag aacataaaat atttcttttc tatatttcag t                        401
<210> 83
   <211> 401
   <212> DNA
   <213> Artificial sequence
<220>
   <221> source
   <223> /note="Description of artificial sequence: rs7146332"
<220>
   <221> variation
   <222> 40
   <223> /replace="c" /replace="g" /replace="t"
<220>
   <221> variation
   <222> 201
   <223> /replace="g"
<400> 83
   ttggttcttt ccctacccag cagccctata ccttgcctta atgctatgta tagctggagt     60
   ttatctcata ccatgaatag ccaagcatca catggaatat atcctaagaa atattccata    120
   aaaatgaact tgatgggatc atgacagaga tggcagatct acaaatttta agaattggag    180
   actatctcat agactttgtt aagatcacaa tcagttctca acttcaatgt ggtacattta    240
   caagcattgc aaacagtgtg agatcccagc acagatgtca aaccagaaat tatgtgactt    300
   gaatggcttg gaataatggc ccatttgggg catccagcac ttccactgtt gggtaagtac    360
   tgttgtcaac agtcacactg tccaagtgga tgatgcaccc t                        401
<210> 84
   <211> 401
   <212> DNA
   <213> Artificial sequence
<220>
   <221> source
   <223> /note="Description of artificial sequence: rs11250017"
<220>
   <221> variation
   <222> 100
   <223> /replace="c" /replace="g" /replace="t"
<220>
   <221> variation
   <222> 201
   <223> /replace="t"
<220>
   <221> variation
   <222> 308
   <223> /replace="c" /replace="g" /replace="t"
<400> 84
   tcttataata aatctatctg tctatatcta tatctacatc tgtcctccta ctggttctgt     60
   ttctctggac aaccctaaga ctagtatata aaaaatagaa caaactttca gattgagaaa    120
   aaagacatac aaggcaaatg cataagagat aagttttatg atatattgat attcaacatc    180
   ttaacataat taaatgaaga cgggaagtca ttgaaagaac aaaggagagt tcatgtttac    240
   tgctacaatg tgcaatgaag atataagtta ttaatatttt tggaaatttt aaaacatctc    300
   tcccataact atacaaatca agtggaccaa aaaattgagt aaggatgtgg aaaaaccagc    360
   attgtcagta agctagaaca cataaatata tattgaagtt g                        401
<210> 85
   <211> 401
   <212> DNA
   <213> Artificial sequence
<220>
   <221> source
   <223> /note="Description of artificial sequence: rs17175096"
<220>
   <221> variation
   <222> 201
   <223> /replace="t"
<400> 85
   ttgtaacaga aataaaatga atgtcattgt tacatatttt tttggtcctt gttagttcag 60
   caaacttctc gtttcctcct cctgctatgc tgtgagatgc atatgatgtc tatggaagag 120
   cgggaataaa atggttaaat tacaccaacc ccactcacct ctgctctttc cactgacagt 180
   gaacgaaacc atagtaaccc cataagaagg catctgtgaa gaaagctttc ctgtaaatac 240
   acccagtaag ttaagaatct gtgttacaaa tgcagagaca cagaattctc tgaaatcact 300
   aaaaagaaac ctgaccaaaa atgaatgaat gaatgaatga gccaacaaca aacaaagtga 360
   agggagctga cagtcctagt accttgcagt ttgtgacaac t 401
<210> 86
   <211> 401
   <212> DNA
   <213> Artificial sequence
<220>
   <221> source
   <223> /note="Description of artificial sequence: rs2299965"
<220>
   <221> variation
   <222> 201
   <223> /replace="t"
<400> 86
   tactcttcct ccaaaccaaa acccacctta acttttaaaa ataaaaatta ttttaaaaaa 60
   ttcttctgag cttttatcag ggaacagcat tgtattattt tttccaatga taaaatggca 120
   cagtgagttt ctcagctggt attttcacta cagaaggtgt actttcaggg gtgttgactc 180
   aatcttatac ctcatgatta ctgcaaaata ggcactgttt acagaattat tttaagtttt 240
   cagattaata ttgactttgt tatcatgctt attctcctaa aaaataactt attagcccgg 300
   tgtggtggct catgcctgta atcccagcac tctgggaggc tgaggccggt ggattgcttg 360
   accccaggag tttgagacca gcctggacaa cgtgataaaa c 401
<210> 87
   <211> 401
   <212> DNA
   <213> Artificial sequence
<220>
   <221> source
   <223> /note="Description of artificial sequence: rs2299967"
<220>
   <221> variation
   <222> 201
   <223> /replace="c"
<220>
   <221> variation
   <222> 396
   <223> /replace="c" /replace="g" /replace="t"
<400> 87
   taaagtgcta cccagaaagg ccataaaact gtttccctgg agattttcaa aacaggatat     60
   tcatctctac aggatggttt aaagacaaag ctgaggtgga aggcaacaat aatagccctg    120
   ataaactgca ggaaattaaa tgtttagcaa caatgaagaa tgagttttcc tagaacggtg    180
   gccaatctgg agagaagacc tgggatacag gtgagggtgg gaaagagcta gagaaaagca    240
   aagacaaaaa ttctctattt cttaacatat taaagaacat gaaatttaag aatgatcaaa    300
   actagtaata acacagtatg aggaaaatga gatatgaaat agagtatact catccgtcag    360
   cctgaatggt taaaaccatt catattttat aaagaaaatt t                        401
<210> 88
   <211> 401
   <212> DNA
   <213> Artificial sequence
<220>
   <221> source
   <223> /note="Description of artificial sequence: rs720733"
<220>
   <221> variation
   <222> 145
   <223> /replace="c" /replace="g" /replace="t"
<220>
   <221> variation
   <222> 201
   <223> /replace="g"
<400> 88
   caactttata gtcacaaaaa ttatctagaa ttgcatctat gatgcccatg ttccaggcag     60
   cagaaaagag ggcaatgaaa atgataaatg tcccccattt aaggaccttc ccaagagtct    120
   caggtatcaa cattatggac attaaattac taattttagc tgcaagggaa gcagggaact    180
   ggagtacttt agctgggctg atctgaatac aattgttttt ctgctattct gaatatttat    240
   taaagtctta actccttact aggtctattt ctcttttaac aattgggtcc acaattgatt    300
   cttcaggatg tttattgtgt ttgaggaata gctaaagagt catcatggtg gttgaagaga    360
   agcagtcagt ccatatgctc atgcttttac taaacttgag g                        401
<210> 89
   <211> 401
   <212> DNA
   <213> Artificial sequence
<220>
   <221> source
   <223> /note="Description of artificial sequence: rs635546"
<220>
   <221> variation
   <222> 201
   <223> /replace="t"
<220>
   <221> variation
   <222> 317
   <223> /replace="c" /replace="g" /replace="t"
<400> 89
   tctgcctgcc tggggccacc acccacccag tcatacacat aggacaagct caagagccct     60
   ccaactcact tagtcacttc ccccaaatca gatgtgactg aatcctatcg attttgcttc    120
   ctaaatagcc tgggaacagc ccctctccac tctggctgcc attttcctat ggaaatgaca    180
   gtgtttctca cctagattac cttcaccacc tcccacatag cctccctgcc tccagccttc    240
   tcccttcttg gatatgcacc aaagtgacct ttatctaata cccagggtta atcacttccc    300
   actcctactg agaacaattc aatggctcct tattgccccc cagtttctca aatgccttag    360
   atcagtggtc accaaccatt ttggcacaag ggaccagttt c                         401
<210> 90
   <211> 401
   <212> DNA
   <213> Artificial sequence
<220>
   <221> source
   <223> /note="Description of artificial sequence: rs7550277"
<220>
   <221> variation
   <222> 29
   <223> /replace="c" /replace="g" /replace="t"
<220>
   <221> variation
   <222> 201
   <223> /replace="t"
<400> 90
   aacttgtcat ttacaaacac ctgtaaatac gaactctact ttttaatatg gcattacaac 60
   ttacaagatt tgtatgtatg tatgcaatta accatgatac aatggcagca taaaatggca 120
   ccggtaaagt gtcaggcaca cagcctattg cagagtgaac actcacattt tgtttccacg 180
   tgccccacct cttttcttga cttaaaggat ttctactggg caaatgataa gagaaacaag 240
   acacttttct gtccttaagg aacttagact ggtaaagagg ataaaataaa cacagacaaa 300
   gccaggcagg atatacattt tgagagcaga atgaatccag gtgatataag aacacaaagg 360
   aggaagacca tgtgccaagt tccctgagga catcaggatc a 401
<210> 91
   <211> 401
   <212> DNA
   <213> Artificial sequence
<220>
   <221> source
   <223> /note="Description of artificial sequence: rs301191"
<220>
   <221> variation
   <222> 174
   <223> /replace="c" /replace="g" /replace="t"
<220>
   <221> variation
   <222> 201
   <223> /replace="g"
<220>
   <221> variation
   <222> 219
   <223> /replace="c" /replace="g" /replace="t"
<220>
   <221> variation
   <222> 230
   <223> /replace="c" /replace="g" /replace="t"
<220>
   <221> variation
   <222> 249
   <223> /replace="c" /replace="g" /replace="t"
<220>
   <221> variation
   <222> 318
   <223> /replace="c" /replace="g" /replace="t"
<400> 91
   tctgtgattg agggctgcaa aacaaagtat gtttaaaaat atgtttatgt tattttgata     60
   attttgaaaa tgtactctca cctgaggatg atctacaaag ccagttcagc tggctcttca    120
   gcactgtatt aaaaatactc ttagttctgg ggaaaaaaaa aaaaacactt tcaaaaaaca    180
   gtttttgaag agtaactgtg ataggttccg aagttggtat ctttcctgaa aggactctaa    240
   gtgccctaaa ttttatatgt aaaatcatgt agtttgcaag acattggaaa ggtggcactt    300
   caattaaacc cttcactacc acccccaaaa gttaaaaaat aagtaatatc tagagaaaaa    360
   ctggttatgg atccactgtg actggatagt atttgatcac c                        401
<210> 92
   <211> 401
   <212> DNA
   <213> Artificial sequence
<220>
   <221> source
   <223> /note="Description of artificial sequence: rs301193"
<220>
   <221> variation
   <222> 26
   <223> /replace="c" /replace="g" /replace="t"
<220>
   <221> variation
   <222> 201
   <223> /replace="t"
<220>
   <221> variation
   <222> 369
   <223> /replace="c" /replace="g" /replace="t"
<400> 92
   ctaattcatg gagtctgggt cggacatggc attctctatt tttagcaagc ttctcaagga     60
   taccatgttg atattcttcc catctttgaa tagcaaccca ttgctttaaa catatttaac    120
   atctctgggc tactattttc tattcaattg ttggtctact ccactaggat atatgtgagg    180
   tcctttaatt cagaataata ccttattcag ctgtgcaata agaaatggct gaaaaaatga    240
   tcattccttc tcctttttca ttattacttt tctttgacca aagaatgcta ttgctctaac    300
   aagaaatgta ccaattctga tgaagtctgt agcttggtaa aaatcacagg ggaggaataa    360
   gaccactaac taaccaatgt atttgctaac ataccataga a                        401
<210> 93
   <211> 401
   <212> DNA
   <213> Artificial sequence
<220>
   <221> source
   <223> /note="Description of artificial sequence: rs12583395"
<220>
   <221> variation
   <222> 180
   <223> /replace="c" /replace="g" /replace="t"
<220>
   <221> variation
   <222> 201
   <223> /replace="a"
<400> 93
   aaaaaataag atgattggag gacaatcagt agtgtctgtg acatctacaa cggagcagtt     60
   ggagaggagt aggcaaatcc ctatctactg gcaagaaaga tgtccaagaa catttccaag    120
   tgaggacgca ggttgtgagg tcatatgtcc attatcaccc atctaaggtt ctgaaaacaa    180
   acatttatca aggcatggag gaagttctgg aagttacact ctttttctct cagagaaagc    240
   tggaattggg gtttggaaag aagaggaaat atgtctattt actcaacata cttctgaatt    300
   ctttaattgt tgaaatcctc taagaacttg tattctgcgg tcatttttaa atacaaggga    360
   aaaacaaaca aacaacccag gctgtaagga agaaagtgat a                        401
<210> 94
   <211> 401
   <212> DNA
   <213> Artificial sequence
<220>
   <221> source
   <223> /note="Description of artificial sequence: rs1037448"
<220>
   <221> variation
   <222> 201
   <223> /replace="c"
<220>
   <221> variation
   <222> 305
   <223> /replace="c" /replace="g" /replace="t"
<400> 94
   gagccctggg catgtgagag tttcataagg tactcttgac caaccttatg tgttgaaaaa     60
   cagccttgtc caagagtcag gggactcttc cagaacattc aattcacctt tgccccaaac    120
   aggcctacca gggctaaaac aggtagttga atgtgtctca tcattagcta tttgggcagg    180
   tggatgttga tacaggttat tcttaggcat cagtgcccca tggctaatgc tgtaggcagt    240
   ggatgttttt ggaagtagcc aagcaggatt gctgcttgcc cttgagtgct ccatgccgtc 300
   tgccaagctc ccatcacttt aactaggaga accggcctcc aggatccctc cctttggttc 360
   taacataggc ctcagatcag atccagtgtc tgtggtttca g 401
<210> 95
   <211> 401
   <212> DNA
   <213> Artificial sequence
<220>
   <221> source
   <223> /note="Description of artificial sequence: rs4939517"
<220>
   <221> variation
   <222> 79
   <223> /replace="c" /replace="g" /replace="t"
<220>
   <221> variation
   <222> 201
   <223> /replace="t"
<400> 95
   tgctctccag cctgggggac agagtgagac tctgtctcaa aaaaaaaaaa aaaaaagaaa 60
   agaaggtcca agggctgcag ggaaaaagct tccatgaagc tggcaggaga aaatcatgcc 120
   accagcctcc tagaagattt gtgactcaac tacttaactc agagatttgc tacccagcct 180
   cagaactgcc tcctcaattc ctccaagggc agaggacgtt tcagatcacc agatattcac 240
   ctaaaacact ctccatccct cagtgaaatg taagagatct tgatcagcag gctcactcat 300
   ctgacaatat ctacttccac aactgttttt tttttctttg taagaggaaa aagtaaatgc 360
   agtccgaaag gatctgtagc cacaccctag aaaaggtcct g 401
<210> 96
   <211> 401
   <212> DNA
   <213> Artificial sequence
<220>
   <221> source
   <223> /note="Description of artificial sequence: rs4388301"
<220>
   <221> variation
   <222> 119
   <223> /replace="c" /replace="g" /replace="t"
<220>
   <221> variation
   <222> 201
   <223> /replace="t"
<400> 96
   aagacatgcc tgcttcccct tcacctccac catgattgta aatttcctga ggcctcccta 60
   gccatgcttc ctgtagaggc tgtggaattg tgagccaatt aaaccttttt tctttataaa 120
   ttacccagtc tcaggtattt ctttatagca gtacaagaac aggctaatat atcctctatt 180
   tatcttctct catttcaaac caaacttgtt ctccttcatc aaagaaagag agaaccctga 240
   aattcaaaga gacacaaaaa cattagtttt tgctgttgtg ctttgatagg cccagctata 300
   ttgctaacgg tagttcttgt aagcaactac tgttacttga agggtcattt tgactatcta 360
   ctatttttgc ctttttagtg tatttagaac aggccattct t 401
<210> 97
   <211> 401
   <212> DNA
   <213> Artificial sequence
<220>
   <221> source
   <223> /note="Description of artificial sequence: rs8095186"
<220>
   <221> variation
   <222> 21
   <223> /replace="c" /replace="g" /replace="t"
<220>
   <221> variation
   <222> 201
   <223> /replace="a"
<220>
   <221> variation
   <222> 281
   <223> /replace="c" /replace="g" /replace="t"
<220>
   <221> variation
   <222> 384
   <223> /replace="c" /replace="g" /replace="t"
<400> 97
   acttaatact gggggcaaga aggttcttgg ctgggcacac aatgctatgg gaacttaaaa 60
   acaaatggac tagctgcagg tttaaataat gctacagtcc ttagacactg tatattattc 120
   atagctcgtt gaactctatt atctcatgtg ttcctctcag tggctctgtg aattacccag 180
   gacaggaaag aaatgataga gtgtgaagat agaagagtca gtagaggtca gaacccttcc 240
   ccagcccctc agcttttttt tttttttttt ttttttttga aatgaagtct ggctatgtca 300
   ctctgaagtg tcgtggtgcg atctgggctc actgcaacct ccgccccctg ggttacagcg 360
   attctcctgc ctcagcctcc cgaatagctg ggactacagg t 401
<210> 98
   <211> 401
   <212> DNA
   <213> Artificial sequence
<220>
   <221> source
   <223> /note="Description of artificial sequence: rs1891877"
<220>
   <221> variation
   <222> 201
   <223> /replace="t"
<400> 98
   acccagagcc aagggtaaaa ggtctgggct tgttataatt tatcacccat ctcaaataat     60
   tggctatcat caagaattag attcatgcaa ggaatcactt aaagattcat ttgtctgtca    120
   cattaaaaac actaacctat ctttttcttc ttccttagca gatttttctt cgaggagcac    180
   aagggtgggt ctggtggaac ccttcaggag ctagggtgtc ggaggtgggg gactagccct    240
   ggtcccactg tccttcctcc cctcctcact ccccaagctg cccctcaccc cctctcttct    300
   ccttgggagc aatcccccat ctccatgctt gtgacacaca ctgagcttac ttctgagcac    360
   tcagttaata aaggcctgta cattgaaaaa tatttttaaa a                        401
<210> 99
   <211> 401
   <212> DNA
   <213> Artificial sequence
<220>
   <221> source
   <223> /note="Description of artificial sequence: rs2348427"
<220>
   <221> variation
   <222> 201
   <223> /replace="c"
<400> 99
   caatcagcag aggacatagt acttggagtt attttctaat agtgacagtg agcttgaatt     60
   tccaatatga ctctatcacc aggtcctctg accaaggaca tttacttgta atattatact    120
   aactaggtaa atcactagag cctgtaccag ttagttagct gggttcttag gatggatttt    180
   atataaagga tttgaagtga tataatgaaa aaatacagtt ttgcaaaaga tcccaaaatg    240
   tcctgtaggt ggtaattatt tcttaaatgg ttcttctatg aaaactaaga gcataacatt    300
   ttgttttaaa taagtgatgc attccatcat ttgggaggga aacaaacata atattgtttt    360
   aatacattac tgtccaatca tctattttat aagagatcaa c                        401
<210> 100
   <211> 401
   <212> DNA
   <213> Artificial sequence
<220>
   <221> source
   <223> /note="Description of artificial sequence: rs9480684"
<220>
   <221> variation
   <222> 53
   <223> /replace="c" /replace="g" /replace="t"
<220>
   <221> variation
   <222> 201
   <223> /replace="g"
<220>
   <221> variation
   <222> 255
   <223> /replace="c" /replace="g" /replace="t"
<220>
   <221> variation
   <222> 358
   <223> /replace="c" /replace="g" /replace="t"
<400> 100
   ccccagtttt acagacaaaa caacaacaac aacaacaaca aaaccccgga agatcaaagg     60
   ggttggccag tttggccaca atctcacagc tgtaactgct gaaccagaat ctggccatag    120
   gctgacttca aacatttatt ttattataat agtgttaggc taaaaatcac aagctgaaag    180
   gccgtgattg cacttagttc agtagagttg atgattctca tgtatacacg tgacctttca    240
   ttgaaaagat ctaaaatctc aaaggaatta acacagccta caggatttca atgcaacatg    300
   tgttcaatga agctaatagg gagtgaactg atggtgcttg ggggagaata aatttagagt    360
   ggttgtcctc aaactttatt tgcatcagaa tcacctggag g                        401